(19) Europäisches Patentamt
European Patent Office
Office européen des brevets

(11) EP 0 584 446 B2

(12) NEW EUROPEAN PATENT SPECIFICATION
After opposition procedure

(45) Date of publication and mention of the opposition decision:
**10.03.2010 Bulletin 2010/10**

(45) Mention of the grant of the patent:
**19.06.2002 Bulletin 2002/25**

(21) Application number: **93103697.4**

(22) Date of filing: **08.03.1993**

(51) Int Cl.:
*C12N 15/12* (2006.01)
*C12Q 1/68* (2006.01)
*A61K 38/02* (2006.01)
*G01N 33/60* (2006.01)
*A61K 31/56* (2006.01)
*A61K 31/23* (2006.01)
*C12Q 1/60* (2006.01)
*C12Q 1/61* (2006.01)
*C12N 9/10* (2006.01)

# (54) Microsomal triglyceride transfer protein

Microsomales Triglyceridtransferprotein

Protéine microsomale de transfert des triglycérides

(84) Designated Contracting States:
**AT BE CH DE DK ES FR GB GR IE IT LI LU MC NL PT SE**

(30) Priority: **06.03.1992 US 847503**

(43) Date of publication of application:
**02.03.1994 Bulletin 1994/09**

(73) Proprietor: **E.R. Squibb & Sons, Inc.**
**Princeton**
**New Jersey 08543-4000 (US)**

(72) Inventors:
- **Wetterau II, John R.**
**Langhorne, PA 19047 (US)**
- **Sharp, Daru Young**
**Perrineville, NJ 08535 (US)**
- **Gregg, Richard E.**
**Pennington, NJ 08534 (US)**

(74) Representative: **Vossius & Partner**
**P.O. Box 86 07 67**
**81634 München (DE)**

(56) References cited:
- **C.C. SHOULDERS ET AL.: 'Abetalipoproteinemia ia caused by defects of the gene encoding the 97 kDa subunit of a microsomal triglyceride transfer protein' HUMAN MOLECULAR GENETICS vol. 2, no. 12, 1993, pages 2109 - 2116, XP008073156**
- **D. SHARP ET AL.: 'Cloning and gene defects in microsomal triglyceride transfer protein associated with abnetalipoproteinemia' NATURE vol. 365, 02 September 1993, pages 65 - 69, XP002093036**
- **J. R. WETTEREAU ET AL.: 'Purification and characterisation of microsomal triglyceride and cholesteryl ester transfer protein from bovine liver microsomes' CHEMISTRY AND PHYSICS OF LIPIDS vol. 38, 1985, pages 205 - 222, XP003002495**
- **J.R. WETTEREAU ET AL.: 'Structural properties of the microsomal triglyceride transfer protein complex' BIOCHEMISTRY vol. 30, 1991, pages 4406 - 4412, XP003002496**
- **J.R. WETTERAU ET AL.: 'Protein Disulfide isomerase is a component of the MTP complex' JOURNAL OF BIOLOGICAL CHEMISTRY vol. 265, no. 17, 1990, pages 9800 - 9807, XP003002497**
- **J.R. WETTERAU ET AL.: 'Absence of MTP in Individuals with abetalipoproteinemia' SCIENCE vol. 258, 1992, pages 999 - 1001, XP003002498**

EP 0 584 446 B2

## Description

This invention relates to genes for the microsomal triglyceride transfer protein, expression vectors comprising the genes, host cells comprising the vectors, methods for producing the protein, methods for detecting inhibitors of the protein, and methods of using the inhibitors.

The microsomal triglyceride transfer protein (MTP) catalyzes the transport of triglyceride (TG), cholesteryl ester (CE), and phosphatidylcholine (PC) between small unilamellar vesicles (SUV). Wetterau & Zilversmit, Chem. Phys. Lipids 38, 205-22 (1985). When transfer rates are expressed as the percent of the donor lipid transferred per time, MTP expresses a distinct preference for neutral lipid transport (TG and CE), relative to phospholipid transport. The protein from bovine liver has been isolated and characterized. Wetterau & Zilversmit, Chem. Phys. Lipids 38, 205-22 (1985). Polyacrylamide gel electrophoresis (PAGE) analysis of the purified protein suggests that the transfer protein is a complex of two subunits of apparent molecular weights 58,000 and 88,000, since a single band was present when purified MTP was electrophoresed under nondenaturing condition, while two bands of apparent molecular weights 58,000 and 88,000 were identified when electrophoresis was performed in the presence of sodium dodecyl sulfate (SDS). These two polypeptides are hereinafter referred to as 58 kDa and 88 kDa, respectively, or the 58 kDa and the 88 kDa component of MTP, respectively, or the low molecular weight subunit and the high molecular weight subunit of MTP, respectively.

Characterization of the 58,000 molecular weight component of bovine MTP indicates that it is the previously characterized multifunctional protein, protein disulfide isomerase (PDI). Wetterau et al., J. Biol. Chem. 265, 9800-7 (1990). The presence of PDI in the transfer protein is supported by evidence showing that (1) the amino terminal 25 amino adds of the bovine 58,000 kDa component of MTP is identical to that of bovine PDI, and (2) disulfide isomerase activity was expressed by bovine MTP following the dissociation of the 58 kDa - 88 kDa protein complex. In addition, antibodies raised against bovine PDI, a protein which by itself has no TG transfer activity, were able to immunoprecipitate bovine TG transfer activity from a solution containing purified bovine MTP.

PDI normally plays a role in the folding and assembly of newly synthesized disulfide bonded proteins within the lumen of the endoplasmic reticulum. Bulleid & Freedman, Nature 335, 649-51 (1988). It catalyzes the proper pairing of cysteine residues into disulfide bonds, thus catalyzing the proper folding of disulfide bonded proteins. In addition, PDI has been reported to be identical to the beta subunit of human prolyl 4-hydroxylase. Koivu et al., J. Biol. Chem. 262, 6447-9 (1987). The role of PDI in the bovine transfer protein is not clear. It does appear to be an essential component of the transfer protein as dissociation of PDI from the 88 kDa component of bovine MTP by either low concentrations of a denaturant (guanidine HCl), a chaotropic agent (sodium perchlorate), or a nondenaturing detergent (octyl glucoside) results in a loss of transfer activity. Wetterau et al., Biochemistry 30, 9728-35 (1991). Isolated bovine PDI has no apparent lipid transfer activity, suggesting that either the 88 kDa polypeptide is the transfer protein or that it confers transfer activity to the protein complex.

The tissue and subcellular distribution of MTP activity in rats has been investigated. Wetterau & Zilversmit, Biochem. Biophys. Acta 875, 610-7 (1986). Lipid transfer activity was found in liver and intestine. Little or no transfer activity was found in plasma, brain, heart, or kidney. Within the liver, MTP was a soluble protein located within the lumen of the microsomal fraction. Approximately equal concentrations were found in the smooth and rough microsomes.

Abetalipoproteinemia is an autosomal recessive disease characterized by a virtual absence of plasma lipoproteins which contain apolipoprotein B (apoB). Kane & Havel in The Metabolic Basis of Inherited Disease, Sixth edition, 1139-64 (1989). Plasma TG levels may be as low as a few mg/dL, and they fail to rise after fat ingestion. Plasma cholesterol levels are often only 20-45 mg/dL. These abnormalities are the result of a genetic defect in the assembly and/or secretion of very low density lipoproteins (VLDL) in the liver and chylomicrons in the intestine. The molecular basis for this defect has not been previously determined. In subjects examined, triglyceride, phospholipid, and cholesterol synthesis appear normal. At autopsy, subjects are free of atherosclerosis. Schaefer et al., Clin. Chem. 34, B9-12 (1988). A link between the apoB gene and abetalipoproteinemia has been excluded in several families. Talmud et al., J. Clin. Invest. 82, 1803-6 (1988) and Huang et al., Am. J. Hum. Genet. 46, 1141-8 (1990).

Subjects with abetalipoproteinemia are afflicted with numerous maladies. Kane & Havel, supra. Subjects have fat malabsorption and TG accumulation in their enterocytes and hepatocytes. Due to the absence of TG-rich plasma lipoproteins, there is a defect in the transport of fat-soluble vitamins such as vitamin E. This results in acanthocytosis of erythrocytes, spinocerebellar ataxia with degeneration of the fasciculus cuneatus and gracilis, peripheral neuropathy, degenerative pigmentary retinopathy, and ceroid myopathy. Treatment of abetalipoproteinemic subjects includes dietary restriction of fat intake and dietary supplementation with vitamins A, E and K.

To date, the physiological role of MTP has not been demonstrated. In vitro, it catalyzes the transport of lipid molecules between phospholipid membranes. Presumably, it plays a similar role in vivo, and thus plays some role in lipid metabolism. The subcellular (lumen of the microsomal fraction) and tissue distribution (liver and intestine) of MTP have led to speculation that it plays a role in the assembly of plasma lipoproteins, as these are the sites of plasma lipoprotein assembly. Wetterau & Zilversmit, Biochem. Biophys. Acta 875, 610-7 (1986). The ability of MTP to catalyze the transport of TG between membranes is consistent with this hypothesis, and suggests that MTP may catalyze the

transport of TG from its site of synthesis in the endoplasmic reticulum (ER) membrane to nascent lipoprotein particles within the lumen of the ER.

Olofsson and colleagues have studied lipoprotein assembly in HepG2 cells. Bostrom et al., J. Biol. Chem. 263, 4434-42 (1988). Their results suggest small precursor lipoproteins become larger with time. This would be consistent with the addition or transfer of lipid molecules to nascent lipoproteins as they are assembled. MTP may play a role in this process. In support of this hypothesis, Howell and Palade, J. Cell Biol. 92, 833-45 (1982), isolated nascent lipoproteins from the hepatic Golgi fraction of rat liver. There was a spectrum of sizes of particles present with varying lipid and protein compositions. Particles of high density lipoprotein (HDL) density, yet containing apoB, were found. Higgins and Hutson, J. Lipid Res. 25, 1295-1305 (1984), reported lipoproteins isolated from Golgi were consistently larger than those from the endoplasmic reticulum, again suggesting the assembly of lipoproteins is a progressive event. However, there is no direct evidence in the prior art demonstrating that MTP plays a role in lipid metabolism or the assembly of plasma lipoprotein.

The present invention concerns a nucleic acid molecule as defined in claim 1.

The present invention also concerns a nucleic acid molecule having a sequence complementary to the above sequences.

The present invention further concerns expression vectors comprising a DNA sequence according to any one of claims 1 to 3.

The present invention additionally concerns prokaryotic or eukaryotic host cells containing an expression vector that comprises a DNA sequence according to any one of claims 1 to 3.

The present invention also concerns methods for detecting nucleic acid sequences according to any one of claims 1 to 3 or related nucleic acid sequences.

The present invention further concerns methods for detecting an inhibitor of MTP.

The present invention further concerns medical uses as defined in claims 13 to 17.

Figure 1 shows bovine cDNA clones. The five bovine cDNA inserts are illustrated. The continuous line at the top of the figure represents the total cDNA sequence isolated. Small, labeled bars above this line map peptide and probe sequences. The open reading frame is indicated by the second line, followed by ** corresponding to 3' noncoding sequences. Clone number and length are indicated to the left of each line representing the corresponding region of the composite sequence. Clones 64 and 76 were isolated with probe 2A, clones 22 and 23 with probe 37A and clone 2 with probe 19A. Eco RI linkers added during the cDNA library construction contribute the Eco RI restriction sites at the 5' and 3' ends of each insert. The internal Eco RI site in inserts 22 and 76 is encoded by the cDNA sequence. The Nhe I restriction site was utilized in preparing probes for isolation of human cDNA clones (below). The arrows under each insert line indicate individual sequencing reactions.

Figure 2 shows TG transfer activity in normal subjects. Protein-stimulated transfer of $^{14}$C-TG from donor SUV to acceptor SUV was measured in homogenized intestinal biopsies obtained from five normal subjects. The results are expressed as the percentage of donor TG transferred per hour as a function of homogenized intestinal biopsy protein.

Figure 3 shows TG transfer activity in abetalipoproteinemic subjects. Protein-stimulated transfer of $^{14}$C-TG from donor SUV to acceptor SUV was measured in homogenized intestinal biopsies obtained from four abetalipoproteinemic subjects. The results are expressed as the percentage of donor TG transferred/hour as a function of homogenized intestinal biopsy protein.

Figure 4 shows TG transfer activity in control subjects. Protein stimulated transfer of $^{14}$C-TG from donor SUV to acceptor SUV in homogenized intestinal biopsies were obtained from three control subjects, one with chylomicron retention disease (open circles), one with homozygous hypobetalipoproteinemia (solid circles), and one non-fasted (x). The results are expressed as the percentage of donor TG transferred/hour as a function of homogenized intestinal biopsy protein.

Figure 5 shows western blot analysis of MTP in normal subjects. An aliquot of purified bovine MTP (lane 1) or the post 103,000 x g proteins following deoxycholate treatment of 23 μg of homogenized intestinal biopsies from 3 normal subjects (lanes 2-4) were fractionated by SDS-PAGE and then transferred to nitrocellulose. The blots were probed with anti-88 kDa.

Figure 6 shows western blot analysis of MTP in control subjects. An aliquot of purified bovine MTP (lane 1) or the post 103,000 x g proteins following deoxycholate treatment of 15 μg, 25 μg, and 25 μg homogenized intestinal biopsies from a subject with chylomicron retention disease (lane 2), a subject with homozygous hypobetalipoproteinemia (lane 3), and a non-fasted subject (lane 4), respectively, were fractionated by SDS-PAGE and then transferred to nitrocellulose. The blots were probed with anti-88 kDa.

Figure 7 shows western blot analysis of MTP in normal subjects with affinity-purified antibodies. An aliquot of purified bovine MTP (lane 1) or the post 103,000 x g proteins following deoxycholate treatment of 34 μg (lane 2) or 25 μg (lane 3) of homogenized intestinal biopsies from 2 normal subjects were fractionated by SDS-PAGE and then transferred to nitrocellulose. The blots were probed with affinity purified anti-88 kDa.

Figure 8 shows western blot analysis of MTP in abetalipoproteinemic subjects. An aliquot of purified bovine

MTP (lane 1) or post 103,000 x g proteins following deoxycholate treatment of 18 μg (lane 2), 23 μg (lane 3), 23 μg (lane 4), 23 μg (lane 5) of homogenized intestinal biopsies from four different abetalipoproteinemic subjects were fractionated by SDS-PAGE and then transferred to nitrocellulose. In lanes 6 and 7, 100 μg of the whole intestinal homogenate (subjects corresponding to lane 4 and 5) was fractionated by SDS-PAGE and transferred to nitrocellulose. The blots were probed with anti-88 kDa.

Figure 9 shows a Southern blot analysis of a gene defect in an abetalipoproteinemic subject. Ten μg of genomic DNA from a control, the abetalipoproteinemic subject (proband), and from the subject's mother and father were cut to completion with Taq I, electrophoresed on 1% agarose and transferred to nitrocellulose. Southern hybridization was performed using exon 13 cDNA as a probe. Two hybridizing bands in the normal lane indicated the presence of a Taq I site in the normal exon 13. One hybridizing band in the abetalipoproteinemic subject lane demonstrated the absence of this restriction sequence in both alleles in exon 13, confirming a homozygous mutation in this subject. The heterozygous state in the mother and father is shown by the three hybridizing bands, corresponding to both the normal and the mutant restriction patterns.

Figure 10 shows inhibition in MTP-catalyzed transport of TG from donor SUV to acceptor SUV by compound A described hereinafter. Compound A was dissolved in DMSO and then diluted into 15/40 buffer. Aliquots were added to a lipid transfer assay to bring the compound to the indicated final concentrations. DMSO concentration in the assay never exceeded 2 μL/600 μL, a concentration that was independently determined to have minimal effect on the assay. MTP-catalyzed lipid transport was measured for 30 minutes at 37°C. TG transfer was calculated and compared to a control assay without inhibitor. Three independent assay conditions were used to demonstrate MTP inhibition by compound A. Assay conditions were: 8 nmol donor PC, 48 nmol acceptor PC, and 75 ng MTP (open circles); 24 nmol donor PC, 144 nmol acceptor PC, and 100 ng MTP (solid circles); 72 nmol donor PC, 432 nmol acceptor PC, and 125 ng MTP (open squares).

Figure 11 shows the dose response of Compound A on ApoB, ApoAI and HSA secretion from HepG2 cells. HepG2 cells were treated with compound A at the indicated doses for 16 hours. The concentration in the cell culture media of apoB, apoAI and HSA after the incubation period was measured with the appropriate ELISA assay and normalized to total cell protein. The data shown are expressed as a percentage of the control (DMSO only).

Figure 12 shows the effect of compound A on TG secretion from HepG2 cells. HepG2 cells were treated with Compound A at the indicated doses for 18 hours, the last two hours of which were in the presence of 5 μCi/mL 3H-glycerol. The concentration of radiolabelled triglycerides in the cell culture media was measured by quantitative extraction, followed by thin layer chromatography analysis and normalization to total cell protein. The data shown are expressed as a percentage of the control (DMSO only).

Figure 13 shows inhibition in MTP-catalyzed transport of TG from donor SUV to acceptor SUV by compound B described hereinafter. Compound B was dissolved in DMSO and then diluted into 15/40 buffer. Aliquots were added to a lipid transfer assay to bring the compound to the indicated final concentrations. DMSO concentration in the assay never exceeded 2 μL/600 μL, a concentration that was independently determined to have minimal effect on the assay. MTP-catalyzed lipid transport was measured for 30 minutes at 37°C. TG transfer was calculated and compared to a control assay without inhibitor. Two independent assay conditions were used to demonstrate MTP inhibition by compound B. Assay conditions were: 24 nmol donor PC, 144 nmol acceptor PC, and 100 ng MTP (open circles); 72 nmol donor PC, 432 nmol acceptor PC, and 125 ng MTP (solid circles).

Figure 14 shows the dose response of compound B on ApoB, ApoAI and HSA secretion from HepG2 cells. HepG2 cells were treated with compound B at the indicated doses for 16 hours. The concentration in the cell culture media of apoB, apoAI and HSA after the incubation period was measured with the appropriate ELISA assay and normalized to total cell protein. The data shown are expressed as a percentage of the control (DMSO only).

## Definition of terms

The following definitions apply to the terms as used throughout this specification, unless otherwise limited in specific instances.

The term "MTP" refers to a polypeptide or protein complex that (1) if obtained from an organism (e. g., cows, humans, etc.), can be isolated from the microsomal fraction of homogenized tissue; and (2) stimulates the transport of triglycerides, cholesterol esters, or phospholipids from synthetic phospholipid vesicles, membranes or lipoproteins to synthetic vesicles, membranes, or lipoproteins and which is distinct from the cholesterol ester transfer protein [Drayna et al., Nature 327, 632-634 (1987)] which may have similar catalytic properties. However, the MTP molecules of the present invention do not necessarily need to be catalytically active. For example, catalytically inactive MTP or fragments thereof may be useful in raising antibodies to the protein.

The term "modified", when referring to a nucleotide or polypeptide sequence, means a nucleotide or polypeptide sequence which differs from the wild-type sequence found in nature.

The term "related", when referring to a nucleotide sequence, means a nucleic acid sequence which is able to

hybridize to an oligonucleotide probe based on the nucleotide sequence of the high molecular weight subunit of MTP.

The phrase "control regions" refers to nucleotide sequences that regulate expression of MTP or any subunit thereof, including but not limited to any promoter, silencer, enhancer elements, splice sites, transcriptional initiation elements, transcriptional termination elements, polyadenylation signals, translational control elements, translational start site, translational termination site, and message stability elements. Such control regions may be located in sequences 5' or 3' to the coding region or in introns interrupting the coding region.

The phrase "stabilizing" atherosclerosis as used in the present application refers to slowing down the development of and/or inhibiting the formation of new atherosclerotic lesions.

The phrase "causing the regression of" atherosclerosis as used in the present application refers to reducing and/or eliminating atherosclerotic lesions.

**Use and utility**

The nucleic acids of the present invention can be used in a variety of ways in accordance with the present invention. For example, they can be used as DNA probes to screen other cDNA and genomic DNA libraries so as to select by hybridization other DNA sequences that code for proteins related to the high molecular weight subunit of MTP. In addition, the nucleic acids of the present invention coding for all or part of the high molecular weight subunit of human or bovine MTP can be used as DNA probes to screen other cDNA and genomic DNA libraries to select by hybridization DNA sequences that code for MTP molecules from other organisms. The nucleic acids may also be used to generate primers to amplify cDNA or genomic DNA using polymerase chain reaction (PCR) techniques. The DNA sequences of the present invention can also be used to identify adjacent sequences in the cDNA or genome; for example, those that encode the gene, its flanking sequences and its regulatory elements.

The polypeptides of the present invention are useful in the study of the characteristics of MTP; for example, its structure, mechanism of action, and role in lipid metabolism or lipoprotein particle assembly.

Various other methods of using the nucleic acids, polypeptides, expression vectors and host cells are described in detail below.

In carrying out the methods of the present invention, the agents that decrease the activity or amount of MTP can be administered to various mammalian species, such as monkeys, dogs, cats, rats, humans, etc., in need of such treatment. These agents can be administered systemically, such as orally or parenterally.

The agents that decrease the activity or amount of MTP can be incorporated in a conventional systemic dosage form, such as a tablet, capsule, elixir or injectable formulation. The above dosage forms will also include the necessary physiologically acceptable carrier material, excipient, lubricant, buffer, antibacterial, bulking agent (such as mannitol), anti-oxidants (ascorbic add or sodium bisulfite) or the like. Oral dosage forms are preferred, although parenteral forms are quite satisfactory as well.

The dose administered must be carefully adjusted according to the age, weight, and condition of the patient; as well as the route of administration, dosage form and regimen, and the desired result. In general, the dosage forms described above may be administered in single or divided doses of one to four times daily.

**Detailed description of specific embodiments**

Nucleic acids

The present invention concerns a nucleic acid molecule as defined in claim 1.

The sequence listing shows a bovine cDNA nucleotide sequence (SEQ. ID. NO. 1), a human cDNA sequence (SEQ. ID. NO. 2), a comparison of the human and bovine cDNA sequences, the bovine amino acid sequence(SEQ. ID. NO. 3), the human amino acid sequence (SEQ. ID. NO. 4), and a comparison of the human and bovine amino acid sequences. In the sequence comparisons, boxed regions represent perfect identity between the two sequences.

BOVINE/HUMAN cDNA SEQUENCE COMPARISON

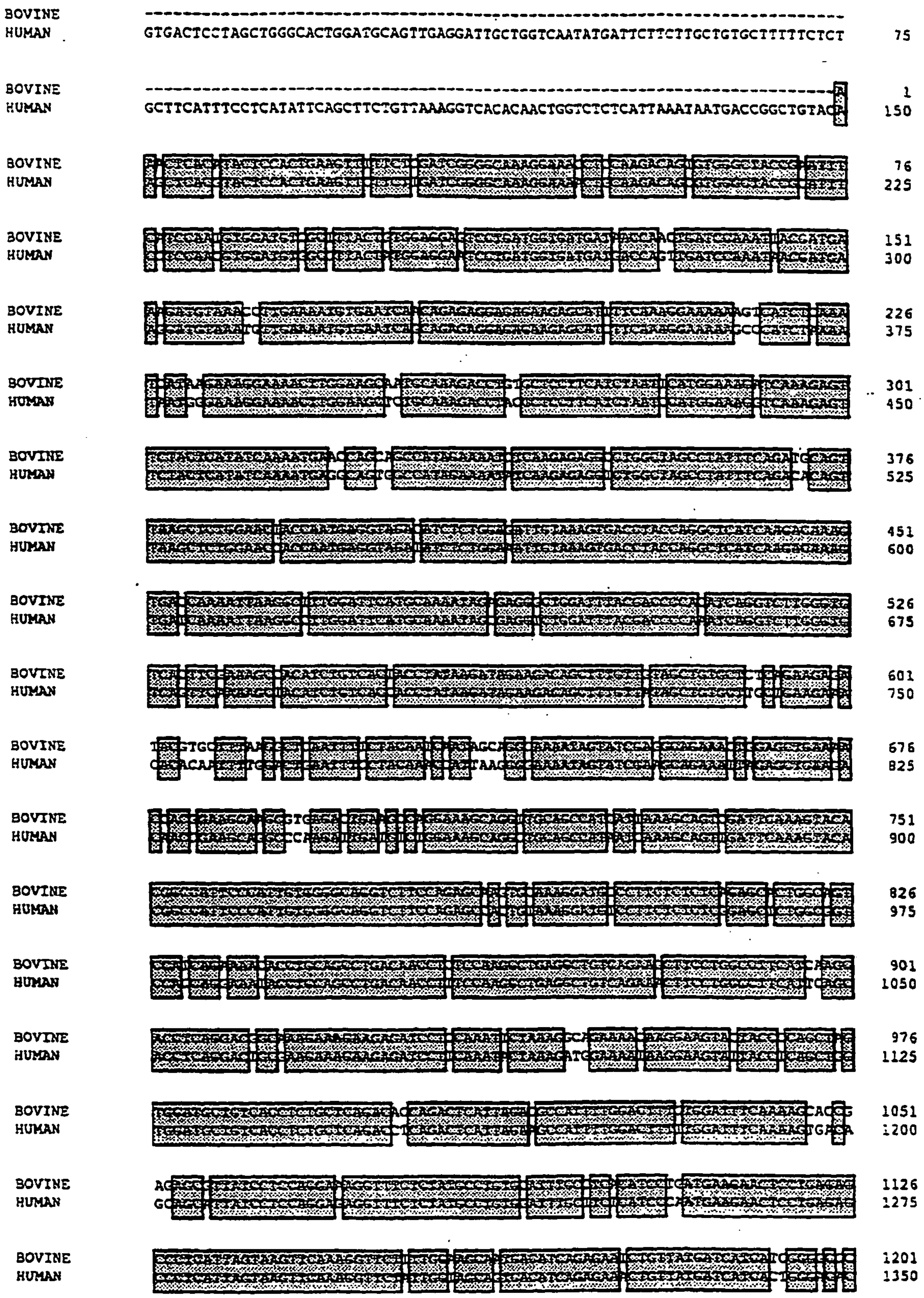

## BOVINE/HUMAN cDNA SEQUENCE COMPARISON

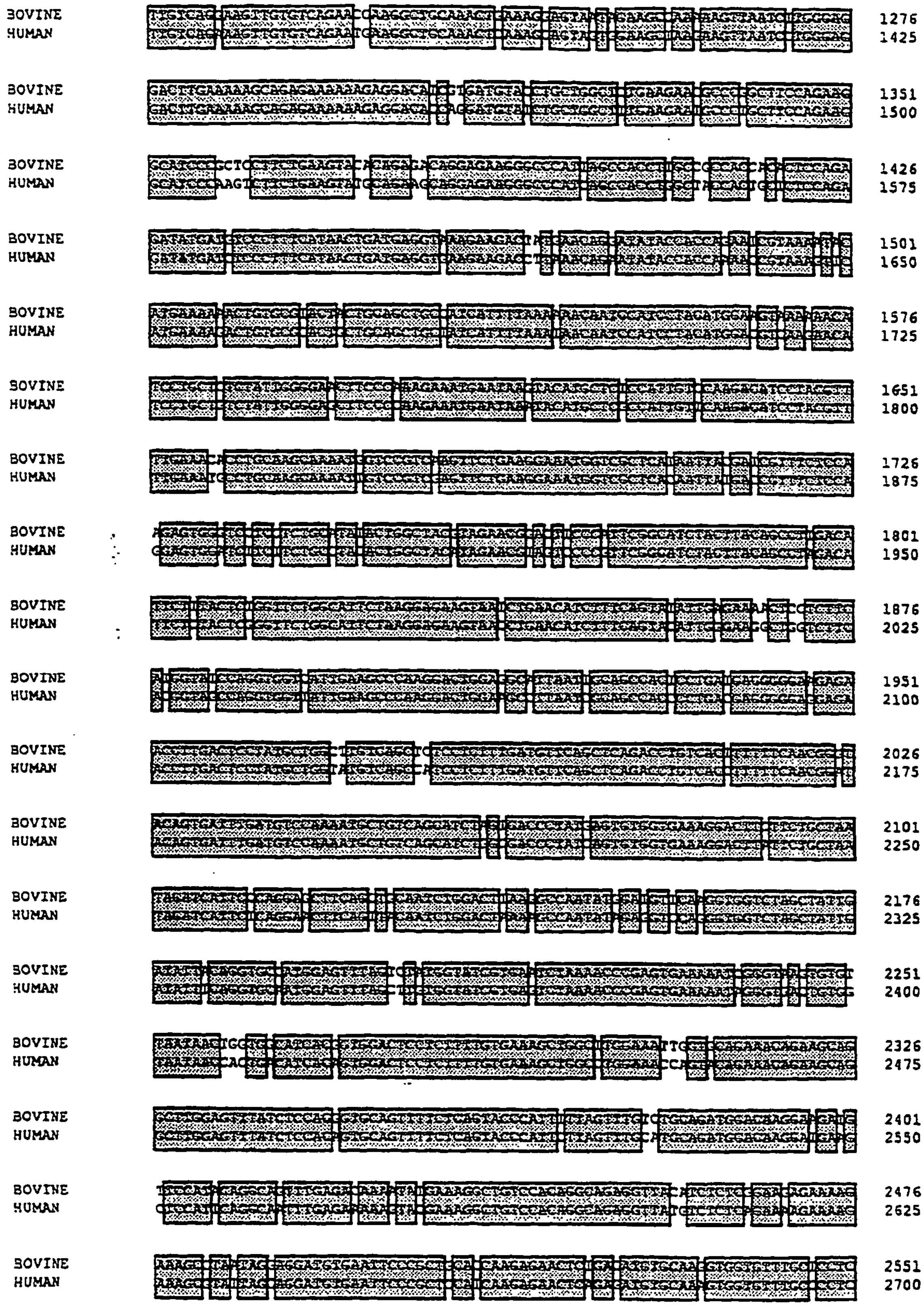

BOVINE/HUMAN cDNA SEQUENCE COMPARISON

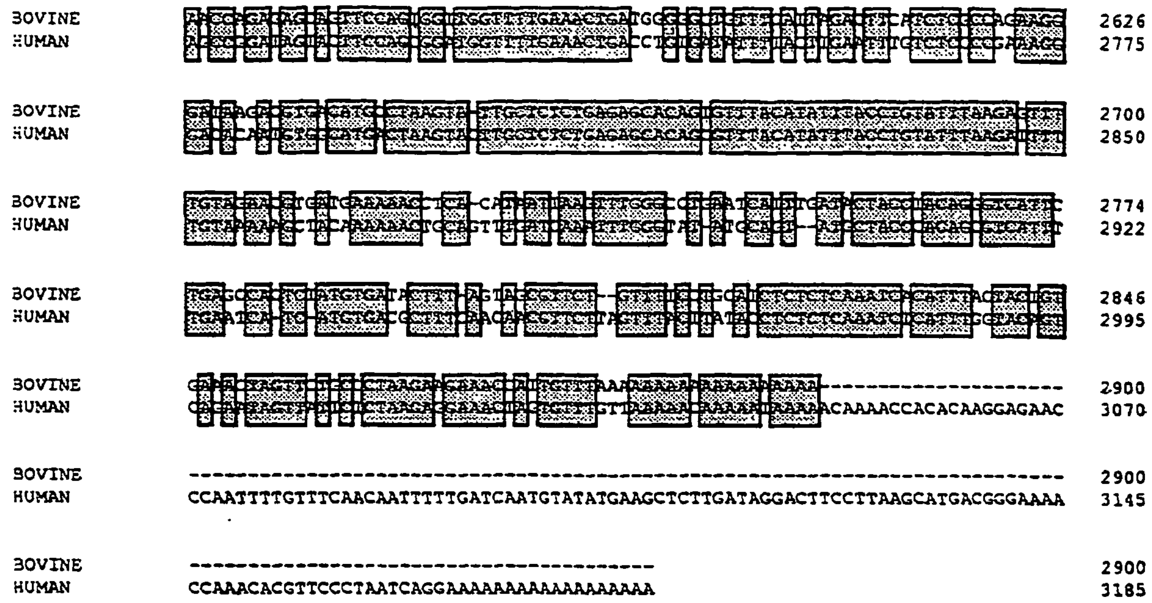

## BOVINE/HUMAN PROTEIN COMPARISON

```
BOVINE    ---------- ---------- ---------- ----KLTYST EVFLDRGKGN    16
HUMAN     MILLAVLFLC FISSYSASVK GHTTGLSLNN DRLYKLTYST EVLLDRGKGK    50

BOVINE    LQDSVGYRIS SNVDVALLWR SPDGDDNQLI QITMKDVNLE NVNQQRGEKS    66
HUMAN     LQDSVGYRIS SNVDVALLWR NPDGDDDQLI QITMKDVNVE NVNQQRGEKS   100

BOVINE    IFKGKKSSQI IRKENLEAMQ REVLLHLIHG KIKEFYSYQN EPAAIENLKR   116
HUMAN     IFKGKSFSKI MGKENLEALQ REILLHLIHG KMKEFYSYQN EAVAIENIKR   150

BOVINE    GLASLEQMQL SSGTTNEVDI SGDCKVTYQA HQDKVIKIKA LDSCKIERAG   166
HUMAN     GLASLEQIQL SSGTTNEVDI SGNCKVTYQA HQDKVIKIKA LDSCKIARSG   200

BOVINE    ETTPHQVLGV ISKATSVTTY KIEDSFVVAV LSEEIRALRE NELQSIPGKI   216
HUMAN     ETTPNQVLGV SSKATSVTTY KIEDSFVIAV LAEETHNFGL NELQTIKGKI   250

BOVINE    VSRQKLEEKT TEASVRLKPG KQVAAIIKAV DSKYTAIPIV GQVFQSKCKG   266
HUMAN     VSKQKLEEKT TEAGPRLMSG KQAAAIIKAV DSKYTAIPIV GQVFQSHCKG   300

BOVINE    CPSLSEHWQS IRKHLQPDNL SKAEAVRSFL AFIKHLRTAK KEEILQILKA   316
HUMAN     CPSLSELWRS IRKYLQPDNL SKAEAVRNFL AFIQHLRTAK KEEILQILKM   350

BOVINE    ENKEVLPQLV DAVTSAQTPD SLDAILDFLD FKSTESVILQ ERFLYACAFA   366
HUMAN     ENKEVLPQLV DAVTSAQTSD SLEAILDFLD FKSDSSIILQ ERFLYACGFA   400

BOVINE    SHPDEELLRA LISKFKGSFG SNDIRESVMI IIGALVRKLC QNQGCKLKGV   416
HUMAN     SHPNEELLRA LISKFKGSIG SSDIRETVMI IIGTLVRKLC QNEGCKLKAV   450

BOVINE    IEAKKLILGG LEKAEKKEDI VMYLLALKNA RLPEGIPLLL KYIEIGEGPI   466
HUMAN     VEAKKLILGG LEKAEKKEDT RMYLLALKNA LLPEGIPSLL KYAEAGEGPI   500

BOVINE    SHLATTILQR YDMPFITDEV KKTMNRIYHQ NRKIHEKTVR TIAAAIILKN   516
HUMAN     SHLATTALQR YDLPFITDEV KKTLNRIYHQ NRKVHEKTVR TAAAAIILNN   550

BOVINE    NPSYMEVKNI LLSIGELPKE MNKYMLSIVQ DILRFEIPAS KMVRQVLKEM   566
HUMAN     NPSYMDVKNI LLSIGELPQE MNKYMLAIVQ DILRFEMPAS KIVRRVLKEM   600

BOVINE    VAHNYDRFSK SGSSSAYTGY VERTSHSAST YSLDILYSGS GILRRSNLNI   616
HUMAN     VAHNYDRFSR SGSSSAYTGY IERSPRSAST YSLDILYSGS GILRRSNLNI   650
```

## BOVINE/HUMAN PROTEIN COMPARISON

```
BOVINE   FQYIEKTPLH GIQVVIEAQG LEALIAATPD EGEENLDSYA GLSALLFDVQ   666
HUMAN    FQYICKAGLH GSQVVIPAQG LEALIAATPD EGEENLDSYA GMSAILPDVQ   700

BOVINE   LRPVTFENGY SDLMSKMLSA SSDPMSVVKG ILLLIDHSQE LQLQSGLKAN   716
HUMAN    LRPVTFENGY SDLMSKMLSA SGDPTSVVKG IILLIDHSQE LQLQSGLKAN   750

BOVINE   MDVQGGLAID ILGAMEFSLW YRESKTRVKG RVSVLITGGL TVDSSEVKAG   766
HUMAN    IEVQGGLAID ISGAMEFSLW YRESKTRVKN RVIVMITTDI TVDSSFVKAG   800

BOVINE   LEIGAETEAG LEEISTVQFS QYPELVGLQM DKEDVPMRQF EIKYERLSTG   816
HUMAN    LETSTETEAG LEEISTVQFS QYPELVCMQM DKDEAPFRQF EKKYERLSTG   850

BOVINE   RGYISRKRKE SLIGGCEEPL HQENSDMCKV VEAPQPESSS SGWE   860
HUMAN    RGYMSQKRRE SVLAGCEEPL HQENSEMCKV VFAPQPDSIS SGWE   894
```

The bovine cDNA is a 2900 base composite of the cDNA sequences of clones 2 and 22 and has an open reading frame between bases 1 and 2580, predicting a translation product of 860 amino acids, followed by a TGA stop codon, 298 bases of 3 prime non-coding sequence, and a poly A region.

In the human cDNA, the 3185 bases predict an 894 amino acid translation product from bases 48 to 2729, followed by a TGA stop codon, 435 bases of 3 prime non-coding sequence, and a poly A region.

In the cDNA comparison, there is about an 88% identity between overlapping sequences in the coding region (bovine bases 1-2583 and human bases 150 - 2732). It is not necessary to introduce any gaps to attain this alignment within the coding region. The homology is somewhat weaker in the 3' noncoding region, including the introduction of several gaps to obtain optimal alignment.

The bovine protein sequence (SEQ. ID. NO. 3) is the 860 amino acid translation product of the combined sequence of bovine cDNA clones 2 and 22. Sequences for the peptide fragments used to design oligonucleotide probes are as follows: peptide 19A is found between residues 37 and 51, peptide 37A between residues 539 and 550, and peptide 2A between residues 565 and 572.

The human protein sequence (SEQ. ID. NO. 4) is the 894 amino acid translation product of human cDNA clone 693.

In the amino acid comparison, the bovine protein shows about 86% identity to the human translation product. When considering highly conserved substitutions at nonidentical residues, the two proteins are about 94 % homologous.

The inventors extended their knowledge of the 5' end of the foregoing bovine cDNA sequence with the sequence shown below, 5' to 3'. The top line shows the nucleotide sequence (SEQ. ID. NO. 5), and the bottom line the amino add sequence (SEQ. ID. NO. 6). The new sequence obtained (83 bases) is underlined.

```
 TT TTT CTC TGC TTC ATT TCC TCA TAT TCA GCT TCT
      F   L   C   F   I   S   S   Y   S   A   S
GTT AAA GGT CAC ACA ACT GGT CTC TCA TTA AAT AAT
 V   K   G   H   T   T   G   L   S   L   N   N
GAC CGA CTA TAC AAA CTC ACA TAC TCC ACT GAA GTT
 D   R   L   Y   K   L   T   Y   S   T   E   V
```

The inventors have also extended their knowledge of the 5' end of the foregoing human cDNA sequence. The additional sequence (SEQ. ID. NO. 7) is as follows:

AGAGTCCACTTCTCA

This sequence extends the 5' end of the human MTP cDNA sequence by 15 bases. These sequences were generated from human liver cDNA clone 754 isolated during the initial human cDNA cloning (see Example 3), but were characterized after clone 693.

The inventors have also elucidated a partial human genomic DNA sequence (SEQ. ID. NO. 8) for the high molecular weight subunit of MTP. Vertical lines indicate intron/exon boundaries. Exon sequences are in plain type, intron sequences in bold. Arrows indicate portions of the introns for which the sequence is not reported (arrow lengths do not indicate the size of the introns). The numbers in the right column indicate the first and last base of each exon relative to the human cDNA sequence shown supra. This extended genomic nucleic acid, as well as the extended cDNA, and fragments thereof are useful in the present invention.

The nucleic acids of the present invention can be isolated from a variety of sources, although the presently preferred sequences have been isolated from human and bovine cDNA and human genomic libraries. The exact amino acid sequence of the polypeptide molecule produced will vary with the initial DNA sequence.

The nucleic acids of the present invention can be obtained using various methods well-known to those of ordinary skill in the art. At least three alternative principal methods may be employed:

(1) the isolation of a double-stranded DNA sequence from genomic DNA or complementary DNA (cDNA) which contains the sequence;
(2) the chemical synthesis of the DNA sequence; and
(3) the synthesis of the DNA sequence by polymerase chain reaction (PCR).

In the first method, a genomic or cDNA library can be screened in order to identify a DNA sequence coding for all or part of the high molecular weight subunit of MTP. For example, bovine or human cDNA libraries can be screened in order to identify a DNA sequence coding for all or part of MTP. Various cDNA libraries, for example, a bovine small intestine lambda gt10 library (Clontech Laboratories, Inc. Palo Alto, CA), a human liver lambda UNI-ZAP™ XR library (Stratagene Cloning Systems, La Jolla, CA), or a human intestine lambda gt10 library (Clontech), can be used.

Various techniques can be used to screen genomic DNA or cDNA libraries for target sequences that code for the high molecular weight subunit of MTP. This technique may, for example, employ a labeled single-stranded DNA probe with a sequence complementary to a sequence that codes for the high molecular weight subunit of MTP. For example, DNA/DNA hybridization procedures may be used to identify the sequence in the cloned copies of genomic DNA or cDNA which have been denatured to a single-stranded form. Suitable probes include cDNA for the high molecular weight subunit of MTP acquired from the same or a related species, synthetic oligonucleotides, and the like.

A genomic DNA or cDNA library can also be screened for a genomic DNA or cDNA coding for all or part of the high molecular weight subunit of MTP using immunoblotting techniques.

In one typical screening method suitable for the hybridization techniques, a genomic DNA or cDNA library is first spread out on agarose plates, and then the clones are transferred to filter membranes, for example, nitrocellulose membranes. The genomic library is usually contained in a vector such as EMBL 3 or EMBL 4 or derivatives thereof (e.g., lambda DASH™). The cDNA library is usually contained in a vector such as λgt10, λgt11, or lambda ZAP. A DNA probe can then be hybridized to the clones to identify those clones containing the genomic DNA or cDNA coding for all or part of the high molecular weight subunit of MTP. Alternatively, appropriate E. coli strains containing vectors λgt11 or lambda ZAP can be induced to synthesize fusion proteins containing fragments of proteins corresponding to the cDNA insert in the vector. The fusion proteins may be transferred to filter membranes, for example, nitrocellulose. An antibody may then be bound to the fusion protein to identify all or part of the high molecular weight subunit of MTP.

In the second method, the nucleic acids of the present invention coding for all or part of MTP can be chemically synthesized. Shorter oligonucleotides, such as 15 to 50 nucleotides, may be directly synthesized. For longer oligonucleotides, the DNA sequence coding for the high molecular weight subunit of MTP can be synthesized as a series of 50-100 base oligonucleotides that can then be sequentially ligated (via appropriate terminal restriction sites) so as to form the correct linear sequence of nucleotides.

In the third method, the nucleic acids of the present invention coding for all or part of the high molecular weight subunit of MTP can be synthesized using PCR. Briefly, pairs of synthetic DNA oligonucleotides generally at least 15 bases in length (PCR primers) that hybridize to opposite strands of the target DNA sequence are used to enzymatically amplify the intervening region of DNA on the target sequence. Repeated cycles of heat denaturation of the template, annealing of the primers and extension of the 3'-termini of the annealed primers with a DNA polymerase results in amplification of the segment defined by the PCR primers. See White, T.J. et al., Trends Genet. 5, 185-9 (1989).

The nucleic acids of the present invention coding for all or part of MTP can also be modified (i.e., mutated) to prepare various mutations. Such mutations may change the amino acid sequence encoded by the mutated codon, or they may be silent and not change the amino acid sequence. These modified nucleic acids may be prepared, for example, by mutating the nucleic acid coding for the high molecular weight subunit of MTP so that the mutation results in the deletion, substitution, insertion, inversion or addition of one or more amino acids in the encoded polypeptide using various methods known in the art. For example, the methods of site-directed mutagenesis described in Taylor, J. W. et al., Nucl. Acids Res. 13. 8749-64 (1985) and Kunkel, J. A., Proc. Natl. Acad. Sci. USA 82, 482-92 (1985) may be employed. In addition, kits for site-directed mutagenesis may be purchased from commercial vendors. For example, a kit for performing site-directed mutagenesis may be purchased from Amersham Corp. (Arlington Heights, IL). In addition, disruption, deletion and truncation methods as described in Sayers, J. R. et al., Nucl. Acids Res. 16, 791-800 (1988) may also be employed. Mutations may be advantageous in producing or using the polypeptides of the present invention. For example, these mutations may modify the function of the protein (e.g., result in higher or lower activity), permit higher levels of protein production or easier purification of the protein, or provide additional restriction endonuclease recognition sites in the nucleic acid. All such modified nucleic adds and polypeptide molecules are included within the scope of the present invention.

Expression vectors

The present invention further concerns expression vectors comprising a DNA sequence as defined in any one of claims 1 to 3. Further preferred are expression vectors comprising one or more regulatory DNA sequences operatively linked to the DNA sequence as defined in any one of claims 1 to 3. As used in this context, the term "operatively linked" means that the regulatory DNA sequences are capable of directing the replication and/or the expression of the DNA sequence coding for all or part of the high molecular weight subunit of MTP.

Expression vectors of utility in the present invention are often in the form of "plasmids", which refer to circular double stranded DNA loops that, in their vector form, are not bound to the chromosome. However, the invention is intended to include such other forms of expression vectors which serve equivalent functions and which become known in the art subsequently hereto. The expression vectors of the present invention may also be used to stably integrate the DNA sequence encoding the high molecular weight subunit of MTP into the chromosome of an appropriate host cell (e.g.,COS or HepG2 cells).

Expression vectors useful in the present invention typically contain an origin of replication, a promoter located 5' to (i.e., upstream of) the DNA sequence, followed by the DNA sequence coding for all or part of the high molecular weight subunit of MTP, transcription termination sequences, and the remaining vector. The expression vectors may also include other DNA sequences known in the art, for example, stability leader sequences which provide for stability of the expression product, secretory leader sequences which provide for secretion of the expression product, sequences which allow expression of the structural gene to be modulated (e.g., by the presence or absence of nutrients or other inducers in the growth medium), marking sequences which are capable of providing phenotypic selection in transformed host cells, sequences which provide sites for cleavage by restriction endonucleases, and sequences which allow expression in various types of hosts, including but not limited to prokaryotes, yeasts, fungi, plants and higher eukaryotes. The characteristics of the actual expression vector used must be compatible with the host cell which is to be employed. For example, when expressing DNA sequences in a mammalian cell system, the expression vector should contain promoters isolated from the genome of mammalian cells, (e.g., mouse metallothionien promoter), or from viruses that grow in these cells (e.g., vaccinia virus 7.5 K promoter). An expression vector as contemplated by the present invention is at least capable of directing the replication, and preferably the expression, of the nucleic acids of the present invention. Suitable origins of replication include, for example, the Col E1, the SV4O viral and the M13 orgins of replication. Suitable promoters include, for example, the cytomegalovirus promoter, the lac Z promoter, the gal 10 promoter and the Autographa californica multiple nuclear polyhedrosis virus (AcMNPV) polyhedral promoter. Suitable termination sequences include, for example, the bovine growth hormone, SV40, lac Z and AcMNPV polyhedral polyadenylation signals. Examples of selectable markers include neomycin, ampicillin, and hygromycin resistance and the like. All of these materials are known in the art and are commercially available.

Suitable commercially available expression vectors into which the DNA sequences of the present invention may be inserted include the mammalian expression vectors pcDNAI or pcDNA/Neo, the baculovirus expression vector pBlueBac, the prokaryotic expression vector pcDNAII and the yeast expression vector pYes2, all of which may be obtained from Invitrogen Corp., San Diego, CA.

Suitable expression vectors containing the desired coding and control sequences may be constructed using standard recombinant DNA techniques known in the art, many of which are described in Sambrook, et al., Molecular Cloning: A Laboratory Manual, Second Edition, Cold Spring Harbor Laboratory, Cold Spring Habor, NY (1989).

Host cells

The present invention additionally concerns host cells containing an expression vector as defined above. See, for example, the expression vector appearing in Example 4 hereinbelow, which is preferred. Further preferred are host cells containing an expression vector comprising one or more regulatory DNA sequences capable of directing the replication and/or the expression of and operatively linked to a DNA sequence coding for all or part of the high molecular weight subunit of MTP. Suitable host cells include both prokaryotic and eukaryotic cells. Suitable prokaryotic host cells include, for example, E. coli strains HB101, DH5a, XL1 Blue, Y1090 and JM101. Suitable eukaryotic host cells include, for example, Spodoptera frugiperda insect cells, COS-7 cells, human skin fibroblasts, and Saccharomyces cerevisiae cells.

Expression vectors may be introduced into host cells by various methods known in the art. For example, transfection of host cells with expression vectors can be carried out by the calcium phosphate precipitation method. However, other methods for introducing expression vectors into host cells, for example, electroporation, liposomal fusion, nuclear injection, and viral or phage infection can also be employed.

Once an expression vector has been introduced into an appropriate host cell, the host cell may be cultured under conditions permitting expression of large amounts of the desired polypeptide, in this case a polypeptide molecule comprising all or part of the high molecular weight subunit of MTP.

Host cells containing an expression vector as defined above may be identified by one or more of the following six general approaches: (a) DNA-DNA hybridization; (b) the presence or absence of marker gene functions; (c) assessing the level of transcription as measured by the production of mRNA transcripts encoding the high molecular weight subunit of MTP in the host cell; (d) detection of the gene product immunologically; (e) enzyme assay; and (f) PCR.

In the first approach, the presence of a DNA sequence as defined above can be detected by DNA-DNA or RNA-DNA hybridization using probes complementary to the DNA sequence.

In the second approach, the recombinant expression vector host system can be identified and selected based upon the presence or absence of certain marker gene functions (e.g., thymidine kinase activity, resistance to antibiotics, etc.). A marker gene can be placed in the same plasmid as the DNA sequence as defined above under the regulation of the same or a different promoter used to regulate the MTP coding sequence. Expression of the-marker gene indicates expression of the DNA sequence as defined above.

In the third approach, the production of mRNA transcripts encoding the high molecular weight subunit of MTP can be assessed by hybridization assays. For example, polyadenylated RNA can be isolated and analyzed by Northern blotting or a nuclease protection assay using a probe complementary to the RNA sequence. Alternatively, the total RNA of the host cell may be extracted and assayed for hybridization to such probes.

In the fourth approach, the expression of all or part of the high molecular weight subunit of MTP can be assessed immunologically, for example, by immunoblotting with antibody to MTP (Western blotting).

In the fifth approach, expression of the high molecular weight subunit of MTP can be measured by assaying for MTP enzyme activity using known methods. For example, the assay described herein below may be employed.

In the sixth approach, oligonucleotide primers homologous to sequences present in the expression system (i.e., expression vector sequences or MTP sequences) are used in a PCR to produce a DNA fragment of predicted length, indicating incorporation of the expression system in the host cell.

The DNA sequences of expression vectors, plasmids or DNA molecules of the present invention may be determined by various methods known in the art. For example, the dideoxy chain termination method as described in Sanger et al., Proc. Natl. Acad. Sci. USA 74, 5463-7 (1977), or the Maxam-Gilbert method as described in Proc. Natl. Acad. Sci. USA 74, 560-4 (1977) may be employed.

In order to express catalytically active MTP, it may be necessary to produce a protein complex containing both the high and low molecular weight subunits of MTP. The low molecular weight subunit of MTP is the previously characterized protein, protein disulfide isomerase (PDI). PDI cDNAs have been cloned from human [Pihlajaniemi et al., EMBO J. 6, 643-9 (1987)], bovine [Yamaguchi et al., Biochem. Biophys. Res. Comm. 146, 1485-92 (1987)], rat [Edman et al., Nature 317, 267-70 (1985)] and chicken [Kao et al., Connective Tissue Research 18, 157-74 (1988)]. Various approaches can be used in producing a protein containing both the high and low molecular weight subunits of MTP. For example, cDNA sequences encoding the subunits may be inserted into the same expression vector or different expression vectors and expressed in an appropriate host cell to produce the protein.

It should, of course, be understood that not all expression vectors and DNA regulatory sequences will function equally well to express the DNA sequences of the present invention. Neither will all host cells function equally well with the same expression system. However, one of ordinary skill in the art may make a selection among expression vectors, DNA regulatory sequences, and host cells using the guidance provided herein without undue experimentation and without departing from the scope of the present invention.

Polypeptides

Further disclosed are polypeptide molecules comprising all or part of the high molecular weight subunit of MTP, said polypeptide molecules preferably having all or part of the amino acid sequence as shown in SEQ. ID. NOS. 3, 4, or 3 together with 6. In the case of polypeptide molecules comprising part of the high molecular weight subunit of MTP, it is preferred that polypeptide molecules be at least about 5 to 8 sequential amino acids in length, more preferably at least about 15 to 20 sequential amino acids in length. Also disclosed are polypeptides at least about 180 sequential amino acids in length, which may approximate the size of a structural domain within the protein.

All amino acid sequences are represented herein by formulas whose left to right orientation is in the conventional direction of amino-terminus to carboxy-terminus.

The polypeptides may be obtained by synthetic means, i.e., chemical synthesis of the polypeptide from its component amino acids, by methods known to those of ordinary skill in the art. For example, the solid phase procedure described by Houghton et al., Proc. Natl. Acad. Sci. 82, 5131-5 (1985) may be employed. The polypeptides are obtained by production in prokaryotic or eukaryotic host cells expressing a DNA sequence according to the invention or by in vitro translation of the mRNA encoded by a DNA sequence according to the present invention. For example, the DNA sequence of SEQ. ID. NOS. 1, 2, 8, 1 together with 5, 2 together with 7, the first 108 bases of 2 together with 8, or the first 108 bases of 2 together with 7 and 8 may be synthesized using PCR as described above and inserted into a suitable expression vector, which in turn may be used to transform a suitable host cell. The recombinant host cell may then be cultured to produce the high molecular weight subunit of MTP. Techniques for the production of polypeptides by these means are known in the art, and are described herein.

The polypeptides produced in this manner may then be isolated and purified to some degree using various protein purification techniques. For example, chromatographic procedures such as ion exchange chromatography, gel filtration chromatography and immunoaffinity chromatography may be employed.

The polypeptides may be used in a wide variety of ways. For example, the polypeptides may be used to prepare in a known manner polyclonal or monoclonal antibodies capable of binding the polypeptides. These antibodies may in turn be used for the detection of the polypeptides of the present invention in a sample, for example, a cell sample, using immunoassay techniques, for example, radioimmunoassay, enzyme immunoassay, or immunocytochemistry. The antibodies may also be used in affinity chromatography for isolating or purifying the polypeptides of the present invention from various sources.

The polypeptides have been defined by means of determined DNA and deduced amino acid sequencing. Due to the degeneracy of the genetic code, other DNA sequences which encode the same amino acid sequences depicted in SEQ. ID. NOS. 3, 4, 3 together with 6, or any part thereof may be used for the production of the polypeptides.

It should be further understood that allelic variations of these DNA and amino acid sequences naturally exist, or may be intentionally introduced using methods known in the art. These variations may be demonstrated by one or more amino acid changes in the overall sequence, such as deletions, substitutions, insertions, inversions or addition of one or more amino acids in said sequence. Such changes may be advantageous in producing or using the polypeptides of the present invention; for example in isolation of MTP or the polypeptides by affinity purification. Amino acid substitutions may be made, for example, on the basis of similarity in polarity, charge, solubility, hydrophobicity, hydrophilicity and/or the amphiphathic nature of the residues involved. For example, negatively charged amino acids include aspartic acid and glutamic acid; positively charged amino acids include lysine and arginine; amino adds with uncharged polar head groups or nonpolar head groups having similar hydrophilicity values include the following: leucine, isoleucine, valine, glycine, alanine; asparagine, glutamine; serine, threonine; phenylalanine, tyrosine. Other contemplated variations include salts and esters of the aforementioned polypeptides, as well as precursors of the aforementioned polypeptides, for example, precursors having N-terminal substituents such as methionine, N-formylmethionine and leader sequences. All such variations are included within the scope of the present invention.

Method for detection of nucleic acids

The present invention further concerns a method for detecting a nucleic acid sequence as defined in claim 7.

A DNA sample containing the DNA sequence can be isolated using various methods for DNA isolation which are well-known to those of ordinary skill in the art. For example, a genomic DNA sample may be isolated from tissue by rapidly freezing the tissue from which the DNA is to be isolated, crushing the tissue to produce readily digestible pieces, placing the crushed tissue in a solution of proteinase K and SDS, and incubating the resulting solution until most of the cellular protein is degraded. The genomic DNA is then deproteinized by successive phenol/chloroform/isoamyl alcohol extractions, recovered by ethanol precipitation, and dried and resuspended in buffer.

Also preferred is the method in which the nucleic acid sequence is an RNA sequence. Preferably, the RNA sequence is an mRNA sequence. Additionally preferred is the method in which the RNA sequence is located in the cells of a tissue sample. An RNA sample containing the RNA sequence may be isolated using various methods for RNA

isolation which are well-known to those of ordinary skill in the art. For example, an RNA sample may be isolated from cultured cells by washing the cells free of medium and then lysing the cells by placing them in a 4 M guanidinium solution. The viscosity of the resulting solution is reduced by drawing the lysate through a 20-gauge needle. The RNA is then pelleted through a cesium chloride step gradient, and the supernatant fluid from the gradient carefully removed to allow complete separation of the RNA, found in the pellet, from contaminating DNA and protein.

The detectable marker useful for detecting a nucleic add sequence coding for all or part of the high molecular weight subunit of MTP or a related nucleic acid sequence, may be a labeled DNA sequence, including a labeled cDNA sequence, having a nucleotide sequence complementary to at least a portion of the DNA sequence coding for all or part of the high molecular weight subunit of MTP.

The detectable marker may also be a labeled RNA having a sequence complementary to at least a portion of the DNA sequence coding for all or part of the high molecular weight subunit of MTP.

The detectable markers of the present invention may be labeled with commonly employed radioactive labels, such as $^{32}P$ and $^{35}S$, although other labels such as biotin or mercury may be employed. Various methods well-known to those of ordinary skill in the art may be used to label the detectable markers. For example, DNA sequences and RNA sequences may be labeled with $^{32}P$ or $^{35}S$ using the random primer method.

Once a suitable detectable marker has been obtained, various methods well-known to those of ordinary skill in the art may be employed for contacting the detectable marker with the sample of interest. For example, DNA-DNA, RNA-RNA and DNA-RNA hybridizations may be performed using standard procedures known in the art. In a typical DNA-DNA hybridization procedure for detecting DNA sequences coding for all or part of MTP in genomic DNA, the genomic DNA is first isolated using known methods, and then digested with one or more restriction enzymes. The resulting DNA fragments are separated on agarose gels, denatured in situ. and transferred to membrane filters. After prehybridization to reduce nonspecific hybridization, a radiolabeled nucleic acid probe is hybridized to the immobilized DNA fragments. The membrane is then washed to remove unbound or weakly bound probe, and is then autoradiographed to identify the DNA fragments that have hybridized with the probe.

The presence of bound detectable marker may be detected using various methods well-known to those of ordinary skill in the art. For example, if the detectable marker is radioactively labeled, autoradiography may be employed. Depending on the label employed, other detection methods such as spectrophotometry may also be used.

It should be understood that nucleic acid sequences related to nucleic acid sequences coding for all or part of the high molecular weight subunit of MTP can also be detected using the methods described herein. For example, a DNA probe that has conserved regions of the gene for the high molecular weight subunit of human or bovine MTP can be used to detect and isolate related DNA sequences (e.g., a DNA sequence coding for the high molecular weight subunit of MTP from mice, rats, hamsters, or dogs). All such methods are included within the scope of the present invention.

Methods for detecting MTP inhibitors

Further described are methods for detecting inhibitors of MTP. In particular, the present invention concerns a process for detecting an inhibitor of MTP comprising: (a) incubating a sample thought to contain an inhibitor of MTP with detectably labeled lipids in donor particles, acceptor particles and MTP; and (b) measuring the MTP stimulated transfer of the detectably labeled lipids from the donor particles to the acceptor particles. In this assay, an inhibitor would decrease the rate of MTP-stimulated transfer of detectable labeled lipid from donor to acceptor particles. The detection may be carried out by nuclear magnetic resonance (NMR), electron spin resonance (ESR), radiolabeling (which is preferred), fluorescent labeling, and the like. The donor and acceptor particles may be membranes, HDL, low density lipoproteins (LDL), SUV, lipoproteins and the like. HDL and SUV are the preferred donor particles; LDL and SUV are the preferred acceptor particles.

The foregoing procedure was carried out to identify the MTP inhibitor

A (reference compound)

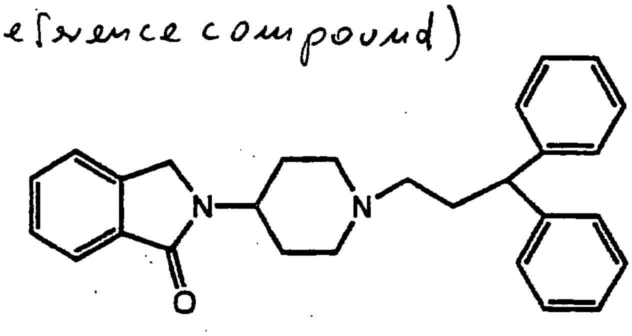

which has the name 2-[1-(3, 3-diphenylpropyl)-4-piperidinyl]-2, 3-dihydro-3-oxo-1H-isoindole hydrochloride (herein referred to as "compound A"). The foregoing procedure also identified the MTP inhibitor

B

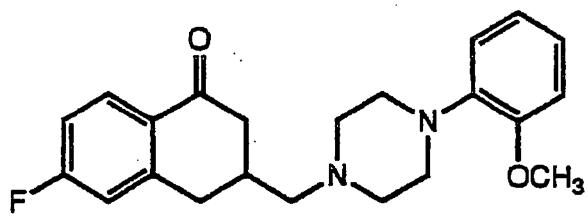

which has the name 1-[3-(6-fluoro-1-tetralanyl)methyl]-4-O-methoxyphenyl piperazine (herein referred to as "compound B").
These compounds were identified by the procedures described in the working examples hereinafter.

Uses

The present invention also concerns a uses according to claims 13 to 17.
Various agents which effectively decrease the amount or activity of MTP can be used in practicing the methods of the present invention. MTP inhibitors can be isolated using the screening methodology described hereinabove and in Example 5 hereinbelow. Compound B, which is identified as inhibitor of MTP (see Example 6 hereinbelow), is useful in specific embodiments of the foregoing methods of treatment.
Antisense molecules may be used to reduce the amount of MTP. [See, Toulme and Helene, Gene 72, 51-8 (1988); Inouye, Gene, 72, 25-34 (1988); and Uhlmann and Peyman, Chemical Reviews 90, 543-584 (1990)]. MTP antisense molecules can be designed based on the foregoing genomic DNA and cDNA, corresponding 5' and 3' flanking control regions, other flanking sequences, or intron sequences. Such antisense molecules include antisense oligodeoxyribonucleotides, oligoribonucleotides, oligonucleotide analogues, and the like, and may comprise about 15 to 25 bases or more. Such antisense molecules may bind noncovalently or covalently to the DNA or RNA for the high molecular weight subunit of MTP. Such binding could, for example, cleave or faciltate cleavage of MTP DNA or RNA, increase degradation of nuclear or cytoplasmic mRNA, or inhibit transcription, translation, binding of transactivating factors, or pre-mRNA splicing or processing. All of these effects would decrease expression of MTP and thus make the antisense molecules useful in the foregoing methods of treatment.
Potential target sequences for an antisense approach include but are not limited to the DNA or RNA sequence encoding MTP, its 5' and 3' flanking control regions, other flanking sequences, and nonclassic Watson and Crick base pairing sequences used in formation of triplex DNA. Antisense molecules directed against tandem sequences for the high molecular weight subunit of MTP may be advantageous.
Antisense molecules may also contain additional functionalities that increase their stability, activity, transport into and out of cells, and the like. Such additional functionalities may, for example, bind or facilitate binding to target molecules, or cleave or facilitate cleavage of target molecules.
Vectors may be constructed that direct the synthesis of antisense DNA or RNA. In this case, the length of the antisense molecule may be much longer; for example, 400 bp.

**Demonstration of relationship between MTP and serum cholesterol levels, TG levels, and atherosclerosis**

The methods of the present invention for lowering serum cholesterol or TG levels or preventing, stabilizing or causing regression of atherosclerosis are based in part on the discovery by the inventors that the genetic disease abetalipoproteinemia is caused by a lack of functional MTP. The inventors have demonstrated a gene defect in two abetalipoproteinemic subjects by the following methods.

**Assay for TG transfer activity in abetalipoproteinemic subjects**

A. MTP Assay

TG transfer activity was measured as the protein-stimulated rate of TG transfer from donor SUV to acceptor SUV. To prepare donor and acceptor vesicles, the appropriate lipids in chloroform were mixed in a 16 x 125 mm

borosilicate glass screw cap tube (Fisher Scientific Co., Pittsburg, PA, Cat. no. 14-933-1A) and then dried under a stream of nitrogen. Two mL 15/40 buffer (15 mM Tris, pH 7.4, 40 mM sodium chloride, 1 mM EDTA, and 0.02% $NaN_3$) were added to the dried lipids (or 100 μL per assay, which ever is the least volume), a stream of nitrogen was blown over the buffer, then the cap was quickly screwed on to trap a nitrogen atmosphere over the lipid suspension. Lipids in the buffer were bath-sonicated in a Special Ultrasonic Cleaner (Cat. no. G112SP1, Laboratory Supplies Co., Hicksville, NY). The donor and acceptor phosphatidylcholine (PC) (egg L-alpha-phosphatidylcholine, Sigma Chem. Co., St. Louis, MO) was radiolabeled by adding traces of [$^3$H] dipalmitoylphosphatidylcholine (phosphatidylcholine L-alpha-dipalmitoyl [2-palmitoyl-9,10, $^3$H (N)], 33 Ci/mmol, DuPont NEN) to an approximate specific activity of 100 cpm/nmol. Donor vesicles containing 40 nmol egg PC, 0.2 mol% [$^{14}$C]TG [mixture of labeled (triolein [carboxyl-$^{14}$C]-, about 100 mCi/mmol, DuPont NEN) and unlabeled (triolein, Sigma Chem. Co., St. Louis, MO) triolein for a final specific activity of about 200,000 cpm/nmol], and 7.3 mol% cardiolipin (bovine heart cardiolipin, Sigma Chemical Co.) and acceptor vesicles containing 240 nmol egg PC and 0.2 mol% TG were mixed with 5 mg fatty acid free bovine serum albumin (BSA) and an aliquot of the MTP samples in 0.7 to 0.9 mL 15/40 buffer and incubated for 1 hour at 37°C. The transfer reaction was terminated by the addition of 0.5 mL DEAE-cellulose suspension (1:1 suspension DE-52, preswollen DEAE-cellulose anion exchange, Fisher, Cat. no. 05720-5 to 15 mM Tris, pH 7.4, 1 mM EDTA, and 0.02% $NaN_3$). The reaction mixture was agitated for 5 minutes and the DEAE-cellulose with bound donor membranes (the donor membranes contained the negatively charged cardiolipin and bound to the DEAE) were sedimented by low speed centrifugation.

The $^{14}$C-TG and $^3$H-PC remaining in the supernatant were quantitated by scintillation counting. TG transfer was calculated by comparing the ratio of $^{14}$C-TG (transferred from the donor membranes to the acceptor membranes) to $^3$H-PC (a marker of acceptor vesicle recovery) present in the supernatant following a transfer reaction to the ratio of total donor$^{14}$C-TG to acceptor [$^3$H]PC in the assay before the transfer reaction. The percentage of $^{14}$C-TG transfer was calculated as follows:

$$\%\ \text{TG Transfer} = \frac{(^{14}\text{C-TG}/^{3}\text{H-PC})_{sup}}{(^{14}\text{C-TG}_{don}/^{3}\text{H-PC}_{acc})_{total}} \times 100\%$$

To calculate the MTP-stimulated rate of TG transfer, the TG transfer rate in the absence of MTP was subtracted from the TG transfer rate in the presence of MTP. First order kinetics was used to calculate total TG transfer.

B. Antibody Production

Anti-88 kDa antibodies were obtained from the University of Cincinnati. The production of anti-88 kDa has been previously described. Wetterau et al., J. Biol. Chem. 265, 9800-7 (1990). To help address the specificity of the anti-sera in human intestinal homogenates, affinity purified anti-88 kDa was generated. Eight to 10 mg of purified MTP was dialyzed into 0.1 M MOPS, pH 7.5 and then added to 4 mL Bio Rad Affigel 15 (Bio-Rad, Richmond, CA) which had been prewashed 3 times with water at 4°C. The MTP was allowed to couple to the matrix at room temperature for two hours and then it was placed at 4°C overnight. The remaining reactive sites on the affigel were blocked by the addition of 0.1 mL 1 M ethanolamine, pH 8.0, per mL gel. Optical density measurements of eluted protein were performed according to the manufacturer's instructions and indicated that more than 90% of the MTP was coupled to the column. The column was washed with 50 mL 10 mM Tris, pH 7.5 followed by 50 mL 100 mM glycine, pH 2.5, followed by 50 mL 10 mM Tris, pH 8.8, followed by 50 mL 100 mM triethylamine, pH 11.5, and finally the column was reequilibrated in 10 mM Tris, pH 7.5.

The antibodies in the antiserum were partially purified by ammonium sulfate precipitation (226 mg ammonium sulfate per mL serum). The pellet was resuspended and dialyzed into 15 mM Tris, pH 7.5, mM EDTA, 0.02% sodium azide, and 150 mM sodium chloride. The partially purified antibodies were slowly applied to the MTP-affigel column over a two-hour period (the antibodies were cycled through the column three times). The column was washed with 100 mL 10 mM Tris, pH 7.5, followed by 100 mL 10 mM Tris, pH 7.5, 500 mM sodium chloride, followed by 50 mL 100 mM glycine, pH 2.5 (this fraction was collected into 5 mL of 1 M Tris, pH 8.0), followed by 10 mM Tris, pH 8.8 until the column was at neutral pH, followed by 50 mL triethylamine pH 11.5 (this fraction was collected into 5 mL 1 M Tris, pH 8.0), and finally the column was reequilibrated with 10 mM Tris, pH 7.5. Antibodies which eluted in the acidic wash were retained and used for immunoblot analysis of protein fractions.

C. Westem Blot with anti-88 kDa Antibodies

To confirm the specificity of the antibodies and to detect the 88 kDa component of MTP in tissue homogenates, purified bovine MTP or the fraction to be tested were fractionated by SDS-PAGE [essentially as described by Laemmli,

Nature 227, 680-5 (1970)] using a 0.75 mm Hoeffer Scientific Instrument Gel Apparatus (San Francisco, CA). The protein was then transferred to nitrocellulose by Western blotting using a BioRad Trans-blot cell (Bio-Rad, Richmond, CA). The blotting buffer (25 mM Tris, 192 mM glycine, pH 8.3, 20% methanol) was precooled to 4˚C. The proteins were transferred for 100 minutes at 250 milliamperes at room temperature. The membranes were blocked 5-10 minutes with blocking buffer (400 μL antifoam, about 10 mg of thimersal, and 200 g nonfat dry milk in 4 liters 50 mM Tris, pH 7.7, 150 mM sodium chloride). An aliquot of the antiserum (1:300 dilution) or affinity purified antibody (1:25 dilution of affinity-purified antibodies) was added and allowed to react overnight at room temperature. Following washing with blocking buffer, the secondary antibody, goat anti-rabbit IgG coupled to horseradish peroxidase (BioRad), was added at a dilution of 1:2000 and allowed to react for 3 hours at room temperature. Following a washing step, the secondary antibody was visualized with developer, 50 mg imidizale, 50 mg 3,3'diaminobenzidine tetrahydrochloride, and 50 μL $H_2O_2$ (30% solution) in 50 mL blocking buffer.

D. MTP in Intestinal Biospies

Intestinal biopsies from fasted control and disease state subjects were frozen and shipped to Bristol-Myers Squibb, Princeton on dry ice. Biopsies were homogenized with a polytron (Polytron PT3000, Brinkmann Instrument, Inc., Westbury, NY) at 1/2 maximal settling. Typically, one biopsy was homogenized in 0.25 mL homogenization buffer (50 mM Tris, pH 7.4, 50 mM KCI, 5 mM EDTA, 5 μg/mL leupeptin, and 2 mM PMSF). An aliquot of the protein was adjusted to 0.7 mL and 1.4% SDS and the protein concentration was measured by the method of Lowry et al. [J. Biol. Chem. 193, 265-75 (1951)]. The homogenate was diluted with homogenization buffer to about 1.75 mg protein/mL. In some cases, the protein was already more dilute and was used directly. To release the soluble proteins from the microsomal fraction, one part deoxycholate solution (0.56%, pH 7.5) was added-to 10 parts diluted homogenate with vortexing. The sample was incubated at 4˚C for 30 minutes, then centrifuged at 103,000 x g for 60 minutes. The supernatant was removed, diluted 1:1 with 15/40 buffer, and then dialyzed overnight into 15/40 buffer. Aliquots of the treated biopsies were assayed for TG transfer activity and Western blot analysis was used to detect 88 kDa protein. TG transfer activity was expressed as the percentage of donor TG transferred per hour as a function of homogenized intestinal biopsy protein.

E. Results with Abetalipoproteinemic Subjects

To investigate whether there is a relationship between defective MTP and abetalipoproteinemia, MTP activity in duodenal or duodenal-jejunal biopsies was measured from five control subjects and four abetalipoproteinemic subjects having the classic genetically recessive form of abetalipoproteinemia. Intestinal biopsies from the five normal subjects were homogenized and treated with detergent as described hereinabove. TG transfer activity was readily detectable in biopsies from all five subjects (Figure 2).

The TG transfer activity in the biopsies was further characterized. To confirm that TG hydrolysis was not interfering with lipid transfer activity measurements, one subject's acceptor vesicles (which contained the transported lipid) were extracted after the transfer reaction, and the identity of the $^{14}$C-TG was confirmed by thin layer chromatography. All of the $^{14}$C-TG had a mobility identical to that of authentic TG, confirming that intact TG was being transported in the assay.

The human MTP was characterized for its heat stability. It was inactivated when heated to 60˚C for 5 minutes. The loss of activity demonstrates that the lipid transfer activity being measured was not from an intracellular form of the cholesteryl ester transfer protein (CETP), which is heat-stable under these conditions. Ihm et al., J. Biol. Chem. 257, 4818-27 (1982).

Intestinal biopsies from four abetalipoproteinemic subjects were obtained, homogenized, and TG transfer activity was measured as desribed herein above. No transfer activity was recovered from the biopsies of any of the four subjects (Figure 3). The lack of detectable TG transfer activity could have been related to an inability to release MTP from the microsomes of the abetalipoproteinemic biopsies by deoxycholate treatment. To test this possibility, the microsomes from one subject were sonicated in addition to being treated with detergent. Bath sonication independently releases TG transfer activity comparable to that of detergent treatment. Even under these conditions, no TG transfer activity was detectable.

The next possibility considered was that the lack of detectable TG transfer activity was related to the inability to detect it in cells which contain large intracellular fat droplets such as those which occur in abetalipoproteinemia. To test this possibility, three controls were run. First, TG transfer activity was measured from a biopsy of a subject with chylomicron retention disease. Subjects with chylomicron retention desease have a defect in the assembly or secretion of chylomicrons and have large fat droplets in their enterocytes, analogous to abetalipoproteinemic subjects. In addition, TG transfer activity was measured from a biopsy taken from an individual who was not fasted prior to the biopsy and from a homozygous hypobetalipoproteinemic subject. Both these subjects also had fat-filled enterocytes. In all three

cases, TG transfer activity comparable to that of the normal subjects was found (Figure 4), confirming that the presence of intracellular lipid droplets does not interfere with our ability to recover and detect TG transfer activity.

To establish the biochemical defect responsible for the absence of transfer activity, the soluble proteins following release of MTP from the microsomal fraction of the homogenized biopsy were analyzed by Western blot analysis with antibodies raised against the 88 kDa component of bovine MTP. When normal (Figure 5) or control (Figure 6) subjects were examined with a polyclonal anti-88 kDa antibody, a band comparable to that of the 88 kDa component of bovine MTP was observed. In addition, additional proteins of increased mobility also cross-reacted with this antibody. To confirm the identity of the 88 kDa component of human MTP, the antibody was affinity-purified on an MTP affinity column. Following this treatment, only the protein of molecular weight comparable to that of the 88 kDa component of bovine MTP was immunoreactive (Figure 7).

Western blot analysis of the soluble proteins following detergent treatment of the microsomes of all five normal subjects and three control subjects demonstrated the presence of the 88 kDa component of MTP (Figures 5 to 7). In contrast, no protein corresponding to the 88 kDa component of bovine MTP was apparent in the abetalipoproteinemic subjects (Figure 8). In addition, a similar analysis was performed with 100 μg protein from the whole intestinal homogenates from two abetalipoproteinemic subjects. Again, no band corresponding to the 88 kDa component of MTP was apparent (Figure 8). As a control, immunoblot analysis with anti-PDI antibodies demonstrated the presence of PDI in the latter two abetalipoproteinemic subjects. These results demonstrate that the biochemical basis for the absence of MTP activity in the abetalipoproteinemic subjects is the marked deficiency or the absence of the 88 kDa component of MTP.

**Demonstration of a gene defect in an abetalipoproteinemic subject**

Amplification of mRNA and DNA by PCR

Two intestinal biopsies were obtained from the duodenal mucosa of a 39-year-old abetalipoproteinemic patient. Previous analysis demonstrated that neither MTP activity nor the 88 kDa component of MTP were detectable in intestinal biopsies taken from this subject . Each biopsy weighed 5-10 mg and was stored frozen at -70˚C. To isolate total RNA, one frozen biopsy was placed into a microfuge tube containing 0.8 mL of cold RNAzol B (CinnaBiotecx labs, Friendswood, Texas). The biopsy was homogenized immediately by polytron (Brinkmann, Westbury, NY) for 6 strokes on setting 10. Chloroform (80 μL) was added and the mixture inverted gently 20 times. After a 5-minute incubation on ice, the mixture was centrifuged at 14,000 rpm in an Eppendorf microfuge 5415 (Brinkmann) for 15 minutes at 4˚C. Total RNA was precipitated by adding 350 μL isopropanol to the supernatant. The yield from the biospy was 20 μg of total RNA, or about 2 μg RNA per mg of tissue (0.2%).

RNA (50 ng) was reverse transcribed into first strand cDNA using 2.5 μM random hexamer primers, 5 mM magnesium chloride, 1 mM each deoxynucleotide triphosphate (dNTP), 1 U/μL RNAsin, 2.5 U/μL Moloney Murine Leukemia Virus reverse transcriptase ((M-MLV-RT), and 1X PCR reaction buffer (Perkin-Elmer-Cetus RNA-PCR kit No. N808-0017). The 20 μL reaction was incubated at room temperature for 10 minutes to anneal the primers, and then at 42˚C for 30 minutes to reverse transcribe the RNA. The reaction was terminated by heating to 99˚C for 5 minutes and cooling to 5˚C. The first strand cDNA was added to a 100 μL PCR containing 0.15 μM forward and reverse primers, 2 mM magnesium chloride, 0.2 mM each dNTP, and 2.5 U Taq polymerase in 1.25X PCR buffer. Amplification was conducted in a Perkin-Elmer GeneAmp PCR System 9600 model thermal cycler for 50 cycles consisting of 94˚C for 30 seconds, 50˚C for 30 seconds, and 72˚C for 1 minute. The reaction was then incubated at 72˚C for 7 minutes. The forward and reverse primers used to amplify the 5' region of the RNA encoding the 88 kDa component of MTP are shown below, 5' to 3'.

| Forward Primers | Sequence | SEO. ID. NO. |
|---|---|---|
| 15F | 15 40<br>GGCACTGGATGCAGTTGAGGATTGCT | 9 |
| 41F | 41 67<br>GGTCAATATGATTCTTCTTGCTGTGC | 10 |
| 578F | 578 602<br>CCG<u>GAATTC</u>CCTACCAGGCTCATCAAGACAAAG | 11 |

(continued)

| Forward Primers | Sequence | SEO. ID. NO. |
|---|---|---|
| 900F | 900 925<br>ACGGCCATTCCCATTGTGGGGCAGGT | 12 |

| Reverse Primers | Sequence | SEQ. ID. NO. |
|---|---|---|
| 678R | 678 653<br>TGACACCCAAGACCTGATTTGGGGTC | 13 |
| 839R | 839 815<br>GCCTGCTTCGGTTGTCTTCAGCTCT | 14 |
| 1029R | 1029 1006<br>CGCGGATCCTTCTGACAGCCTCAGCCTTGGA | 15 |
| 1588R | 1588 1563<br>GGGAGATCATATCTCTGGAGAGCAGT | 16 |
| 2117R | 2117 2097<br>CGGCGGATCCAGCATAGGAGTCAAGGTTCTC | 17 |

Shown below are the primer combinations used the PCR product length.

| Primer Pair | Product Length (bp) |
|---|---|
| 15F + 678R | 664 |
| 15F + 839R | 825 |
| 41F + 1029R | 998 |
| 578F + 1029R | 470 |
| 900F + 1588R | 689 |
| 900F + 2117R | 1228 |

The primer sequences are based on the normal human cDNA encoding the 88 kDa component of MTP. All primers are written 5' to 3'. F refers to the forward primer, and R to the reverse primer. The underlining identifies restriction sites recognized by Eco RI (primer 578F) or Bam HI (primers 1029R and 2117R), which were incorporated into the 5' end of the primers.

Subject genomic DNA was isolated from a second frozen intestinal biopsy. The biopsy was placed into a microfuge tube containing 400 μL extraction buffer (10 mM Tris.Cl, pH 8.0, 0.1 M EDTA, 0.5% SDS, 20 μg/mL RNAse I) and homogenized immediately. Homogenization was by polytron for 5 strokes at setting 10. Proteinase K was added to a final concentration of 100 μg/ml and the reaction incubated at 50°C for 3 hours. The mixture was swirled periodically.

After cooling the reaction to room temperature, 400 μL Tris-saturated phenol/chloroform (pH 8.0) was added. The tube was inverted gently for 5 minutes and then centrifuged for 5 minutes at 14,000 rpm at room temperature. 2 M sodium chloride (35 μL) and ethanol (0.7 ml) were added to the supernatant (350 μL) to precipitate the DNA. The DNA was centrifuged briefly, washed gently with 70% ethanol, dried briefly, and resuspended in 20 μL of deionized water ($dH_2O$). The yield of DNA was 20 μg, or about 2 μg DNA per mg tissue (0.2%).

Genomic DNA (0.5 μg) was heated to 95°C for 5 minutes and added immediately to a 100 μL PCR reaction containing 0.15 μM forward and reverse primers, 2 mM magnesium chloride, 0.2 mM each dNTP, and 2.5 U Taq polymerase in 1.25X PCR buffer (Perkin-Elmer-Cetus). Amplification was conducted in a Perkin-Elmer GeneAmp PCR

System 9600 model thermal cycler for 3 cycles consisting of 97°C for 30 seconds, 50°C for 30 seconds, and 72°C for 1 minute. An additional 32 cycles consisting of 94°C for 30 seconds, 50°C for 30 seconds, and 72°C for 1 minute were run. The reaction was then incubated at 72°C for 7 minutes.

Exon 2 of the gene encodes bases 109-296 of the 88 kDa component of MTP RNA. The primers (SEQ. ID. NOS. 18 and 19) used to amplify exon 2 of the gene encoding the 88 kDa component of MTP from subject genomic DNA are shown below.

| Primer Pair | SEO. ID. NO. |
|---|---|
| CCCTTACAATGAAAACTGG | 18 |
| GGTACACTTCTCCAAAAACTT | 19 |

These primers were designed based on the normal human DNA sequence encoding the 88 kDa component of MTP. The primers are complementary to the introns flanking the 188 bp exon 2 so that the entire exon is amplified in the PCR reaction. The amplification product size, including the primers and flanking intronic regions, is 292 bp long.

B. Sequencing of PCR products

The PCR products obtained from both RNA- and DNA-PCR were electrophoresed on a 1.4% agarose gel in TAE buffer (40 mM Tris-acetate, 1 mM EDTA pH 8.0). The gel was stained for 5 minutes in 0.5 mg/mL ethidium bromide in water, and destained in water for 10 minutes. The DNA was visualized on an ultraviolet light box. The bands containing the desired PCR product were excised with a razor blade, and the DNA was purified by the GeneClean method (Bio 101, La Jolla, CA). The DNA was eluted from the silica matrix in 20 μL of distilled water. Each PCR reaction yielded approximately 1 μg of the desired DNA fragment. A portion of the purified DNA was sequenced directly by Taq polymerase cycle sequencing on an Applied Biosystems, Inc., 373 Automatic Sequencer, as described by Tracy and Mulcahy, Biotechniques, 11, 68 (1991).

The remaining DNA was prepared for cloning into a plasmid vector by producing blunt-ends with T4 DNA polymerase followed by phosphorylation with T4 polynucleotide kinase. DNA (500 ng) was added to a 50 μL reaction mixture containing 20 μM each dNTP, 1 mM ATP, 4.5 units T4 DNA polymerase, 5 units T4 polynucleotide kinase in 50 mM Tris HCl pH 7.5, 10 mM magnesium chloride, 1 mM dithiothreitol, and 50 μg/mL BSA. Incubation was at 37°C for 1 hour. The DNA was then purified from the reaction mixture by GeneClean. The DNA was eluted in 10 μL $dH_2O$. The blunt-ended DNA was ligated into pUC18 cut previously with Sma I and dephosphorylated (Pharmacia). Dh5α cells (100 μL, Gibco-BRL) were transformed according to the protocol supplied by the manufacturer. Plasmid DNA was amplified and isolated by the alkaline lysis procedure described in Molecular Cloning (Sambrook, Fritsch, and Maniatis, eds.) Cold Spring Harbor Laboratory Press, 1.25-1.28 (1989). The plasmid clones were sequenced as described in Example 1.

Results

Direct sequence from three independent RNA-PCR reactions revealed a deleted cytosine at base 262 of the cDNA relative to the start site of translation in the abetalipoproteinemic subject. The one base deletion shifts the reading frame and leads to a stop codon (TGA) 21 bases downstream. Translation of the mutant RNA would terminate at amino acid residue 78. Below is a comparison of the normal and the abetalipoproteinemic subject's DNA and deduced amino acid sequences

```
Base 255                                        287
     AGG AAT CCT GAT GGT GAT GAT GAC CAG TTG ATC Normal
AA    R   N   P   D   G   D   D   D   Q   L   I
```

```
Base 255                                          286
     AGG AAT C-TG ATG GTG ATG ATG ACC AGT TGA TG Abeta
AA    R   N    L   M   V   M   M   T   S  STOP
```

(SEQ. ID. NOS. 20 to 23, respectively).

Direct sequence analysis of 2 independent PCR amplifications of genomic DNA showed the deletion. This indicates that both alleles of the gene encoding the 88 kDa component of MTP in this subject exhibits the frameshift mutation. In addition, the DNA fragments were cloned into pUC18 for sequencing. Eight plasmid clones also exhibit the deleted cytosine further confirming the frameshift mutation on both alleles.

**Demonstration of a gene defect in a second abetalipoproteinemic subject**

A. Methods

Genomic DNA was isolated from blood from a second abetalipoproteinemic subject using Qiagen (Chatsworth, Ca) kit no. 13343, following the manufacturer's protocol. Like the first subject, we have previously demonstrated that neither MTP activity nor the 88 kDa component of MTP could be detected in intestinal biopsies from this subject. Three hundred μg of this genomic DNA was sent to Stratagene (La Jolla, CA) to be made into a genomic DNA library in the lambda DASH™ Vector(Stratagene). In addition, a normal genomic library in the lambda DASH™ vector was purchased from Stratagene (catalogue no. 943202).

Two million independent recombinant phage plaques from each library were screened for genomic DNA inserts containing sequences homologous to bovine MTP cDNA. The screening process was similar to that for the cDNA library screen in Example 1 except that the E. coli host strain was PLK 17, hybridization and wash temperatures were at 60°C, and the wash buffer was 1 X SSC, 0.1% SDS. The probe for the genomic library screen was the 2.4 kb Eco RI fragment from the bovine cDNA clone no. 22, $^{32}P$-labeled exactly as in example 2. Putative positive clones (about 30 from each library) were rescreened and remained positive through two additional rounds of hybridization analysis. Following the tertiary screen, single, isolated positive plaques were excised from the agar plates and deposited into 1 mL of SM buffer with 50 μL chloroform. Phage titer was amplified for each phage stock following the "Small-scale liquid cultures" protocol from Sambrook, et al., supra, p 2.67. One hundred μL of the amplified stocks was mixed with 100 μL of prepared PLK 17 plating cells and 100 μL of 10 mM magnesium chloride, 10 mM calcium chloride and incubated at 37°C for 15 minutes. This mixture was then used to inoculate 50 mL 2X NZY (Bethesda Research Laboratories) with 0.2% Casamino Acids (CAA, Fisher Scientific no. DF0288-01-2) and grown overnight at 37°C. Lambda DNA was isolated from the lysed cultures using the Qiagen kit no. 12543 using Qiagen buffers and protocol.

Direct DNA sequencing of the genomic DNA inserts was performed as described in Example 1 using lambda DNA as template. Oligonucleotides of about 20 bases, complementary to human cDNA sequence, were used as primers for sequencing normal or abetalipoproteinemic genomic clones. Characterization and sequencing of abetalipoproteinemic and normal genomic clones were performed in parallel (see Example 9). Intron-exon boundaries were identified by comparing genomic and cDNA sequences. Sequencing primers were designed against intron sequences 5' and 3' to each exon and used to confirm intron/exon boundaries by resequencing the boundaries. In addition, the coding sequence of both DNA strands for each exon of at least one abetalipoproteinemia genomic clone was sequenced. DNA sequence analysis of exon 13 of the abetalipoproteinemic subject revealed a C-to-T point mutation at base 1830 of the human cDNA. This base change introduces a stop codon at a site that normally encodes the amino acid residue $Arg_{595}$.

The nucleotide sequence around base 1830 encodes a Taq I endonuclease restriction site (TCGA) in the normal DNA sequence but not in the abetalipoproteinemic subject's DNA sequence (TTGA). To confirm this nucleotide change and address homozygosity of this allele, Taq I digests were performed on genomic DNA from a normal control, the abetalipoproteinemic subject and both parents of the abetalipoproteinemic subject. Genomic DNA was isolated from blood from a normal control, the abetalipoproteinemic subject and the abetalipoproteinemic subject's mother and father as described above. Ten μg of genomic DNA from each sample was digested with 100 units of Taq I (Bethesda Research Laboratories) in 100 μL 1 X REact buffer no. 2 (Bethesda Research Laboratories) at 65°C for 5 hours. Each digestion reaction was spun at 2,000 rpm in an Ultrafree-MC 10,000 NMWL filter unit (no. UFC3 TGC 00 from Millipore) with a molecular weight cut-off of 10,000, for 30 minutes to reduce the reaction volume to 50 μL. The restriction digest reactions were then subjected to agarose gel electrophoresis through a 1 % gel in TEA buffer at 20 volts for 16 hours. The agarose gel was stained with ethidium bromide, photographed, and then transferred to a nitrocellulose membrane by the method of Southern. E.M. Southern, J. Mol. Biol. 98, 503-17 (1975).

The probe for the Southern hybridization was a PCR product containing exon 13 and some flanking intron sequences (see SEQ. ID NO.24, below). The PCR was performed using the GeneAmp Kit (Perkin-Elmer, Cetus Industries) components and protocol with 0.3 µg normal genomic DNA as template and 10 picomoles each of the forward and reverse primers in a 100 µL reaction volume. The reaction mix was incubated at 97°C for two minutes, then subjected to 30 cycles consisting of 94°C for 30 seconds, 45°C for 30 seconds, and 72°C for 1 minute, followed by one 7-minute incubation at 72°C and storage at 4°C. The amplified DNA was subjected to electrophoresis through agarose as in example I and the expected 302 bp fragment was excised and eluted from the gel. This exon 13 PCR product was then $^{32}P$-labeled as in example 2 and used as a probe for the Southern hybridization. Hybridization and wash conditions were as in example 2. The blot was exposed to X-ray film at -80°C for 5 days.

B. Results

A human genomic library was generated from DNA isolated from a second abetalipoproteinemic subject. Two million phage were probed with a bovine cDNA probe and thirty phage with human genomic DNA inserts homologous to the bovine MTP cDNA were characterized.

DNA sequence analysis of the genomic DNA inserts from the abetalipoproteinemic subject revealed a C-to-T point mutation at base 1830 in exon 13 of the human MTP gene (exon 13 corresponds to bases 1817 to 1914 of the human cDNA). This C-to-T point mutation changes the normal CGA arginine codon at residue 595 to a TGA translational stop signal, resulting in a 300 amino acid truncation of this protein. This nucleotide change was found on all four independent genomic DNA inserts characterized from this individual.

Shown below is the position of the C-to-T mutation in exon 13 of an abetalipoproteinemic subject. The 302 base DNA sequence of the normal exon 13 with flanking intron sequence is shown. DNA corresponding to the forward (-->) and reverse (<--) PCR primers used to make the probe for the Southern hybridization are indicated above the appropriate arrows. Horizontal lines represent the intron/exon boundaries. The Taq I recognition sequence is boxed. An asterisk (*) designates base 1830, the site of the C-to-T mutation.
SEQ. ID. NO. 24.

```
ATTTGGCTTC CTCTTTTTTC CACTGAGGAT TTTTTTTTCC AAATTTGACT    50
TGGGAAACAG TCATTACAAT GAATGTGCAG CTTTTTTTTT CCTCATATGT   100
TGCAGCAAAA TTGTCCGTCG AGTTCTGAAG GAAATGGTCG CTCACAATTA   150
INTRON EXON
TGACCGTTTC TCCAGGAGTG GATCTTCTTC TGCCTACACT GGCTACATAG   200
AACGTATGTA CACCAAAAAG AGGTTCTCCT TCCATACCCC ACAACTTAGC   250
EXON INTRON
ATTGCTGGAA CTGCTATTAA ATTACAGTTA TAGTGTGTCA TCAGGTAGTC   300
CC                                                       302
```

The normal nucleotide sequence surrounding the C at base 1830 (TCGA) encodes a Taq I restriction site. In this abetalipoproteinemic subject, the sequence at this site is mutated (TTGA). Therefore, Taq I should cut exon 13 at this site in normal DNA, but not in DNA which contains the mutation. There is only one Taq I site in the normal exon 13.

A Southern blot confirms this nucleotide change (Figure 9). The genomic DNA isolated from a control subject, the abetalipoproteinemic subject, and the subject's mother and father was cut to completion with Taq I and probed with sequences from exon 13. The normal DNA is cut by Taq I into two pieces which hybridize to exon 13; the abetalipoproteinemia DNA is not cut with Taq I, evidenced by only one hybridizing band. This result confirms the lack of a Taq I recognition sequence. The DNA from both parents exhibits a mixed pattern, demonstrating the presence of one normal allele and one mutated allele.

C. Analysis

The foregoing results and the conclusions drawn from them can be summarized as follows.

MTP activity and protein are undetectable in the abetalipoproteinemic subjects studied. Mutations in the MTP gene fully explain the lack of protein and activity. Previous results demonstrate that abetalipoproteinemia is a monogenetic disease Kane & Havel, supra. From these results, one can conclude that abetalipoproteinemia is caused by a loss of MTP activity.

These results demonstrate that MTP activity is required for the efficient assembly and secretion of lipoprotein particles which contain apolipoprotein B. Loss of MTP activity results in lower serum levels of cholesterol, triglycerides, phospholipids, and cholesterol esters. One can thus conclude that a decrease in the amount of activity of MTP will result in lower serum lipid levels.

Moreover, lower serum lipid levels are associated with prevention, stabilization, or regression of atherosclerosis. As discused above, loss of the amount or activity of MTP results in lower serum lipid levels. In addition, abetalipoproteinemic subjects lack atherosclerosis. Schaefer, supra; Dische and Porro, Am. J. Med., 49, 568-71 (1970); and Sobrevilla et al., Am. J. Med., 37, 821 (1964). One can thus also conclude that inhibition of MTP will result in the prevention, stabilization, or regression of atherosclerosis.

The following examples further illustrate the present invention. These examples are not intended to limit the scope of the present invention, and may provide further understanding of the invention.

**Example 1**

**Isolation and DNA Sequence Analysis of cDNA Clones Encoding the 88 kDa Component of the Bovine MTP**

A commercially available bacteriophage lambda gt10/bovine small intestine cDNA library was purchased from Clontech. 1 X $10^6$ independent recombinant phage plaques were screened for the cDNA corresponding to the 88 kDa component of bovine MTP.

An E. coli bacteria host, strain C600 (Clontech), was prepared for phage infection by growing overnight to saturation at 30˚C in 50 mL of Luria Broth (LB = 10 g sodium chloride, 10 g Bacto-Tryptone and 5 g Yeast Extract per liter) supplemented with 0.2% maltose and 10 mM magnesium sulfate. The cells were pelleted by low speed centrifugation, resuspended in 20 mL of 10 mM magnesium sulfate and stored at 4˚C. Twenty aliquots each of 50,000 phage and 300 μL of the C600 cells were incubated at 37˚C for 15 minutes, mixed with 7 mL LB + 0.7% agarose and plated on 132 mm LB Plates. The plates were incubated for 7-10 hours at 37˚C until distinct phage plaques appeared, then transferred to 4˚C.

Duplicate plaque transfers to nitrocellulose membranes were performed for each plate as follows. A nitrocellulose membrane (Schleicher & Schuell, Keene, NH) was placed directly on the phage for 1 minute (first transfer) or 3 minutes (second transfer). The phage DNA adhering to the membrane was then denatured for 1 minute in 0.5 N sodium hydroxide, 1.5 M sodium chloride, neutralized for 1 minute in 1 M Tris, pH 8.0, 1.5 M sodium chloride, and finally washed for 1 minute in 2 X SSC ( 1 X SSC = 0.15 M sodium chloride, 0.015 M sodium citrate, pH 7.0). The DNA was then permanently fixed onto the nitrocellulose membrane by baking in an 80˚C vacuum oven for 2 hours.

The isolation of bovine MTP, including the 88 kDa component, has been previously described. See, Wetterau and Zilversmit, Chem. Phys. Lipids 38, 205-72 (1985); Wetterau et al., J. Biol. Chem. 265, 9800-7 (1990). The sequences of internal peptides of the 88 kDa component were used to design oligonucleotides which would hybridize to cDNA that encodes the protein. See Lathe, R., J. Mol. Biol. 183, 1-12 (1985).

The procedures described herein employed probes having the following DNA sequences (listed 5' to 3'):

| Probe | Sequence | SED ID. NO. |
|---|---|---|
| 2A | CTCTACCAGCGAGTATTAAT<br>  T      C  G  G  G | 25 |
| 37A | ACGTAGGATGTCTTGGACAATGGAGAGCATGTA | 26 |
| 19A | GATCAGTTGGTTATCATCACCATCAGGACT | 27 |

Probe 2A is a mixture of thirty-two twenty base oligonucleotides, each encoding the amino acid sequence of the peptide from which this probe was designed. Probe 37A is a unique 33 base sequence and probe 19A is a unique thirty-mer. These oligonucleotide sequences encode amino acid sequences that correspond to internal peptides.

Oligonucleotides were obtained from commercial sources as indicated herein or synthesized on a Milligen/ Biosearch (Millipore Corp., Bedford, MA) 8700 DNA Synthesizer using beta-cyanoethyl phosphoramidite chemistry. Sequencing primers were desalted on NAP-10 columns (Pharmacia LKB Biotechnologies, Inc., Piscataway, NJ) prior to use. Probes were purified on NENSORB Prep Resin (DuPont Company, NEN Research Products, Boston, MA).

Probe 2A was purchased from Genosys Biotechnologies, Inc. (The Woodlands, Texas) and was labeled by

incubating 1 μg of the oligonucleotide in 50 mM Tris-Cl, pH 7.5, 10 mM magnesium chloride, 5 mM dithiothreitol (DTT), 0.1 mM ethylenediaminetetraacetate (EDTA), and 0.1 mM spermidine with 10 units T4 polynucleotide kinase and 120 μCi of gamma labeled $^{32}$P-ATP in a 50 μL reaction volume at 37°C for 30 minutes followed by heat inactivation of the kinase at 68°C for 5 minutes. Unreacted ATP was removed utilizing a G-25 Sephadex spin column (Boehringer Mannheim Corp., Indianapolis, IN) following the manufacturer's instructions. The labeled oligonucleotide had a specific activity of approximately 2 X $10^8$ dpm/μg.

The nitrocellulose membranes were prehybridized for 2 hours at 37°C in 150 mL of hybridization buffer (6 X SSC, 20 mM NaPO4, 2 X Denhardts, 0.1% SDS, and 100 μg/mL salmon sperm DNA) (See, Sambrook et al., supra, p. B15 for Denhardts). The hybridization buffer was replaced and the labeled oligonucleotide probe 2A was added and allowed to hybridize overnight at 37°C. The membranes were washed in 1 liter of 2 X SSC, 0.1% SDS at 40°C, air-dried, and exposed to Kodak XAR-5 X-ray film for 5 days at -80°C, with a Dupont lightening plus intensifying screen (Dupont, NEN).

Putative positive clones (40) were rescreened with the same probe through two subsequent rounds of hybridization. Agar plugs corresponding to positive signals on the X-ray films were excised from the original plates and placed in 1 mL SM + 5% CHCl3 (SM = 5.8 g sodium chloride, 2.0 g magnesium sulfate, 50 mL 1 M Tris-Cl pH 7.5, and 5 mL 2% gelatin per liter). The phage were replated by mixing 0.001 μL of phage stock with 100 μL C600 cells in 10 mM magnesium sulfate, incubating at 37°C for 15 minutes, adding 3 mL LB + 0.7% agarose and plating onto 82 mm LB plates. After overnight incubation at 37°C followed by 1 hour at 4°C, the phage plaques were transferred to nitrocellulose, and reprobed as above to labeled oligonucleotide probe 2A. Following the tertiary hybridization screen, 16 phage plaques were isolated.

The inserts of each of the 16 recombinant phage were amplified by PCR using the commercially available lambda gt10 amplimers (Clontech) and the GeneAmp Kit (Perkin-Elmer, Cetus Industries, Norwalk, CT) following the manufacturer's protocols exactly. The amplified DNA was subjected to electrophoresis through 1.2% agarose gels in Tris-EDTA-Acetate (TEA = 40 mM Tris-Acetate, 1 mM EDTA) buffer, for 2-3 hours at 100 volts. The agarose gels were then stained in ethidium bromide (EtBr), rinsed in water and photographed. The DNA was then transferred from the gel to a nitrocellulose membrane by the method of Southern. A Southern hybridization was performed using labeled oligonucleotide probe 2A in 50 mL hybridizaiton buffer (above) at 40°C overnight then washing at 45°C, 48°C and 51°C. Two amplified inserts, corresponding to phage no. 64 and no. 76 (Figure 1), hybridized to probe 2A at 51°C in 2 X SSC. Lambda DNA of these 2 clones was prepared following the plate lysate procedure (Sambrook, et al., supra, p. 2.118). One-tenth (5 mL of 50ml) of the phage DNA was digested with 20 units of the restriction enzyme Eco RI (New England Biolabs, Beverly, MA) in the manufacturer's buffer at 37°C for 2 hours and subjected to agarose gel electrophoresis. Upon EcoRI cleavage of these phage, no. 64 yielded a 1.0 kb insert fragment and the cDNA from phage no. 76 yielded two EcoRI pieces, of 0.9 kb and 0.4 kb. These bands were excised from the gel.

DNA was eluted from the agarose gel slices by first forcing the gel slices through a 21 gauge needle into 3 mL of $T_{10}E_1N_{.3}$(10 mM Tris-Cl pH 7.4, 1 mM EDTA pH 8.0 and 0.3 M sodium chloride) and freezing at -20°C overnight. The samples were then thawed at 37°C for 30 minutes, centrifuged to pellet the agarose, diluted 1:1 with water and passed through an Elu.Tip column (Schleicher & Schuell) following the manufacture's protocol. The DNA samples were then ethanol precipitated, ethanol washed, and resuspended to an approximate concentration of 0.05 pmoles/μL.

The plasmid vector bluescript SK+ (Stratagene) was prepared to receive the cDNA inserts by digestion with 20 units of the restriction endonuclease Eco RI (New England Biolabs) in the manufacturer's buffer at 37°C for 2 hours, followed by a 30 minute treatment with 1 unit of calf alkaline phosphatase (Boehringer-Mannheim) which is added directly to the Eco RI reaction. This DNA was then electrophoresed through a 1.2% agarose/TEA gel at 100 volts for 2 hours. The linear plasmid band was excised, eluted and resuspended as above.

cDNA insert fragments were ligated into the prepared bluescript plasmid vector by mixing 0.05 pmole of vector with 0.10 pmoles of cDNA insert in 50 mM Tris-Cl pH 7.4, 10 mM magnesium chloride, 1 mM DTT, 1 mM ATP, and 40 units T4 DNA ligase (New England Biolabs). The 10 μL reaction was incubated at 15°C overnight. The ligation reaction was then mixed with 100 μL of transformation competent E. coli cells, strain DH5a (Bethesda Research Laboratories), and the plasmid DNA transformed into the E coli cells following the standard protocol of Sambrook et al., supra, p. 1.74. Transformed cells were plated on LB-agar plates containing 100 μg/mL ampicillin and grown overnight at 37°C.

Plasmid DNA was isolated from ampicillin resistant colonies following the alkaline lysis procedure of Bimboin and Doly [Nucleic Acids Res. 7, 1513-23 (1979)]. The purified plasmid DNA was digested with Eco RI as above, subjected to agarose gel electrophoresis and analyzed for the generation of the correct size Eco RI cDNA insert fragment. Cells from a unique colony positive for a cDNA insert were used to innoculate 100 mL of LB containing 100 μg/mL ampicillin and grown to saturation at 37°C. Plasmid DNA was extracted using a Qiagen plasmid isolation kit no. 12143(Qiagen, Inc., Chatsworth, CA) following the manufacturer's protocol.

Sequencing of cDNA clones was performed with the Applied Biosystems, Inc. (ABI, Foster City, CA) 373 Automated DNA Sequencer utilizing either dye-labeled primers or dye-labeled dideoxynucleotides. Cycle sequencing with dye-labeled primers was performed with Taq Dye Primer Cycle Sequencing Kits (ABI part nos. 401121 and 401122).

One μg of double-stranded DNA was used per reaction. Methods used for cycling and concentration of sequencing samples were as described in the Cycle Sequencing of DNA with Dye Primers manual (ABI part no. 901482). Alternatively, cycle sequencing with dye-labeled dideoxynucleotides was performed using the Taq Dye-Deoxy™ Terminator Cycle Sequencing Kit (ABI part no. 401113). Typically, 1.25 μg of template with 4 pmol of primer was used per reaction. The template and primer concentrations were varied as necessary to optimize sequencing reactions. Cycling of reactions was performed using a Perkin-Elmer Cetus thermal cycler (model 9810) as described in the Taq Dye Deoxy™ Terminator Cycle Sequencing Protocol (ABI part no. 901497).

Following the cycle reactions, Centri-Sep™ spin columns (Princeton Separations, Adelphia, NJ) were used to remove excess dye terminators and primers. Spin column eluants were then precipitated and washed as described in the Taq Dye Deoxy™ Terminator Cycle Sequencing Protocol (ABI part no. 901497). A 6% acrylamide denaturing gel was prepared as described in the ABI 373A DNA Sequencing System User's Manual. Just prior to running the gel, samples were resuspended in 5 μL of deionized formamide/50 mM EDTA (pH 8.0) 5/1 (v/v). Samples were denatured at 90°C for two minutes, cooled quickly on ice, then loaded onto a pre-run gel (gel was prerun for approximately 15-20 minutes). The gel was run for 12 hours at the following settings: 2500 volts, 40 amps, 30 watts, 40°C. Sequence analysis was performed with ABI 373A DNA Analysis software (version 1.0.2). Final sequence was obtained using ABI DNA Sequence Editor software seqEd™ (version 1.0) ABI, Inc..

The entire 1036 bp insert of clone no. 64 was sequenced. It encoded 936 bp of open reading frame continuing through the 3 prime end of the insert (corresponding to a polypeptide with a molecular weight of at least 34,000). Comparison of the sequence of this clone to available sequence in nucleotide sequence data banks revealed that the first 91 bases at the 5' end of the clone corresponded to the bovine mitochondrial genome. Therefore, the 1036 bp insert of clone no. 64 resulted from the ligation of two independent cDNAs during the construction of this library.

The 400 bp EcoRI fragment of clone no. 76 was sequenced entirely indicating 81 bp of open reading frame followed by 298 bases of 3 prime untranslated sequence and a poly A region.

The lambda gt10 bovine small intestine cDNA library was rescreened with an oligonucleotide probe 37A, an exact 33 bp match to the 5' most peptide sequence encoded by clone no. 64. Two positive clones, no. 22 and no. 23 (Figure 1) were isolated through tertiary screens, subcloned and sequenced as for clone no. 64.

Clones no. 22 and 23 contained 2.8 kb and 1.7 kb cDNA inserts respectively. The 2.8 kb cDNA insert of clone no. 22 predicted a continuous open reading frame of 835 amino acids between bases 2 and 2506 (corresponding to a 93.2 kDa polypeptide), followed by 298 base of 3' untranslated sequences and a poly A region.

The lambda gt10 library was rescreened with probe 19A, an exact match to the sequence of clone no. 22 corresponding to the 5'-most peptide encoded by that clone, and clone no. 2 was isolated as above. DNA sequence analysis of the 1 kb cDNA insert from clone no. 2 indicated it overlapped clone no. 22 and extended the 5' end of the bovine cDNA by 100 bases. A composite of the DNA sequences of clones no. 2 and no. 22 and the predicted translation product is shown in SEQ. ID. NOS. 1 and 3, respectively.

In summary, sequencing of bovine small intestine cDNA clones corresponding to the 88 kDa component of MTP yielded 2900 bp of continuous sequence which encodes an open reading frame of 860 amino acids followed by a 298 bp 3' noncoding region and a poly A region. The predicted protein product of this composite sequence is 96.1 kDa.

## Example 2

### DNA Hybridization Analysis of Related Species

Southern hybridization analysis was performed on DNAs from cow, human, mouse, hamster (Chinese hamster ovary or CHO cells), rat, and dog. 10 μg of each genomic DNA (Clontech) was digested with 140 units of Eco RI (New England Biolabs) in 100 μL 1 X Eco RI buffer (New England Biolabs) at 37°C, overnight. Each digestion reaction was spun at 2,000 rpm in a Ultrafree-MC 10,000 NMWL filter unit (no. UFC3 TGC 00 from Millipore) with a molecular weight cut-off of 10,000, for 30 minutes to reduce the reaction volume to 50 μL. The restriction digest reactions were then subjected to agarose gel electrophoresis through a 0.75% gel in TEA buffer at 80 volts for 3 hours. The agarose gel was stained with ethidium bromide, photographed, and then transferred to a nitrocellulose membrane by the method of Southern.

A Southern hybridization was performed using the 2.4 kb Eco RI fragment from the bovine cDNA clone no. 22 as a probe. Twenty-five ng of the DNA fragment was labeled using the Multiprime DNA Labelling System (Amersham Corp., Arlington Heights, IL) and 50 μCi of $^{32}$P-α-dCTP. Unincorporated $^{32}$P was separated from the labeled probe using a Sephadex G25 spin column as above. The nitrocellulose membranes was prehybridized in 100 mL hybridization buffer (above) at 37°C for 2 hours. The hybridization was performed overnight in 50 mL fresh hybridization buffer at 60°C with 1.2 X $10^7$ dpm denatured probe. The membrane was washed in 500 mL 1 X SSC, 0.1% SDS at 65°C for 1 hour, air-dried, and then exposed to X-ray film at -80°C with an intensifying screen for 4 days. The 2.4 kb Eco RI fragment from bovine clone no. 22 specifically hybridized to at least two DNA bands in every species tested. Therefore, it was concluded

that the hybridization conditions established for the bovine cDNA probe allows detection of homologous DNAs from other species, such as human, mouse, hamster, rat and dog.

## Example 3

### Isolation and DNA Sequence Analysis of cDNA Clones encoding the 88 kDa Component of Human MTP

### A. Cloning and Sequence Analysis

To obtain the full coding sequence of the 88 kDa component of human MTP, a human liver cDNA library was screened with a bovine MTP cDNA insert described herein above. The library was obtained from Stratagene. It contained oligo dT primed liver cDNA directionally cloned (EcoRI to XhoI) into the lambda ZAP vector. The probe was obtained by digestion of 10 $\mu$g of bovine intestinal clone no. 22 above in universal buffer (Stratagene) with 50 units of EcoRI, electrophoresis at 80-150 volts through a gel consisting of 0.9% low melting point agarose (Bethesda Research Laboratories, Gaithersburg, MD), TAE (40 mM Tris acetate, 1 mM EDTA), and 0.5 $\mu$g/mL ethidium bromide. The resulting 2.4 Kb fragment was purified by phenol extraction as described in Sambrook et al., supra, p. 6.30. The purified fragment was then radiolabelled with a multiprime DNA labelling kit and alpha 32p dCTP (Amersham) to 109 cpm/$\mu$g using the manufacturer's instructions. Unincorporated $^{32}$P was separated from the labeled probe using a Sephadex G-25 spin column as above.

$10^6$ plaques from the library were screened as follows according to the manufacturer's instructions (Stratagene). *E. coli* bacteria, strain XL 1 Blue (Stratagene), were grown with shaking overnight at 37˚C in 50 mL LB broth (Bethesda Research Laboratories) supplemented with 0.2% maltose and 10 mM magnesium sulfate. The cells were sedimented by low speed centrifugation and then resuspended in 10 mM magnesium sulfate to an $OD_{600}$= 0.5 and stored at 4˚C. Phage were diluted to a concentration of 50,000 plaque forming units/25 $\mu$L SM buffer. For each plate, 600 $\mu$L of bacteria, and 25 $\mu$L of phage were mixed and incubated at 37˚C for 15 minutes. Top agar (6.5 mL) consisting of NZY broth (Bethesda Research Laboratories), 0.7% agarose (Bethesda Research Laboratories) preheated to 50˚C, was added to the bacteria and phage mixture, and then plated onto a 150 mm NZY plate. The top agar was allowed to solidify and the plates were incubated overnight at 37˚C.

The plates were then cooled to 4˚C for 2 hours and the plaques were lifted onto nitrocellulose filters. Duplicate lifts were performed in which the alignment of the membranes relative to the plate were recorded by placing needle holes through the filter into the agar plate. The filters were incubated 1 minute in 0.5 N sodium hydroxide, 1.5 M sodium chloride, 1 minute in 1 M Tris, pH 8.0, 3 M sodium chloride, and 1 minute in 2 x SSC. Filters were then baked at 80˚C in a vacuum chamber for 2 hours. The filters were incubated for 2 hours at 60˚C in 5 mL per filter of hybridization buffer (6 X SSC, 20 mM $NaPO_4$, 2 X Dendardts, 0.1% SDS, and 100 $\mu$g/mL salmon sperm DNA). The buffer was replaced with an equal volume of hybridization buffer containing the probe at a concentration of 3.5 x $10^6$ cpm per filter and incubated overnight at 60˚C. The filters were washed in 1 X SSC, 0.1% SDS first at room temperature and then at 50˚C for 2 hours. Autoradiography revealed 56 positives.

A small plug of agarose containing each positive was incubated overnight at 4˚C with 1 mL of SM buffer and a drop of chloroform. The positive phage were purified by replating at a low density (approximately 50 - 500 per 100 mm plate), screening and isolating single positive plaques as described above.

When XL1 Blue cells are infected with the ZAP vector (Stratagene) and coinfected with a helper phage, the bluescript part of the vector is selectively replicated, circularized and packaged into a single stranded phagemid. This phagemid is converted to a double stranded plasmid upon subsequent infection into naive XL1 Blue cells. The cDNA insert of the resultant plasmid can be sequenced directly. Plasmids containing the positive human liver cDNA inserts were excised in this manner utilizing the helper phage provided by Stratagene according to the manufacturer's directions.

DNA from these clones was purified as follows. A single colony was inoculated into 2 mL of LB and incubated with shaking at 37˚C overnight. 1.5 mL of this was centrifuged and resuspended in 50 $\mu$L of LB. 300 $\mu$L of TENS (1 X TE, 0.1 N sodium hydroxide, 0.5% SDS) was added and vortexed for 5 seconds. 150 $\mu$L of 3 M sodium acetate, pH 5.2 was added and vortexed for 5 seconds. The samples were then spun in a microfuge for 10 minutes. The supernatant was recovered, 0.9 mL of ethanol was added and the samples were spun in a microfuge for 10 minutes. The pellet was washed in 70% ethanol, dried, and resuspended in 20 $\mu$L of TE (10 mM Tris pH 7.4, 1 mM EDTA pH 8).

The DNA from the clones was characterized as follows. Five $\mu$L of the DNA from each clone were digested with 10 units Eco RI, 10 units XhoI, and 10 $\mu$g RNAse, and then fractionated and visualized by electrophoresis through a 1% agarose, TBE (45 mM Tris-Borate, 1 mM EDTA), 0.5 $\mu$g/mL ethidium bromide gel. A Southern blot of the gel was performed as described in Sambrook et al., supra, p. 9.41. This Southern blot was probed with a fragment of the bovine cDNA near the 5' end of the coding sequence. This 5' probe was prepared by digesting 25 $\mu$g of bovine intestinal clone no. 2 above with 50 units EcoR1 and 50 units of NheI, isolating as above the 376 base pair fragment from a 2% low melting point agarose, TBE, 0.5 ug/mL ethidium bromide gel, and radiolabelling as described above. The results are as

follows: Clone no. 693, 3.7 kB insert, hybridizes with the 5' probe; Clone no. 754, 1.2 kB insert, hybridizes with the 5' probe; Clone no. 681, 1.8 kB insert, does not hybridize with the 5' probe.

Overnight cultures containing these three clones were grown in 200 mL of LB with 100 μg/mL ampicillin. Large amounts of plasmid were purified using a Qiagen plasmid maxiprep kit according to the manufacturer's instructions. The sequence of clone no. 693 reveals that it contained two inserts. The 5' 500 bp insert was homologous to haptoglobin and will not be discussed further. This was followed by a mutant XhoI and an EcoRI restriction site (the two sites used in the directional cloning). The 3' insert was the cDNA of interest. It contained some 5' untranslated sequence as indicated by the stop codons in all three reading frames. At bases 48 - 2729 there is an ATG-initiated open reading frame corresponding to 894 amino acids. The deduced amino acid sequence begins

M I L L A V L F L C F I

(SEQ. ID. NO. 28). The stop codon is found at bases 2730 - 2732 followed by a 3' untranslated region of 435 bases and a poly A region. The sequence of clone no. 681 confirmed the 3' 1768 bases of this clone, and clone no. 754 confirmed bases 1 through 442.

**B. Tissue Localization of the 88 kDa mRNA**

A MultiTissue Northern Blot (Clontech) contained 2 μg per lane of polyA+ RNA from human heart, brain, placenta, lung, liver, skeletal muscle, kidney or pancreas. Northern hybridization was performed as for the genomic Southern blot. Prehybridization was in 50 mL hybridization buffer at 37°C for 2 hours followed by an overnight hybridization in 20 mL fresh buffer at 60°C with 5.2 x $10^7$ dpm labeled 2.4 kb Eco RI fragment from the bovine intestinal clone no. 22 as above. The Northern blot was washed in 500 mL 0.2 X SSC, 0.1% SDS at 60°C, 1 hour and subjected to autoradiography at -80°C. After a 20 hour exposure to X-ray film there is a predominant signal in the liver RNA lane at about 4.4 kb and no other detectable hybridization. Therefore, cross hybridization of the 2.4 kb fragment of the bovine cDNA detects a human liver RNA specifically. As liver and intestine are the only two tissues in which significant MTP activity has been reported, the cloning and northern blot analysis support the biochemical localization for MTP. Also, the results of the northern analysis extend this detection to include DNA:RNA hybrids as well as DNA:DNA interactions.

**Example 4**

**Expression of MTP in Human Fibroblast Cell Line**

**I. Methods**

All standard molecular biology protocols were taken from Sambrook, supra, except where indicated below. All restriction enzymes used in this example were obtained from Bethesda Research Laboratories (BRL, Gaithersburg, MD). A 3.2 kb fragment extending from nucleotide -64 to 3135 (relative to the translation start site with A of the translation start site ATG codon designated +1), was constructed from plasmids p754 (bases -64 to 384) and p693 (bases 385 to 3135) as follows. A 448 bp EcoRI-NcoI restriction endonuclease fragment and a 2750 bp NcoI-XhoI restriction endonuclease fragment were excised from p754 and p693, respectively. Following gel purification, the fragments were ligated into EcoRI-XhoI cut plasmid pBluescript-SK to yield plasmid pBS/hMTP. The entire hMTP fragment was isolated from pBS/hMTP by restriction endonuclease digestion with HindIII and XhoI and was subcloned into plasmid pcDNA/Neo (Invitrogen,San Diego, CA) to yield plasmid pcDNA/MTP. This places the full-length hMTP coding sequence under the transcriptional control of the highly active Cytomegalovirus promoter.

Plasmids were transfected into 1508T [J. Biol. Chem. 267, 13229-38 (1992)] transformed human skin fibroblasts by the lipofectin reagent (BRL). Cells were split into 100 mm dishes at a density of 25% of confluency, 24 hours prior to transfection. At the time of transfection, 50 mg of plasmid per 100 mm plate were dissolved in 1.5 mL of serum-free Dulbecco's Modified Eagles Medium (DMEM) and added dropwise to a solution of 120 μL lipofectin reagent in 1.5 mL of serum free DMEM. After a 15-minute incubation at room temperature, the transfection mixtures were added to the 1508T cultures containing 7 mL of serum free DMEM. Twenty four hours later, the transfection mixtures were removed and 10 mL of fresh DMEM containing 10% fetal bovine serum was added for an additional 24 hours. Cells were scraped from the dish and washed twice with ice cold phosphate buffered saline (PBS). Cell extracts, MTP activity measurements and Western analyses were carried out as described in the foregoing "Assay for TG transfer activity in Abetalipoproteinemic subjects" herein.

## II. Results

The cDNA containing the full coding sequence for MTP was subcloned into expression vector pcDNA/Neo, yielding construct pcDNA/MTP. This plasmid was transiently expressed in 1508T transformed human skin fibroblasts [J. Biol. Chem. 267, 13229-38 (1992)] by liposome mediated transfection. Forty-eight hours after transfection, TG transfer activity was readily detectable above background levels assayed in extracts from cells transfected with the parent plasmid, pcDNA/Neo. Western blot analysis showed the presence of the the 88 kDa component of MTP in cells transfected with pcDNA/MTP but not in cells transfected with pcDNA/Neo. A comparison of the protein mass and activity in the transfected cells to that found in HepG2 cells suggests that the expressed MTP was efficiently incoporated into an active transfer protein complex with PDI.

## Example 5

### Screen for Identifying Inhibitors of MTP

In this screen, the rate of detectably labeled lipid (for example, NMR, ESR, radio or fluorescently labeled TG, CE, or PC) transfer from donor particles (e.g., donor membranes, vesicles, or lipoproteins) to acceptor particles (e.g., acceptor membranes, vesicles, or lipoproteins) in the presence of MTP is measured. A decrease in the observed transfer rate in the presence of an inhibitor of MTP (e.g., contained in a natural products extract or known compounds) may be used as an assay to identify and isolate inhibitors of MTP function. A variety of assays could be used for this purpose, for example, the synthetic vesicle assays previously published by Wetterau & Zilversmit, J. Biol. Chem. 259, 10863-6 (1984) or Wetterau et al., J. Biol. Chem. 265, 9800-7 (1990) or the assay outlined hereinabove in the "Assay for TG transfer activity in Abetalipoproteinemic subjects." An example of one such assay is as follows.

### A. Substrate Preparation

In a typical screen using labeled lipoproteins, labeling of lipoproteins with [$^3$H]-TG is accomplished by the lipid dispersion procedure described by Morton and Zilversmit [Morton, R.E. et al., J. Biol. Chem. 256, 1992-5 (1981)] using commercially available materials. In this preparation, 375 μCi of [$^3$H] triolein (Triolein, [9,10-$^3$H (N)]-, NEN Research Products, cat. no. NET-431), 1.5 mg of egg phosphatidylcholine and 160 μg of unlabeled triolein in chloroform are mixed and evaporated under a stream of nitrogen to complete dryness. Two mL of 50 m$\underline{M}$ Tris-HCl, 0.01% $Na_2$ EDTA, 1 m$\underline{M}$ dithiothreitol, pH 7.4, is added and the tube flushed with nitrogen. The lipids are resuspended by vortexing and the suspension is then sonicated for two 20-minutes intervals in a bath sonicator. The sonicated lipids are added to 75 mL rabbit plasma (Pel-Freez Biologicals, Rogers, AR) with 5.8 mL of 8.2 m$\underline{M}$ diethyl $\underline{p}$-nitrophenyl phophate (Sigma, Cat. No. D9286) and 0.5 mL of 0.4 $\underline{M}$ $Na_2$EDTA, 4% $NaN_3$. The plasma is then incubated under nitrogen for 16-24 hours at 37°C. Low density lipoproteins (LDL) and high density lipoproteins (HDL) are isolated from the incubation mixture and from control plasma which was not labeled by sequential ultracentrifugation [Schumaker & Puppion, Methods Enzymology 128, 155-170 (1986)]. Isolated lipoproteins are dialyzed at 4°C against 0.9% sodium chloride, 0.01% $Na_2$EDTA, and 0.02% $NaN_3$ and stored at 4°C.

### B. Transfer Assay

In a typical 150 μL assay, transfer activity is determined by measuring the transfer of radiolabeled TG from [$^3$H]-HDL (5 μg cholesterol) donor particles to LDL (50 μg cholesterol) acceptor particles at 37°C for three hours in 15 m$\underline{M}$ Tris, pH 7.4, 125 m$\underline{M}$ MOPS, 30 m$\underline{M}$ Na acetate, 160 m$\underline{M}$ NaCl, 2.5 m$\underline{M}$ $Na_2$ EDTA, 0.02% $NaN_3$, 0.5% BSA with about 50-200 ng purified MTP in the well of a 96-well plate. The material to be tested (e.g., natural product extracts in an assay compatible solvent such as ethanol, methanol or DMSO (typically, 5 μL of material in 10% DMSO is added) can be screened by addition to a well prior to incubation. The transfer is terminated with the addition of 10 μL of freshly prepared, 4°C heparin/$MnCl_2$ solution (1.0 g heparin, Sigma Cat. No. H3393 187 U/mg, to 13.9 mL, 1.5 $\underline{M}$ $MnCl_2$. 0.4% heparin (187 I.U.)/0.1 $\underline{M}$ $MnCl_2$) to precipitate the $^3$H-TG-LDL acceptor particles and the plate centrifuged at 800 x g. An aliquot of the supernatant from each well containing the [$^3$H]-TG-HDL donor particles is transferred to scintillation cocktail and the radioactivity quantitated. The enzyme activity is based on the percentage of TG transfer and is calculated by the following equation:

$$\text{Enzyme activity} = 1 - \frac{\text{[3H]-TG recovery (+ MTP)}}{\text{[3H]-TG recovery (- MTP)}} \times 100\%$$

In such an assay, the percent TG transfer will increase with increasing MTP concentration. An inhibitor candidate will decrease the percent TG transfer. A similar assay could be performed with labeled CE or PC.

**Example 6**

**Identification and Demonstration of the Activity of MTP inhibitors**

**I. Methods**

**A. Identification of MTP inhibitors**

Using the method outlined in Example 5, MTP inhibitor compounds A and B were identified. The assay measured the bovine MTP-catalyzed rate of transport of radiolabeled TG from donor HDL to acceptor LDL. In this method, an inhibitor decreases the rate of radiolabeled TG transfer.

The MTP-inhibiting activity of these compounds was confirmed in an independent assay following the procedures outlined in the foregoing "Assay for TG transfer activity in abetalipoproteinemic subjects." That assay measured the bovine MTP-catalyzed transport of radiolabeled TG from donor to acceptor SUV.

**B. Cell culture**

The human hepatoblastoma cell line, HepG2, was obtained from the American Type Culture Collection (Rockville, MD; ATCC accession no. 8065). Cultures were maintained at 37˚C in a 5% carbon dioxide atmosphere in T-75 culture flasks with 12 mL of RPMI 1640 medium containing 10% fetal bovine serum (all cell culture media and buffers were obtained from GIBCO Life Technologies, Gaithersburg, MD). Cells were subcultured 1:4 once a week and fed fresh medium 3 times a week.

Experiments to measure the effects of compounds A and B on protein secretion were carried out in 48-well plates. HepG2 cells were subcultured 1:2 and allowed to come to confluency at least 24 hours before drug treatment. Before commencement of drug treatment, culture medium was removed, the cells washed once with PBS and 1 mL of fresh medium was added quantitatively. Compound A was added to duplicate wells in 10 μL of dimethylsulfoxide (DMSO) to yield varying compound concentrations. DMSO alone (10 μL) was used as the negative control. (Note: DMSO at this concentration has negligible effect on HepG2 cells.) After a 16-hour incubation under standard cell culture conditions, the plates were centrifuged at 2,500 rpm for 5 minutes at 4˚C to sediment any loose cells. The media were diluted with cell culture medium 10 times for the apolipoprotein B (apoB) and human serum albumin (HSA) assays, and 20 times for the apolipoprotein AI (apoAI) assays. The cells were washed twice with cold PBS, and 0.5 mL of homogenization buffer was then added (0.1 M sodium phosphate, pH 8.0; 0.1% Triton X-100). The cells were homogenized by trituration with a 1 mL micropipettor, and protein was measured using the Coomassie reagent (Pierce Chemical Co, Rockford, IL) as described by the manufacturer.

**C. ELISA assays for ApoB and ApoAI and HSA**

The ELISA assays to measure protein mass were of the "sandwich" design. Microtiter plates were coated with a monoclonal antibody (primary antibody), specific for the protein of interest (Biodesigns International, Kennebunkport, ME), followed by the antigen or sample, a polyclonal antibody (secondary antibody) directed to the protein of interest (Biodesigns International), and a third antibody conjugated to alkaline phosphatase directed to the secondary antibody (Sigma Biochemical, St.Louis, MO). The 96-well microtiter plates (Coming no. 25801) were coated overnight at room temperature with 100 μL of diluted monoclonal antibody (final concentrations were 1 μg/mL, 2 μg/mL and 4 μg/mL for anti- apoB, apoAI and HSA, respectively, in 0.1 M sodium carbonate-sodium bicarbonate, pH 9.6 and 0.2 mg/mL sodium azide). Coating was carried out overnight at room temperature. After coating and between each subsequent incubation step, the plates were washed five times with 0.9% sodium chloride with 0.05% Tween 20. Duplicate aliquots (100 μL) of diluted culture media or standard (purified apoB, apoAI or HSA diluted to 0.3125-320 ng/mL with cell culture medium) were added to wells coated with monoclonal antibody. Following incubation for 1.5 hours at room temperature, the antigen or sample was removed and the wells washed. The secondary antibodies were diluted 1:500 in PBS + 0.05% Tween 20 (Buffer III), then 100 μL was added to each well and incubated for 1 hour at room temperature. The antibody was removed and the wells were washed. All secondary antibodies were polyclonal antisera raised in goat against the human proteins. A rabbit antigoat IgG, conjugated to alkaline phosphatase, was diluted 1:1000 with Buffer III and 100 μL was added to each well. Following incubation for 1 hour at room temperature, the antibody was removed and wells washed eight times. The substrate p-nitrophenylphosphate (Sigma Biochemical, St. Louis, MO) was added at 1 mg/mL in 0.05 M NaCarbonate-NaBicarbonate, pH 9.8 + 1 mM magnesium chloride. Following a 45-minute reaction at room

temperature, the assay was stopped and the color stabilized with the addition of 100 μL of 0.1 M Tris, pH 8.0 + 0.1 M EDTA. The microtiter plates were read at 405 nm in a V-Max 96-well plate reader (Molecular Devices, Menlo Park, CA).

After subtraction of background, the standards were plotted on a semi-log graph and logarithmic regression was performed. The equation for the curve was used to calculate the concentration of apoB, apoAl and HSA. The protein concentration was normalized to total cell protein yielding concentrations with units of ng/ml/mg cell protein. Each drug treatment was performed in duplicate and the results were averaged. The apoB, apoAl, and HSA concentrations for each drug treatment were divided by the corresponding protein concentration in the DMSO control. The results were plotted as a percentage of control versus the drug concentration.

**D. Lipid analysis**

HepG2 cells were subcultured into 6-well dishes and allowed to come to confluency at least 24 hours before drug treatment. Prior to addition of the drug, culture media were removed, cells washed once with PBS, and 1 mL of fresh medium (RPMI 1640 + 10% FBS) was added quatitatively. Compound A was added to duplicate wells in 10 μL of DMSO to yield varying compound concentrations. DMSO alone (10 μL) was used as the negative control. After a 16-hour incubation under standard cell culture conditions, the media were removed and 1 mL-of labeling medium (RPMI 1640; 16.5 mg/mL fatty acid free BSA; 1 mM sodium oleate; 1 mM glycerol; 5 μCi/mL 3H-glycerol (Amersham, Arlington Heights, IL, Catalog no. TRA.244) was added with a second addition of compound A. The cultures were incubated for 2 hours under standard cell culture conditions. Media (1 mL) were removed to 15-mL glass tubes and immediately diluted with 2 mL of ice cold methanol and 1 mL of dH2O. Cells were washed once with PBS and were processed for total protein measurements as described in section I-B.

Total lipids were extracted from the media and analyzed as follows. After addition of 5.0 mL of chloroform and 0.2 mL of 2% acetic acid, the tubes were vortexed for 1 minute and centrifuged at 2,000 rpm for 5 minutes to separate the aqueous and organic phases. The upper aqueous phase was removed and 3.6 mL of methanol:water (1:1) containing 0.1% acetic acid added. After briefly vortexing, the tubes were centrifuged as before and the aqueous phase again removed. The organic phase was quatitatively transferred to clean 15-mL glass tubes and the solvent evaporated under nitrogen. Dried lipids were dissolved in 0.1 mL of chloroform and 30 μL of each sample were spotted onto silica gel 60A, 19 channel thin layer chromatography plates (Whatman). 5-10 μg of TG in 10 μL of chloroform were added as carrier and the plates were developed in hexane:diisopropyl ether: acetic acid (130:70:4, V/V). After drying, lipid was stained by exposing the plates to iodine. Bands corresponding to TG were scraped into scintillation vials. 0.5 mL of dH2O and 10 mL of EcoLite (ICN Biomedical) scintillation fluid were added and the samples vortexed vigorously. Raw data was normalized to cell protein and expressed as percent of DMSO control.

**II. Results**

**A. Identification of MTP inhibitors**

The primary screen suggested that compound A (reference compound) inhibited the MTP-catalyzed transport of $^3$H-TG from HDL to LDL. The ability of compound A to inhibit MTP-catalyzed lipid transport was confirmed in a second assay which measures the MTP-catalyzed transport of $^3$H-TG from donor SUV to acceptor SUV. The $IC_{50}$ for compound A is about 1 μM (Figure 10).

**B. Inhibition of apoB and TG secretion**

Compound A was administered to HepG2 cells in a twofold dilution series ranging from 0.156 to 20 μM. After a 16-hour incubation under standard cell culture conditions, aliquots of the conditioned media were assayed by ELISA for apoB, apoAl and HSA. ApoB secretion was inhibited in a dose-responsive manner with an $IC_{50}$ of 5 μM (Figure 11). The secretion of apoAl and HSA was unaffected up to the maximum dose of 20 μM confirming that the inhibition was specific for apoB. These data indicate that addition of an MTP inhibitor to a human liver cell line inhibits the secretion of lipoproteins which contain apoB.

HepG2 cells were treated with doses of compound A ranging from 1.25 μM - 20 μM under conditions identical to those utilized for the apoB, apoAl and HSA secretion experiment. The intracellular pool of TG was radiolabelled for two hours with 3H-glycerol in the presence of vehicle or varying doses of compound A. The accumulation of radiolabelled TG in the medium was measured by quantitative extraction, followed by thin layer chromatography analysis and normalization to total cell protein. DMSO alone was used as a control. TG secretion was inhibited by compound A in a dose-dependent manner. The $IC_{50}$ was observed to be about 2.0 μM, which is similar to the $IC_{50}$ for inhibition of apoB secretion (Figure 12). The data confirm that compound A inhibits the secretion of TG-rich lipoproteins that contain apoB.

The foregoing procedures were repeated with compound B. Compound B inhibits MTP-catalyzed $^3$H-TG trans-

port from donor SUV to acceptor SUV. The $IC_{50}$ is about 4 to 6 μM (Figure 13). The secretion of lipoproteins that contain apoB is also inhibited in HepG2 cells by compound B (Figure 14).

**Example 7**

**Inhibition of MTP-catalyzed CE and PC Transport**

**I. Methods**

To measure the effect of compound A on bovine MTP-catalyzed transport of CE or PC between membranes, the lipid transfer assay which measures TG transfer between SUV was modified. The composition of the donor vesicles was the same, except 0.25 mol% $^{14}$C-CE or $^{14}$C-PC replaced the labeled TG. The composition of the acceptor vesicles were the same, except labeled PC and unlabeled TG were not included. Following precipitation of donor vesicles, the percentage of lipid transfer was calculated by comparing the $^{14}$C-CE or -PC in the acceptor vesicles in the supernatant following a transfer reaction to the total $^{14}$C-CE or -PC in the assay. The labeled lipid in the supernatant in the absence of MTP was subtracted from the labeled lipid in the presence MTP to calculate the MTP-catalyzed lipid transfer from donor SUV to acceptor SUV. The remainder of the assay was essentially as described previously.

**II. Results**

The ability of compound A to inhibit the MTP-catalyzed transport of radiolabeled CE and PC between membranes was also investigated. Compound A inhibited CE transfer in a manner comparable to its inhibition of TG transfer. Compound A inhibited PC transfer, but it was less effective at inhibiting PC transfer than CE and TG transfer. Approximately 40% of the PC transfer was inhibited at concentrations of inhibitor which decreased TG and CE transfer more than 80%.

**Example 8**

**Cloning of bovine MTP - 5' end**

A bovine small intestinal cDNA library, packaged in lambda gt10, was obtained from Clontech (#BL1010A). The library was diluted in SM to contain 50,000 phage/100 μL (a 1:100,000 dilution). The diluted phage (100 μL) were mixed with 300 μL E.Coli C600 cells (Clontech) and incubated for 15 minutes at 37°C. After adding 7 mL of top agarose, the mixture was poured onto a 150 mm plate containing 75 mL of LB agarose. A total of 25 plates, each containing approximately 5 X $10^4$ phage, were prepared in this manner. The plates were incubated overnight at 37°C.

To isolate phage DNA, 10 mL SM (no gelatin) was added to each plate. The plates were then rocked gently at room temperature for 2 hours. The eluted phage (approximately 8 mL/plate) were collected and pooled. E.Coli cells were sedimented by centrifugation for 10 minutes at 12,000 X g.

Lambda DNA was isolated from the supernatant using the QIAGEN tip-100 (midi) preparation according to the protocol supplied by the manufacturer. The purified DNA was resuspended in a total of 200 μL TE (10 mM Tris.Cl pH 8.0, 1 mM EDTA).

1 μg lambda phage DNA (approximately 3 X $10^7$ molecules) was added to a 100 μL PCR reaction containing 2 mM magnesium chloride, 0.2 mM each deoxynucleotide triphosphate, 1.25X buffer, and 2.5 units Taq polymerase (Perkin-Elmer Cetus, kit #N801-0555). The concentration of each primer was 0.15 mM. The sequence of the forward primer (SEQ. ID. NO. 29) was as follows:

41 66

GGTCAATATGATTCTTCTTGCTGTGC.

The forward primer's sequence was based on the human cDNA sequence encoding bases 41 to 66 of the 88 kDa component of MTP. The reverse primer (SEQ. ID. NO. 30) had the following sequence:

658 636 (bovine)
807 785 (human) -

GCCTCGATACTATTTGCCTGCT

The reverse primer's sequence was based on the known bovine cDNA sequence encoding the 88 kDa component of MTP and hybridizes from base 658 to 636 of the bovine cDNA, which correspond to bases 807-785 of the human cDNA.

PCR-amplification was conducted in a Perkin-Elmer thermal cycler, model 9600. After a two-minute incubation at 97˚C, the reaction was cycled at 94˚C for 30 seconds, 50˚C for 30 seconds, and 72˚C for one minute for 35 cycles. A final incubation at 72˚C for 7 minutes was performed.

The PCR product was electrophoresed on a 1% agarose gel in TAE buffer as described previously. The yield of the desired 766 base pair fragment was approximately 2 μg. The DNA was excised from the gel, purified using GeneClean (Bio101 La Jolla, CA), blunt-ended, cloned into pUC 18-Sma1 (Pharmacia), and sequenced as described previously.

The new sequence obtained from the bovine cDNA encoding the 5' region of the 88 kDa component of MTP is shown in SEQ. ID. NO. 5. The sequence adds 83 bases to the 5' end of the bovine cDNA reported previously.

**Example 9**

**Sequencing of human genomic DNA for the 88 kDa component of MTP**

Sequencing of human genomic DNA was carried out by the procedures described in "Demonstration of a gene defect in a second abetalipoproteinemic subject" and in Example 1. The result of this procedure is the human genomic sequence SEQ. ID. NO. 8.

# BOVINE cDNA SEQUENCE

(SEQ. ID. NO. 1)
2 900 nucleotides

```
        10         20         30         40         50
1234567890 1234567890 1234567890 1234567890 1234567890
AAACTCACAT ACTCCACTGA AGTTTTTCTC GATCGGGGCA AAGGAAACCT     50
CCAAGACAGT GTGGGCTACC GAATTTCATC CAATGTGGAT GTCGCTTTAC    100
TGTGGAGGAG TCCTGATGGT GATGATAACC AACTGATCCA AATTACCATG    150
AAAGATGTAA ACCTTGAAAA TGTGAATCAA CAGAGAGGAG AGAAGAGCAT    200
TTTCAAAGGA AAAAAGTCAT CTCAAATCAT AAGAAAGGAA AACTTGGAAG    250
CAATGCAAAG ACCTGTGCTC CTTCATCTAA TTCATGGAAA GATCAAAGAG    300
TTCTACTCAT ATCAAAATGA ACCAGCAGCC ATAGAAAATC TCAAGAGAGG    350
CCTGGCTAGC CTATTTCAGA TGCAGTTAAG CTCTGGAACT ACCAATGAGG    400
TAGACATCTC TGGAGATTGT AAAGTGACCT ACCAGGCTCA TCAAGACAAA    450
GTGACCAAAA TTAAGGCTTT GGATTCATGC AAAATAGAGA GGGCTGGATT    500
TACGACCCCA CATCAGGTCT TGGGTGTCAC TTCGAAAGCC ACATCTGTCA    550
CTACCTATAA GATAGAAGAC AGCTTTGTTG TAGCTGTGCT CTCAGAAGAG    600
ATACGTGCTT TAAGGCTCAA TTTTCTACAA TCAATAGCAG GCAAAATAGT    650
ATCGAGGCAG AAACTGGAGC TGAAAACCAC GGAAGCAAGC GTGAGACTGA    700
AGCCAGGAAA GCAGGTTGCA GCCATCATTA AAGCAGTCGA TTCAAAGTAC    750
ACGGCCATTC CCATTGTGGG GCAGGTCTTC CAGAGCAAGT GCAAAGGATG    800
CCCTTCTCTC TCAGAGCACT GGCAGTCCAT CAGAAAACAC CTGCAGCCTG    850
ACAACCTCTC CAAGGCTGAG GCTGTCAGAA GCTTCCTGGC CTTCATCAAG    900
```

BOVINE cDNA SEQUENCE

(SEQ. ID. NO. 1, continued)

| 10<br>1234567890 | 20<br>1234567890 | 30<br>1234567890 | 40<br>1234567890 | 50<br>1234567890 | |
|---|---|---|---|---|---|
| CACCTCAGGA | CGGCAAAGAA | AGAAGAGATC | CTCCAAATTC | TAAAGGCAGA | 950 |
| AAACAAGGAA | GTACTACCCC | AGCTAGTGGA | TGCTGTCACC | TCTGCTCAGA | 1000 |
| CACCAGACTC | ATTAGACGCC | ATTTTGGACT | TTCTGGATTT | CAAAAGCACC | 1050 |
| GAGAGCGTTA | TCCTCCAGGA | AAGGTTTCTC | TATGCCTGTG | CATTTGCCTC | 1100 |
| ACATCCTGAT | GAAGAACTCC | TGAGAGCCCT | CATTAGTAAG | TTCAAAGGTT | 1150 |
| CTTTTGGAAG | CAATGACATC | AGAGAATCTG | TTATGATCAT | CATCGGGGCC | 1200 |
| CTTGTCAGGA | AGTTGTGTCA | GAACCAAGGC | TGCAAACTGA | AAGGAGTAAT | 1250 |
| AGAAGCCAAA | AAGTTAATCT | TGGGAGGACT | TGAAAAAGCA | GAGAAAAAAG | 1300 |
| AGGACATCGT | GATGTACCTG | CTGGCTCTGA | AGAACGCCCG | GCTTCCAGAA | 1350 |
| GGCATCCCGC | TCCTTCTGAA | GTACACAGAG | ACAGGAGAAG | GGCCCATTAG | 1400 |
| CCACCTTGCC | GCCACCACAC | TCCAGAGATA | TGATGTCCCT | TTCATAACTG | 1450 |
| ATGAGGTAAA | GAAGACTATG | AACAGGATAT | ACCACCAGAA | TCGTAAAATA | 1500 |
| CATGAAAAAA | CTGTGCGTAC | TACTGCAGCT | GCCATCATTT | TAAAAAACAA | 1550 |
| TCCATCCTAC | ATGGAAGTAA | AAAACATCCT | GCTCTCTATT | GGGGAACTTC | 1600 |
| CCAAAGAAAT | GAATAAGTAC | ATGCTCTCCA | TTGTCCAAGA | CATCCTACGT | 1650 |
| TTTGAAACAC | CTGCAAGCAA | AATGGTCCGT | CAAGTTCTGA | AGGAAATGGT | 1700 |
| CGCTCATAAT | TACGATCGTT | TCTCCAAGAG | TGGGTCCTCC | TCTGCATATA | 1750 |
| CTGGCTACGT | AGAACGGACT | TCCCATTCGG | CATCTACTTA | CAGCCTTGAC | 1800 |

## BOVINE cDNA SEQUENCE

(SEQ. ID. NO. 1, continued)

```
        10         20         30         40         50
1234567890 1234567890 1234567890 1234567890 1234567890
ATTCTTTACT CTGGTTCTGG CATTCTAAGG AGAAGTAATC TGAACATCTT   1850

TCAGTATATT GAGAAAACTC CTCTTCATGG TATCCAGGTG GTCATTGAAG   1900

CCCAAGGACT GGAGGCATTA ATTGCAGCCA CTCCTGATGA GGGGGAAGAG   1950

AACCTTGACT CCTATGCTGG CTTGTCAGCT CTCCTCTTTG ATGTTCAGCT   2000

CAGACCTGTC ACTTTTTTCA ACGGGTACAG TGATTTGATG TCCAAAATGC   2050

TGTCAGCATC TAGTGACCCT ATGAGTGTGG TGAAAGGACT TCTTCTGCTA   2100

ATAGATCATT CCCAGGAGCT TCAGCTGCAA TCTGGACTTA AGGCCAATAT   2150

GGATGTTCAA GGTGGTCTAG CTATTGATAT TACAGGTGCC ATGGAGTTTA   2200

GTCTATGGTA TCGTGAATCT AAAACCCGAG TGAAAAATCG GGTAAGTGTG   2250

TTAATAACTG GTGGCATCAC GGTGGACTCC TCTTTTGTGA AAGCTGGCTT   2300

GGAAATTGGT GCAGAAACAG AAGCAGGCTT GGAGTTTATC TCCACGGTGC   2350

AGTTTTCTCA GTACCCATTT TTAGTTTGTC TGCAGATGGA CAAGGAAGAT   2400

GTTCCATACA GGCAGTTTGA GACAAAATAT GAAAGGCTGT CCACAGGCAG   2450

AGGTTACATC TCTCGGAAGA GAAAAGAAAG CCTAATAGGA GGATGTGAAT   2500

TCCCGCTGCA CCAAGAGAAC TCTGACATGT GCAAGGTGGT GTTTGCTCCT   2550

CAACCAGAGA GCAGTTCCAG TGGTTGGTTT TGAAACTGAT GGGGGCTGTT   2600

TCATTAGACT TCATCTCGCC AGAAGGGATA AGACGTGACA TGCCTAAGTA   2650

TTGCTCTCTG AGAGCACAGT GTTTACATAT TTACCTGTAT TTAAGAGTTT   2700
```

BOVINE cDNA SEQUENCE

(SEQ. ID. NO. 1, continued)

| | | | | | |
|---|---|---|---|---|---|
| 10 | 20 | 30 | 40 | 50 | |
| 1234567890 | 1234567890 | 1234567890 | 1234567890 | 1234567890 | |
| TGTAGAACGT | GATGAAAAAC | CTCACATAAT | TAAGTTTGGG | CCTGAATCAT | 2750 |
| TTGATACTAC | CTACAGGGTC | ATTCTGAGCC | ACTCTATGTG | ATACTTTAGT | 2800 |
| AGCGTTCTGT | TTTCCTGCAT | CTCTCTCAAA | TCACATTTAC | TACTGTGAAA | 2850 |
| CTAGTTCTGC | CCTAAGAAGA | AACCATTGTT | TAAAAAAAAA | AAAAAAAAAA | 2900 |

## HUMAN cDNA SEQUENCE

(SEQ. ID. NO. 2); 3185 nucleotides

| 10<br>1234567890 | 20<br>1234567890 | 30<br>1234567890 | 40<br>1234567890 | 50<br>1234567890 | |
|---|---|---|---|---|---|
| GTGACTCCTA | GCTGGGCACT | GGATGCAGTT | GAGGATTGCT | GGTCAATATG | 50 |
| ATTCTTCTTG | CTGTGCTTTT | TCTCTGCTTC | ATTTCCTCAT | ATTCAGCTTC | 100 |
| TGTTAAAGGT | CACACAACTG | GTCTCTCATT | AAATAATGAC | CGGCTGTACA | 150 |
| AGCTCACGTA | CTCCACTGAA | GTTCTTCTTG | ATCGGGGCAA | AGGAAAACTG | 200 |
| CAAGACAGCG | TGGGCTACCG | CATTTCCTCC | AACGTGGATG | TGGCCTTACT | 250 |
| ATGGAGGAAT | CCTGATGGTG | ATGATGACCA | GTTGATCCAA | ATAACGATGA | 300 |
| AGGATGTAAA | TGTTGAAAAT | GTGAATCAGC | AGAGAGGAGA | GAAGAGCATC | 350 |
| TTCAAAGGAA | AAAGCCCATC | TAAAATAATG | GGAAAGGAAA | ACTTGGAAGC | 400 |
| TCTGCAAAGA | CCTACGCTCC | TTCATCTAAT | CCATGGAAAG | GTCAAAGAGT | 450 |
| TCTACTCATA | TCAAAATGAG | GCAGTGGCCA | TAGAAAATAT | CAAGAGAGGT | 500 |
| CTGGCTAGCC | TATTTCAGAC | ACAGTTAAGC | TCTGGAACCA | CCAATGAGGT | 550 |
| AGATATCTCT | GGAAATTGTA | AAGTGACCTA | CCAGGCTCAT | CAAGACAAAG | 600 |
| TGATCAAAAT | TAAGGCCTTG | GATTCATGCA | AAATAGCGAG | GTCTGGATTT | 650 |
| ACGACCCCAA | ATCAGGTCTT | GGGTGTCAGT | TCAAAAGCTA | CATCTGTCAC | 700 |
| CACCTATAAG | ATAGAAGACA | GCTTTGTTAT | AGCTGTGCTT | GCTGAAGAAA | 750 |
| CACACAATTT | TGGACTGAAT | TTCCTACAAA | CCATTAAGGG | GAAAATAGTA | 800 |
| TCGAAGCAGA | AATTAGAGCT | GAAGACAACC | GAAGCAGGCC | CAAGATTGAT | 850 |
| GTCTGGAAAG | CAGGCTGCAG | CCATAATCAA | AGCAGTTGAT | TCAAAGTACA | 900 |
| CGGCCATTCC | CATTGTGGGG | CAGGTCTTCC | AGAGCCACTG | TAAAGGATGT | 950 |
| CCTTCTCTCT | CGGAGCTCTG | GCGGTCCACC | AGGAAATACC | TGCAGCCTGA | 1000 |

## HUMAN cDNA SEQUENCE

(SEQ. ID. NO. 2, continued)

| 10<br>1234567890 | 20<br>1234567890 | 30<br>1234567890 | 40<br>1234567890 | 50<br>1234567890 | |
|---|---|---|---|---|---|
| CAACCTTTCC | AAGGCTGAGG | CTGTCAGAAA | CTTCCTGGCC | TTCATTCAGC | 1050 |
| ACCTCAGGAC | TGCGAAGAAA | GAAGAGATCC | TTCAAATACT | AAAGATGGAA | 1100 |
| AATAAGGAAG | TATTACCTCA | GCTGGTGGAT | GCTGTCACCT | CTGCTCAGAC | 1150 |
| CTCAGACTCA | TTAGAAGCCA | TTTTGGACTT | TTTGGATTTC | AAAAGTGACA | 1200 |
| GCAGCATTAT | CCTCCAGGAG | AGGTTTCTCT | ATGCCTGTGG | ATTTGCTTCT | 1250 |
| CATCCCAATG | AAGAACTCCT | GAGAGCCCTC | ATTAGTAAGT | TCAAAGGTTC | 1300 |
| TATTGGTAGC | AGTGACATCA | GAGAAACTGT | TATGATCATC | ACTGGGACAC | 1350 |
| TTGTCAGAAA | GTTGTGTCAG | AATGAAGGCT | GCAAACTCAA | AGCAGTAGTG | 1400 |
| GAAGCTAAGA | AGTTAATCCT | GGGAGGACTT | GAAAAAGCAG | AGAAAAAAGA | 1450 |
| GGACACCAGG | ATGTATCTGC | TGGCTTTGAA | GAATGCCCTG | CTTCCAGAAG | 1500 |
| GCATCCCAAG | TCTTCTGAAG | TATGCAGAAG | CAGGAGAAGG | GCCCATCAGC | 1550 |
| CACCTGGCTA | CCACTGCTCT | CCAGAGATAT | GATCTCCCTT | TCATAACTGA | 1600 |
| TGAGGTGAAG | AAGACCTTAA | ACAGAATATA | CCACCAAAAC | CGTAAAGTTC | 1650 |
| ATGAAAAGAC | TGTGCGCACT | GCTGCAGCTG | CTATCATTTT | AAATAACAAT | 1700 |
| CCATCCTACA | TGGACGTCAA | GAACATCCTG | CTGTCTATTG | GGGAGCTTCC | 1750 |
| CCAAGAAATG | AATAAATACA | TGCTCGCCAT | TGTTCAAGAC | ATCCTACGTT | 1800 |
| TTGAAATGCC | TGCAAGCAAA | ATTGTCCGTC | GAGTTCTGAA | GGAAATGGTC | 1850 |
| GCTCACAATT | ATGACCGTTT | CTCCAGGAGT | GGATCTTCTT | CTGCCTACAC | 1900 |
| TGGCTACATA | GAACGTAGTC | CCCGTTCGGC | ATCTACTTAC | AGCCTAGACA | 1950 |
| TTCTCTACTC | GGGTTCTGGC | ATTCTAAGGA | GAAGTAACCT | GAACATCTTT | 2000 |

## HUMAN cDNA SEQUENCE

(SEQ. ID. NO. 2, continued)-

| 10<br>1234567890 | 20<br>1234567890 | 30<br>1234567890 | 40<br>1234567890 | 50<br>1234567890 | |
|---|---|---|---|---|---|
| CAGTACATTG | GGAAGGCTGG | TCTTCACGGT | AGCCAGGTGG | TTATTGAAGC | 2050 |
| CCAAGGACTG | GAAGCCTTAA | TCGCAGCCAC | CCCTGACGAG | GGGGAGGAGA | 2100 |
| ACCTTGACTC | CTATGCTGGT | ATGTCAGCCA | TCCTCTTTGA | TGTTCAGCTC | 2150 |
| AGACCTGTCA | CCTTTTTCAA | CGGATACAGT | GATTTGATGT | CCAAAATGCT | 2200 |
| GTCAGCATCT | GGCGACCCTA | TCAGTGTGGT | GAAAGGACTT | ATTCTGCTAA | 2250 |
| TAGATCATTC | TCAGGAACTT | CAGTTACAAT | CTGGACTAAA | AGCCAATATA | 2300 |
| GAGGTCCAGG | GTGGTCTAGC | TATTGATATT | TCAGGTGCAA | TGGAGTTTAG | 2350 |
| CTTGTGGTAT | CGTGAGTCTA | AAACCCGAGT | GAAAAATAGG | GTGACTGTGG | 2400 |
| TAATAACCAC | TGACATCACA | GTGGACTCCT | CTTTTGTGAA | AGCTGGCCTG | 2450 |
| GAAACCAGTA | CAGAAACAGA | AGCAGGCTTG | GAGTTTATCT | CCACAGTGCA | 2500 |
| GTTTTCTCAG | TACCCATTCT | TAGTTTGCAT | GCAGATGGAC | AAGGATGAAG | 2550 |
| CTCCATTCAG | GCAATTTGAG | AAAAAGTACG | AAAGGCTGTC | CACAGGCAGA | 2600 |
| GGTTATGTCT | CTCAGAAAAG | AAAAGAAAGC | GTATTAGCAG | GATGTGAATT | 2650 |
| CCCGCTCCAT | CAAGAGAACT | CAGAGATGTG | CAAAGTGGTG | TTTGCCCCTC | 2700 |
| AGCCGGATAG | TACTTCCAGC | GGATGGTTTT | GAAACTGACC | TGTGATATTT | 2750 |
| TACTTGAATT | TGTCTCCCCG | AAAGGGACAC | AATGTGGCAT | GACTAAGTAC | 2800 |
| TTGCTCTCTG | AGAGCACAGC | GTTTACATAT | TTACCTGTAT | TTAAGATTTT | 2850 |
| TGTAAAAAGC | TACAAAAAAC | TGCAGTTTGA | TCAAATTTGG | GTATATGCAG | 2900 |
| TATGCTACCC | ACAGCGTCAT | TTTGAATCAT | CATGTGACGC | TTTCAACAAC | 2950 |
| GTTCTTAGTT | TACTTATACC | TCTCTCAAAT | CTCATTTGGT | ACAGTCAGAA | 3000 |

HUMAN cDNA SEQUENCE
(SEQ. ID. NO. 2, continued)

| 10 | 20 | 30 | 40 | 50 | |
|---|---|---|---|---|---|
| 1234567890 | 1234567890 | 1234567890 | 1234567890 | 1234567890 | |
| TAGTTATTCT | CTAAGAGGAA | ACTAGTGTTT | GTTAAAAACA | AAAATAAAAA | 3050 |
| CAAAACCACA | CAAGGAGAAC | CCAATTTTGT | TTCAACAATT | TTTGATCAAT | 3100 |
| GTATATGAAG | CTCTTGATAG | GACTTCCTTA | AGCATGACGG | GAAAACCAAA | 3150 |
| CACGTTCCCT | AATCAGGAAA | AAAAAAAAAA | AAAAA | | 3185 |

## BOVINE PROTEIN SEQUENCE

(SEQ. ID. NO. 3) ; 860 amino acids

| 10 | 20 | 30 | 40 | 50 | |
|---|---|---|---|---|---|
| 1234567890 | 1234567890 | 1234567890 | 1234567890 | 1234567890 | |
| KLTYSTEVFL | DRGKGNLQDS | VGYRISSNVD | VALLWRSPDG | DDNQLIQITM | 50 |
| KDVNLENVNQ | QRGEKSIFKG | KKSSQIIRKE | NLEAMQRPVL | LHLIHGKIKE | 100 |
| FYSYQNEPAA | IENLKRGLAS | LFQMQLSSGT | TNEVDISGDC | KVTYQAHQDK | 150 |
| VTKIKALDSC | KIERAGFTTP | HQVLGVTSKA | TSVTTYKIED | SFVVAVLSEE | 200 |
| IRALRLNFLQ | SIAGKIVSRQ | KLELKTTEAS | VRLKPGKQVA | AIIKAVDSKY | 250 |
| TAIPIVGQVF | QSKCKGCPSL | SEHWQSIRKH | LQPDNLSKAE | AVRSFLAFIK | 300 |
| HLRTAKKEEI | LQILKAENKE | VLPQLVDAVT | SAQTPDSLDA | ILDFLDFKST | 350 |
| ESVILQERFL | YACAFASHPD | EELLRALISK | FKGSFGSNDI | RESVMIIIGA | 400 |
| LVRKLCQNQG | CKLKGVIEAK | KLILGGLEKA | EKKEDIVMYL | LALKNARLPE | 450 |
| GIPLLLKYTE | TGEGPISHLA | ATTLQRYDVP | FTTDEVKKIM | NRIYHQNRKI | 500 |
| HEKTVRTTAA | AIILKNNPSY | MEVKNILLSI | GELPKEMNKY | MLSIVQDILR | 550 |
| FETPASKMVR | QVLKEMVAHN | YDRFSKSGSS | SAYTGYVERT | SHSASTYSLD | 600 |
| ILYSGSGILR | RSNLNIFQYI | EKTPLHGIQV | VIEAQGLEAL | IAATPDEGEE | 650 |
| NLDSYAGLSA | LLFDVQLRPV | TFFNGYSDLM | SKMLSASSDP | MSVVKGLLLL | 700 |
| IDHSQELQLQ | SGLKANMDVQ | GGLAIDITGA | MEFSLWYRES | KTRVKNRVSV | 750 |
| LITGGITVDS | SFVKAGLEIG | AETEAGLEFI | STVQFSQYPF | LVCLQMDKED | 800 |
| VPYRQFETKY | ERLSTGRGYI | SRKRKESLIG | GCEFPLHQEN | SDMCKVVFAP | 850 |
| QPESSSSGWF | | | | | 860 |

## HUMAN PROTEIN SEQUENCE

(SEQ. ID. NO. 4); 894 amino acids

| 10<br>1234567890 | 20<br>1234567890 | 30<br>1234567890 | 40<br>1234567890 | 50<br>1234567890 | |
|---|---|---|---|---|---|
| MILLAVLFLC | FISSYSASVK | GHTTGLSLNN | DRLYKLTYST | EVLLDRGKGK | 50 |
| LQDSVGYRIS | SNVDVALLWR | NPDGDDDQLI | QITMKDVNVE | NVNQQRGEKS | 100 |
| IFKGKSPSKI | MGKENLEALQ | RPTLLHLIHG | KVKEFYSYQN | EAVAIENIKR | 150 |
| GLASLFQTQL | SSGTTNEVDI | SGNCKVTYQA | HQDKVIKIKA | LDSCKIARSG | 200 |
| FTTPNQVLGV | SSKATSVTTY | KIEDSFVIAV | LAEETHNFGL | NFLQTIKGKI | 250 |
| VSKQKLELKT | TEAGPRLMSG | KQAAAIIKAV | DSKYTAIPIV | GQVFQSHCKG | 300 |
| CPSLSELWRS | TRKYLQPDNL | SKAEAVRNFL | AFIQHLRTAK | KEEILQILKM | 350 |
| ENKEVLPQLV | DAVTSAQTSD | SLEAILDFLD | FKSDSSIILQ | ERFLYACGFA | 400 |
| SHPNEELLRA | LISKFKGSIG | SSDIRETVMI | ITGTLVRKLC | QNEGCKLKAV | 450 |
| VEAKKLILGG | LEKAEKKEDT | RMYLLALKNA | LLPEGIPSLL | KYAEAGEGPI | 500 |
| SHLATTALQR | YDLPFITDEV | KKTLNRIYHQ | NRKVHEKTVR | TAAAAIILNN | 550 |
| NPSYMDVKNI | LLSIGELPQE | MNKYMLAIVQ | DILRFEMPAS | KIVRRVLKEM | 600 |
| VAHNYDRFSR | SGSSSAYTGY | IERSPRSAST | YSLDILYSGS | GILRRSNLNI | 650 |
| FQYIGKAGLH | GSQVVIEAQG | LEALIAATPD | EGEENLDSYA | GMSAILFDVQ | 700 |
| LRPVTFFNGY | SDLMSKMLSA | SGDPISVVKG | LILLIDHSQE | LQLQSGLKAN | 750 |
| IEVQGGLAID | ISGAMEFSLW | YRESKTRVKN | RVTVVITTDI | TVDSSFVKAG | 800 |
| LETSTETEAG | LEFISTVQFS | QYPFLVCMQM | DKDEAPFRQF | EKKYERLSTG | 850 |
| RGYVSQKRKE | SVLAGCEFPL | HQENSEMCKV | VFAPQPDSTS | SGWF | 894 |

(SEQ. ID. NO. 8)

**AAGGTTCCTGAGCCCCACTGTGGTAGAGAGATGCACTGATGGTGAGACAG**

**CATGTTCCCTTACAATGAAAACTGGATATGTGTCATTATCTTTATGCAG**G 109

TCACACAACTGGTCTCTCATTAAATAATGACCGGCTGTACAAGCTCACGT

ACTCCACTGAAGTTCTTCTTGATCGGGGCAAAGGAAAACTGCAAGACAGC EXON 2

GTGGGCTACCGCATTTCCTCCAACGTGGATGTGGCCTTACTATGGAGGAA

TCCTGATGGTGATGATGACCAGTTGATCCAAATAACG**GTGGGCATTTTCT** 296

**ACCAGATAAATGCAAAGATTAGATATCAGAAGTTTTTGGAGAAGTGTACC**

**ATTGGACAGCACTTGTATTGGGTTCCCGTTTATAATCCATTAGTTTCTTA**

**TCTATCACTAAAACAAGCAGGTCTTTGTTTTAAGGTTTGGTGATGAAAG** →

**TTATTTTAAGCCTAAAGTCACAGAGTTCTTTAAGTATTGCTATTTTTGCC**

**TTATTAAAAAACCTAGTTTATAAATACCTTCTCCATTCTTTTAAAGTGAG**

**TGGCAAGGTCCTATAAATCATGAATTGAAAAATGACAGAAGAAATTGTGG**

**CCAACTCTTTCTGTTTCTTTATCATTTTATTTTCAGAGATACTCTGATGA**

**AGACAGATATAGGAAGTTTTTTTAACAGCTTTCTTTCTGTTACTCCAG**A 297

TGAAGGATGTAAATGTTGAAAATGTGAATCAGCAGAGAGGAGAGAAGAGC

EXON 3

ATCTTCAAAGGAAAAAGCCCATCTAAAATAATGGGAAAGGAAAACTTGGA

AGCTCTGCAAAGACCTACGCTCCTTCATCTAATCCATGGAAAG**GTAAAGG** 440

**GGCGTTTAGATTCCACAACTTTTTCTCCAACTTCATATTTTTCTTCCCTT**

**CAGTAGATATTATTTTGAGGTAATCACATTGTAACTACTTTTATGGTAAA**

**TGGAATTTCTTCAAGAACTAAAGAACAGAGGTTGTAAATTAAATGTTTCC**

**AAACTGAATCAATGCCCTGAGTTCCCTTACATTTACTAGCCAATTTGTTT**

**CCTATTTTTCTGGAAATCTTTATAGTGGAATGAAGTATTTATTTATTGAT**

**GAAAGGCATTATTAAAAGGTAAATTTCTCATCAAATTATAAGGATTACA**

**AACATAATGTAACAAAGCAAGTCATCAAAGCATGATTGGATGAATTC** →

(SEQ. ID. NO. 8, continued)

TCTGATAAATGATGCATTTTTGCTTCATTTGTGTTCTGTTCCCCTCTCCC
CACCAG|GTCAAAGAGTTCTACTCATATCAAAATGAGGCAGTGGCCATAGA 441
AAATATCAAGAGAGGTCTGGCTAGCCTATTTCAGACACAGTTAAGCTCTG EXON 4
GAACCACCAATGAG|GTACTTACCAATATTAATAAGGATTCAGCATCTCAA 548
TAAAATTTGTAAGGATTTCTACTTATACAATTTCAGTAGAAGAGTTACTA
CTAAGGTAATGCTCAGAAAAGGTGACTTGTGTAG →
TCCCCTATGGCCTATTAGAGACCTCAATTTTCAAGCCACTTCTCACTAGA
ATTCAAATGGCCCACAAGGAATCCCAAGCATTATGCCCTTGCCTTTCTTT
TTAG|GTAGATATCTCTGGAAATTGTAAAGTGACCTACCAGGCTCATCAAG 549
ACAAAGTGATCAAAATTAAGGCCTTGGATTCATGCAAAATAGCGAGGTCT EXON 5
GGATTTACGACCCCAAATCAG|GTATGATAGATGTCACTTTCTTTGAGGCA 665
TTAAAATAATTACATTTTGTAGAGACTAATTTA →
CGATGATTACTTGTTATAAAGATGGCTATTTATTTATTTAG|GTCTTGGGT 666
GTCAGTTCAAAAGCTACATCTGTCACCACCTATAAGATAGAAGACAGCTT
EXON 6
TGTTATAGCTGTGCTTGCTGAAGAAACACACAATTTTGGACTGAATTTCC
TACAAACCATTAAGGGGAAAATAGTATCGAA|GTAAGATAATGCTAAAATT 805
TTTATTTTCTTTGCTATTCTTTGTTATATTATTATACTTGATTTGT →
ATGATTATAATATAGCATTTCCCTTTGGTATTATGCAG|GCAGAAATTAGA 806
GCTGAAGACAACCGAAGCAGGCCCAAGATTGATGTCTGGAAAGCAGGCTG
EXON 7
CAGCCATAATCAAAGCAGTTGATTCAAAGTACACGGCCATTCCCATTGTG
GGGCAGGTCTTCCAGAGCCACTGTAAAGGATGTCCTTCT|GTAAGTGCAGA 956
CAAATATGGGAATAATCATGACATCAGACTCTGTTTTCATTTTGTCTCCA
GTGAAAGCATCAACTCATTCA →

(SEQ. ID. NO. 8, continued)

**GGAGAACACCCTTTGTAAATGTGGATGTTCACAGTTATGAGTGGGGTATG**

**AGCCTGCAGTGTATGTTTTGCAG**CTCTCGGAGCTCTGGCGGTCCACCAGG 957

AAATACCTGCAGCCTGACAACCTTTCCAAGGCTGAGGCTGTCAGAAACTT EXON 8

CCTGGCCTTCATTCAGCACCTCAGGACTGCGAAGAAAGAAGAGATCCTTC

AAATACTAAAGATGGAAAATAAGGAAGTATT**GTAAGTTCCCCAACCTTTG** 1114

**TGTGGGGTTGTCTGTCAGAAACATTTCTGGAGTG** →

**GATATCCATGATTATGCCTTTTTTTATAG**ACCTCAGCTGGTGGATGCTGT 1115

CACCTCTGCTCAGACCTCAGACTCATTAGAAGCCATTTTGGACTTTTTGG EXON 9

ATTTCAAAAGTGACAGCAGCATTATCCTCCAGGAGAGGTTTCTCTATGCC

TGTGGATTTGCTTCTCATCCCAATGAAGAACTCCTGAGAGCCCTCATT**GT** 1283

**AAGTCAAATAGAAAATAAAGACCCTCAACTCCTATAAAACTTCTTAAGAA**

**TATTAACAGTAATTAAAAGTTTCTTAGATCCGAATTCTTCGCCCTATAGT**

**GAGTCA** →

**CTATTTTATCCCTGGGTGGTTAATAG**AGTAAGTTCAAAGGTTCTATTGGT 1284

AGCAGTGACATCAGAGAAACTGTTATGATCATCACTGGGACACTTGTCAG EXON 10

AAAGTTGTGTCAGAATGAAGGCTGCAAACTCAA**GTAAGTGCAAATCCAA** 1391

**TCTCATGTATTACATCATTCTACACCATTGTCCATTTGATACTCACCATG**

**CTGCCTACTATTGGCACTCCTAATTCTCTTTACTCTATTCTACTTACCTT**

**ATTTGNATAGCAAT** →

(SEQ. ID. NO. 8, continued)

**AACACAATATGCCCATTATTGATAATACTCATTGCTTCTTAAGAATGTAT**

**ATGTATTTTTTTAAAAAAAGCATAACACCTTTATCAAGCTTTACTTGTT**

**TGCTTTTATTCCACTGTGTGCCTCAGTCAAGCAACCAATGCAAAACTTTG**

**TAAAACTGTAGGTTGCTTTCTTGGACCCAAGAATAAAGCCAGTCTCACCC**

**AAGTCTTCTTCAATGTATGGTCATGCATATATCTAAGGTATATGATTTTT**

**CAG**|GCAGTAGTGGAAGCTAAGAAGTTAATCCTGGGAGGACTTGAAAAAGC 1392

AGAGAAAAAAGAGGACACCAGGATGTATCTGCTGGCTTTGAAGAATGCCC

EXON 11

TGCTTCCAGAAGGCATCCCAAGTCTTCTGAAGTATGCAGAAGCAGGAGAA

GGGCCCATCAGCCACCTGGCTACCACTGCTCTCCAGAGATATGATCTCCC

TTTCATAACTGATGAG|**GTAAAATCTCCAAGAATATTTGCAACATTTACAG** 1604

**AAGAAAAAAAAAAAAGCATGCTGAACATGAGTCAAATGCAAATTCCGCTCA**

**AGTCACTCTGTATTTTCCCCAAATAGTCTTCTCTCCTGCTTAAAAATAAC**

**TCTTAAATTGCATTTGGGGCTATTCTAA** ⟶

**ATGTTTAATTTCTCAGGCTATGCCTAATGTGCATAAGGAAGTATGTGGTC**

**TGAAGTTCACTACAGTCATGGAAGAAAGAGATGGAGAAAGCCACCAGCTC**

**TTAACGGCCTCAGCCTAGAAGTGATCCTCATAGATTCTATCCATGGCGTA**

**TTAGCCAGAACTAGTCACGTGGCCCCCACCAAATCACAAAGGAATCTGGG**

**AAATGTAGTAACACATGTATATTTTTATGAACACTCACTATTCCTGCTAT**

**TCCTGCTGAAATGTCCATTTTAAAAATCTAGATGTGCACTAAGTTTGAAC**

**ATCTTATGAACAG**|GTGAAGAAGACCTTAAACAGAATATACCACCAAAACC 1605

GTAAAGTTCATGAAAAGACTGTGCGCACTGCTGCAGCTGCTATCATTTTA

AATAACAATCCATCCTACATGGACGTCAAGAACATCCTGCTGTCTATTGG EXON 12

GGAGCTTCCCCAAGAAATGAATAAATACATGCTCGCCATTGTTCAAGACA

TCCTACGTTTTGAAATGCCTGCAAG|**GTATAATACATTGCACATGTCTCTC** 1816

**TGTGTATTCAAGCTTATTTGTGTGTTCATGGGGTACCGATGTAGCTAATA**

**ATAATGATGTGGTCATTATGCAA** ⟶

(SEQ. ID. NO. 8, continued)

AGCTGGACACCCTTGCCTTGCTGTCATTTTGATAGCAAACTAAATTTCAA

ATATCTGAGTAATGAAGGGGCTAGCCCTAATCCTGATGCTACCACGCCAG

CTGGCACCACCCTGGCTCTTGGAAAGGCATGAGGAAAATTTGGCTTCCTC

TTTTTTCCACTGAGGATTTTTTTTTTCCAAATTTGACTTGGGAAACAGTC

ATTACAATGAATGTGCAGCTTTTTTTTTCCTCATATGTTGCAG|CAAAATT 1817

GTCCGTCGAGTTCTGAAGGAAATGGTCGCTCACAATTATGACCGTTTCTC EXON 13

CAGGAGTGGATCTTCTTCTGCCTACACTGGCTACATAGAAC|GTATGTACA 1914

CCAAAAAGAGGTTCTCCTTCCATACCCCACAACTTAGCATTGCTGGAACT

GCTATTAAATTACAGTTATTGTGTGTCATCAG|GTAGTCCCCGTTCGGCAT 1915

CTACTTACAGCCTAGACATTCTCTACTCGGGTTCTGGCATTCTAAGGAGA

AGTAACCTGAACATCTTTCAGTACATTGGGAAGGCTGGTCTTCACGGTAG EXON 14

CCAG|GTAACTCACTTCTCATGGATTTTGCTTAATAAAGTATGCAAGAAAT 2036

CAGGCTGAGGTAAAATAAAACATATATGCTGTGGGTAATGCTATAGAATG

TATAAGTTAATGGTGGCTTCTGTCATATTTTGCCCATGATTTCCTTATCT

GTAAGAGGCTGTATGGTTTATAGTCACTCAGAGAAAGTTTCGAATTTGAA

CTTGAAACCTAAGTAATTTGATCCATTGAACTTGACAAATGTCCATT →

TGGCCCCTTGAGAAGTTCTAGCTGCAGCTCAGAAGCTTCACCATTATTTA

CAGAGCAGGCAGGGAGCTTGCGTCATGAACATTATATTGATTTTATCCAG

|GTGGTTATTGAAGCCCAAGGACTGGAAGCCTTAATCGCAGCCACCCCTGA 2037

CGAGGGGGAGGAGAACCTTGACTCCTATGCTGGTATGTCAGCCATCCTCT

TTGATGTTCAGCTCAGACCTGTCACCTTTTTCAACGGATACAGTGATTTG EXON 15

ATGTCCAAAATGCTGTCAGCATCTGGCGACCCTATCAGTGTGGTGAAAGG

ACTTATTCTGCTAATAGATCATTCTCAG|GTAATTCANYCAGTCTGTGAGT 2264

ATTTATTGAGTCCCTAAACTACGCCAGGCACGTA →

(SEQ. ID. NO. 8, continued)

ATCAACACAACTCAAATGGAATTATCTACAGCAGGAGGTCAAATGTNCCA
TTGGAAAGGGGGTTAACTAAATTGTACTTATTATTTTTATAACTATTATT
ATGCTTTTTTCTTCTAG|GAACTTCAGTTACAATCTGGACTAAAAGCCAAT 2265
ATAGAGGTCCAGGGTGGTCTAGCTATTGATATTTCAGGTGCAATGGAGTT EXON 16
TAGCTTGTGGTATCGTGAGTCTAAAACCCGAGTGAAAAATAG|GTAAGTGT 2389
TTATGCATTATACATTTATGAATTACATATAAGACTATAT →
CTTGGGTATTTCTGACCTGCTGAGAGGACCTGGGTTCCAAGAATGTTTTT
CATTTTGGTCTTTGTTATGCCCATACGAAACAATGTAGTATCTTACAGAC
ACTCCCCACATCTGCAACTGAAGGCAGGGGAGAGCTCAGGGGAAGGGCAA
ACCTTCCCTGCCCAATATCTGAGACTCACCAGGCCCTGGTTACCAGCAGA
ACTCTAAGCACATCCAGGTCACCTCTGAATCCCTTAAGTGTTTCCTTCCA
GTCACTGGCATCATACGTTCAGACCCTGTAAAGTTACAGCTGTTAGTCCA
ATACCATTAAATATAATATGAACAAGTTTTTTCTTTTTTTCTCAAATGTT
TAG|GGTGACTGTGGTAATAACCACTGACATCACAGTGGACTCCTCTTTTG 2390
TGAAAGCTGGCCTGGAAACCAGTACAGAAACAGAAGCAGGTTTGGAGTTT EXON 17
ATCTCCACAGTGCAGTTTTCTCAGTACCCATTCTTAGTTTGCATGCAGAT
GGACAAGGATGAAGCTCCATTCAG|GTAAGATGCAGCGTACAGGTCATGTT 2560
CCAGGACCATCCCCAGTGCACCAGGAACTTGCATTCAGTTTAGAACATTC
AGTTTCAGAATTAAAACAAAACAGTAGAAACCCAGGGAAAGATGAATTTT
CTTTAAATGAGTAGAAGAATAATTGATAAGGCCAAAAAAAGTCAGTTTCT
GGATACCAAAAAAAAATCTAATGACTAGTTCATGTGATTCTGGAGATAG
TTATCATATTCTAATCCAGAAACAATTT →

(SEQ. ID. NO. 8, continued)

**TGCTTTGGAACAGAAACTTCAAGTACATTCAGTAACTTGGCTGGAGAGGT**

**ATAGGGTGACTTAACTGTGTGTGTAATTCTGTTAATGTTGCTGTTGTTGT**

**ACAG**|GCAATTTGAGAAAAAGTACGAAAGGCTGTCCACAGGCAGAGGTTAT 2561

GTCTCTCAGAAAAGAAAAGAAAGCGTATTAGCAGGATGTGAATTCCCGCT

CCATCAAGAGAACTCAGAGATGTGCAAAGTGGTGTTTGCCCCTCAGCCGG

ATAGTACTTCCAGCGGATGGTTTTGAAACTGACCTGTGATATTTTACTTG

AATTTGTCTCCCCGAAAGGGACACAATGTGGCATGACTAAGTACTTGCTC

TCTGAGAGCACAGCGTTTACATATTTACCTGTATTTAAGATTTTTGTAAA EXON 18

AAGCTACAAAAAACTGCAGTTTGATCAAATTTGGGTATATGCAGTATGCT

ACCCACAGCGTCATTTTGAATCATCATGTGACGCTTTCAACAACGTTCTT

AGTTTACTTATACCTCTCTCAAATCTCATTTGGTACAGTCAGAATAGTTA

TTCTCTAAGAGGAAACTAGTGTTTGTTAAAAACAAAAATAAAAACAAAAC

CACACAAGGAGAACCCAATTTTGTTTCAACAATTTTTGATCAATGTATAT

GAAGCTCTTGATAGGACTTCCTTAAGCATGACGGGAAAACCAAACACGTT

CCCTAATCAGGAAAAAAAAAAAAAAAAAAGGTAGGACACAACCAACCCAT

TTTTTTTCTCTTTTTTTGGAGTTGGGGGCCCAGGGAGAAGGGACAAGACT

TTTAAAAGACTTGTTAGCCAACTTCAAGAATTAATATTTATGTCTCTGTT

ATTGTTAGTTTTAAGCCTTAAGGTAGAAGGCACATAGAAATAACATCTCA

TCTTTCTGCTGACCATTTTAGTGAGGTTGTTCCAAAGACATTCAGGTCTC

TACCTCCAGCCCTGCAAAAATATTGGACCTAGCACAGAGGAATCAGGAAA

ATTAATTTCAGAAACTCCATTTGATTTTTCTTTTGCTGTGTCTTTTTGAG

ACTGTAATATGGTACACTGTCCTCTAAGGGACATCCTCATTTTATCTCAC

(SEQ. ID. NO. 8, continued)

CTTTTTGGGGGTGAGAGCTCTAGTTCATTTAACTGTACTCTGCACAATAG
CTAGGATGACTAAGAGAACATTGCTTCAAGAAACTGGTGGATTTGGATTT
CCAAAATATGAAATAAGGAAAAAAATGTTTTTATTTGTATGAATTAAAAG
ATCCATGTTGAACATTTGCAAATATTTATTAATAAACAGATGTGGTGATA
AACCCAAAACAAATGACAGGTCCTTATTTTCCACTAAACACAGACACATG
AAATGAAAGTTTAGCTAGCCCACTATTTGTTGTAAATTGAAAACGAAGTG
TGATAAAATAAATATGTAGAAATCATATTGAATTC

## Claims

1. A nucleic acid molecule comprising a nucleic acid sequence (i) coding for all or art of the high molecular weight subunit of microsomal triglyceride transfer protein and (ii) having the nucleotide sequence shown in SEQ ID NOS: 1, 2, 8, 1 together with 5, 2 together with 7, the first 108 bases of 2 together with 8, or the first 108 bases of 2 together with 7 and 8.

2. The nucleic acid molecule according to claim 1 which is a DNA molecule and wherein the nucleic acid sequence is a DNA sequence.

3. A DNA molecule comprising a DNA sequence which

   (a) is complementary to the DNA sequence according to claim 2; or
   (b) hybridizes to the DNA sequence according to claim 2 and encodes a protein having the biological activity or antigenic activity of the high molecular weight subunit of microsomal triglyceride transfer protein; or
   (c) is an allelic derivative of any one of the sequences according to claim 2 or (b); or
   (d) is degenerated to any of the sequences according to claim 2, (b) or (c).

4. An expression vector comprising the DNA sequence according to any one of claims 1 to 3 wherein said DNA sequence is under the control of regulatory elements suitable to direct expression in an appropriate host cell.

5. A prokaryotic or eukaryotic host cell comprising the expression vector according to claim 4.

6. A method for producing a polypeptide molecule having all or part of the high molecular weight subunit of microsomal triglyceride transfer protein, which comprises culturing a host cell according to claim 5 under conditions permitting expression of the polypeptide.

7. A method for detecting a nucleic acid sequence according to any one of claims 1 to 3 coding for all or part of the high molecular weight subunit of microsomal triglyceride transfer protein or a related nucleic acid sequence, which comprises:

   (a) contacting the nucleic acid sequence with a detectable marker which binds specifically to at least part of the

nucleic acid sequence, and
(b) detecting the marker so bound;

wherein the presence of bound marker indicates the presence of the nucleic acid sequence.

**8.** The method according to claim 7 wherein the detectable marker is a nucleotide sequence of at least about 15 nucleotides in length complementary to at least a portion of the nucleic acid sequence coding for the high molecular weight subunit of microsomal triglyceride transfer protein.

**9.** The method according to claim 8 wherein the nucleotide sequence is a genomic DNA sequence, a cDNA sequence, an RNA sequence, a sense RNA sequence or an antisense RNA sequence.

**10.** The method according to claim 9, wherein the detectable marker is labeled with a radioisotope and the detecting step is carried out by autoradiography.

**11.** The nucleic acid molecule according to any one of claims 1 to 3 or the high molecular weight subunit of microsomal triglyceride transfer protein encoded by a nucleic acid molecule of any one of claims 1 to 3 for use in therapy or diagnosis.

**12.** A pharmaceutical composition comprising the nucleic acid molecule according to any one of claims 1 to 3 or the high molecular weight subunit of microsomal triglyceride transfer protein encoded by a nucleic acid molecule of any one of claims 1 to 3, optionally together with a pharmaceutically acceptable carrier.

**13.** Use of a therapeutically effective amount of (a) an inhibitor of microsomal triglyceride transfer protein activity or (b) the DNA molecule of claim 3(a) which decreases the amount of microsomal triglyceride transfer protein for the preparation of a pharmaceutical composition for preventing, stabilizing or causing regression of atherosclerosis in a mammalian species, wherein said inhibitor is 1-[3-(6-fluoro-1-tetralanyl)methyl]-4-O-methoxyphenyl piperazine.

**14.** Use of a therapeutically effective amount of (a) an inhibitor of microsomal triglyceride transfer protein activity or (b) the DNA molecule of claim 3(a) which decreases the amount of microsomal triglyceride transfer protein for the preparation of a pharmaceutical composition for decreasing serum lipid levels in a mammalian species, wherein said inhibitor is 1-[3-(6-fluoro-1-tetralanyl)methyl]-4-O-methoxyphenylpiperazine.

**15.** The use of claim 14, wherein the lipid is cholesterol, triglyceride, cholesteryl ester, or phosphatidylcholine.

**16.** Use of a therapeutically effective amount of (a) an inhibitor of microsomal triglyceride transfer protein activity or (b) the DNA molecule of claim 3(a) which decreases the amount microsomal triglyceride transfer protein for the preparation of a pharmaceutical composition for preventing or treating pancreatitis in a mammalian species, wherein said inhibitor is 1-[3-(6-fluoro-1-tetralanyl)methyl]-4-O-methoxyphenyl piperazine.

**17.** Use of a therapeutically effective amount of (a) an inhibitor of microsomal triglyceride transfer protein activity or (b) the DNA molecule of claim 3(a) which decreases the amount of microsomal triglyceride transfer protein for the preparation of a pharmaceutical composition for preventing or treating obesity in a mammalian species, wherein said inhibitor is 1-[3-(6-fluoro-1-tetralanyl)methyl]-4-O-methoxyphenyl piperazine.

**18.** A process for the preparation of a nucleic acid molecule or a DNA molecule according to any one of claims 1 to 3 or 11 which comprises using any of the following techniques:

(a) screening a genomic or cDNA library for the respective DNA sequence and isolating it, or
(b) chemically synthesizing the DNA sequence, or
(c) synthesizing the DNA sequence by the polymerase chain reaction (PCR).

**19.** A process for the preparation of the pharmaceutical composition of claim 12 which comprises combining the nucleic acid molecule or the protein with the pharmaceutically acceptable carrier.

**Patentansprüche**

1. Nucleinsäuremolekül, umfassend eine Nucleinsäuresequenz, die (i) die gesamte oder einen Teil der Untereinheit mit hohem Molekulargewicht des mikrosomalen Triglyceridtransferproteins codiert und (ii) die in SEQ ID NOs: 1, 2, 8 dargestellte Nucleotidsequenz, die Nucleotidsequenz von SEQ ID NO: 1 zusammen mit SEQ NO: 5, SEQ ID NO: 2 zusammen mit SEQ ID NO: 7, die ersten 108 Basen von SEQ ID NO: 2 zusammen mit SEQ ID NO: 8 oder die ersten 108 Basen von SEQ ID NO: 2 zusammen mit SEQ ID NO: 7 und 8 besitzt.

2. Nucleinsäuremolekül nach Anspruch 1, das ein DNA-Molekül ist, und wobei die Nucleinsäuresequenz eine DNA-Sequenz ist.

3. DNA-Molekül, das eine DNA-Sequenz umfasst, die

   (a) zu der DNA-Sequenz nach Anspruch 2 komplementär ist; oder
   (b) mit der DNA-Sequenz nach Anspruch 2 hybridisiert und ein Protein codiert, das die biologische Aktivität oder antigene Aktivität der Untereinheit mit hohem Molekulargewicht des mikrosomalen Triglyceridtransferproteins besitzt; oder
   (c) ein allelisches Derivat von einer der Sequenzen nach Anspruch 2 oder (b) ist; oder
   (d) im Vergleich zu einer der Sequenzen nach Anspruch 2, (b) oder (c) degeneriert ist.

4. Expressionsvektor, der die DNA-Sequenz nach einem der Ansprüche 1 bis 3 umfasst, wobei die DNA-Sequenz unter der Kontrolle eines regulatorischen Elements steht, das geeignet ist, die Expression in einer geeigneten Wirtszelle zu vermitteln.

5. Prokaryontische oder eukaryontische Wirtszelle, die den Expressionsvektor nach Anspruch 4 umfasst.

6. Verfahren zur Herstellung eines Polypeptidmoleküls, das die gesamte oder einen Teil der Untereinheit mit hohem Molekulargewicht des mikrosomalen Triglyceridtransferproteins besitzt, wobei das Verfahren das Kultivieren einer Wirtszelle nach Anspruch 5 unter Bedingungen, die die Expression des Polypeptids erlauben, umfasst.

7. Verfahren zum Nachweis einer Nucleinsäuresequenz nach einem der Ansprüche 1 bis 3, die die gesamte oder einen Teil der Untereinheit mit hohem Molekulargewicht des mikrosomalen Triglyceridtransferproteins codiert, oder einer verwandten Nucleinsäuresequenz, umfassend:

   (a) Inkontaktbringen der Nucleinsäuresequenz mit einem nachweisbaren Marker, der spezifisch an wenigstens einen Teil der Nucleinsäuresequenz bindet, und
   (b) Nachweis des derart gebundenen Markers;

   wobei die Anwesenheit des gebundenen Markers die Anwesenheit der Nucleinsäuresequenz anzeigt.

8. Verfahren nach Anspruch 7, wobei der nachweisbare Marker eine Nucleotidsequenz mit einer Länge von mindestens etwa 15 Nucleotiden ist, die zu mindestens einem Teil der die Untereinheit mit hohem Molekulargewicht des mikrosomalen Triglyceridtransferproteins codierenden Nucleinsäuresequenz komplementär ist.

9. Verfahren nach Anspruch 8, wobei die Nucleotidsequenz eine genomische DNA-Sequenz, eine cDNA-Sequenz, eine RNA-Sequenz, eine Sense-RNA-Sequenz oder eine Antisense-RNA-Sequenz ist.

10. Verfahren nach Anspruch 9, wobei der nachweisbare Marker mit einem Radioisotop markiert ist und der Nachweisschritt durch Autoradiographie erfolgt.

11. Nucleinsäuremolekül nach einem der Ansprüche 1 bis 3 oder die Untereinheit mit hohem Molekulargewicht des mikrosomalen Triglyceridtransferproteins, das von einem Nucleinsäuremolekül nach einem der Ansprüche 1 bis 3 codiert wird, zur Verwendung in der Therapie oder Diagnose.

12. Arzneimittel, umfassend das Nucleinsäuremolekül nach einem der Ansprüche 1 bis 3 oder die Untereinheit mit hohem Molekulargewicht des mikrosomalen Triglyceridtransferproteins, das von einem Nucleinsäuremolekül nach einem der Ansprüche 1 bis 3 codiert wird, gegebenenfalls zusammen mit einem pharmazeutisch verträglichen Träger.

**13.** Verwendung einer therapeutisch wirksamen Menge (a) eines Inhibitors der Aktivität des mikrosomalen Triglyceridtransferproteins oder (b) des DNA-Moleküls nach Anspruch 3(a), der/das die Menge des mikrosomalen Triglyceridtransferproteins vermindert, für die Herstellung eines Arzneimittels zur Verhinderung, Stabilisierung oder Regression von Atherosklerose in einer Säugerspezies, wobei der Inhibitor 1-[3-(6-Fluor-1-tetralanyl)methyl]-4-O-methoxyphenyl-piperazin ist.

**14.** Verwendung einer therapeutisch wirksamen Menge (a) eines Inhibitors der Aktivität des mikrosomalen Triglyceridtransferproteins oder (b) des DNA-Moleküls nach Anspruch 3(a), der/das die Menge des mikrosomalen Triglyceridtransferproteins vermindert, für die Herstellung eines Arzneimittels zur Verringerung von Serumlipidspiegeln in einer Säugerspezies, wobei der Inhibitor 1-[3-(6-Fluor-1-tetralanyl)methyl]-4-O-methoxyphenyl-piperazin ist.

**15.** Verwendung nach Anspruch 14, wobei das Lipid Cholesterol, Triglycerid, Cholesterylester oder Phosphatidylcholin ist.

**16.** Verwendung einer therapeutisch wirksamen Menge (a) eines Inhibitors der Aktivität des mikrosomalen Triglyceridtransferproteins oder (b) des DNA-Moleküls nach Anspruch 3(a), der/das die Menge des mikrosomalen Triglyceridtransferproteins vermindert, für die Herstellung eines Arzneimittels zur Verhinderung oder Behandlung von Pankreatitis in einer Säugerspezies, wobei der Inhibitor 1-[3-(6-Fluor-1-tetralanyl)methyl]-4-O-methoxyphenylpiperazin ist.

**17.** Verwendung einer therapeutisch wirksamen Menge (a) eines Inhibitors der Aktivität des mikrosomalen Triglyceridtransferproteins oder (b) des DNA-Moleküls nach Anspruch 3(a), der/das die Menge des mikrosomalen Triglyceridtransferproteins vermindert, für die Herstellung eines Arzneimittels zur Verhinderung oder Behandlung von Obesitas einer Säugerspezies, wobei der Inhibitor 1-[3-(6-Fluor-1-tetralanyl)methyl]-4-O-methoxyphenyl-piperazin ist.

**18.** Verfahren zur Herstellung eines Nucleinsäuremoleküls oder eines DNA-Moleküls nach einem der Ansprüche 1 bis 3 oder 11, umfassend die Verwendung einer der folgenden Methoden:

(a) Durchmustern einer genomischen oder cDNA-Bibliothek nach der betreffenden DNA-Sequenz und Isolieren dieser DNA-Sequenz; oder
(b) chemische Synthese der DNA-Sequenz; oder
(c) Synthese der DNA-Sequenz durch die Polymerasekettenreaktion (PCR).

**19.** Verfahren zur Herstellung des Arzneimittels nach Anspruch 12, umfassend die Kombination des Nucleinsäuremoleküls oder des Proteins mit dem pharmazeutisch verträglichen Träger.

## Revendications

**1.** Molécule d'acide nucléique comprenant une séquence d'acides nucléiques (i) codant tout ou partie de la sous-unité de haut poids moléculaire de la protéine microsomale de transfert des triglycérides et (ii) ayant la séquence nucléotidique indiquée dans SEQ ID NOS: 1,2,8, 1 avec 5, 2 avec 7, les 108 premières bases de 2 avec 8, ou les 108 premières bases de 2 avec 7 et 8.

**2.** Molécule d'acide nucléique selon la revendication 1, qui est une molécule d'ADN et dans laquelle la séquence d'acides nucléiques est une séquence d'ADN.

**3.** Molécule d'ADN comprenant une séquence d'ADN qui

(a) est complémentaire à la séquence d'ADN selon la revendication 2; ou
(b) s'hybride à la séquence d'ADN selon la revendication 2 et code une protéine ayant l'activité biologique ou l'activité antigénique de la sous-unité de haut poids moléculaire de la protéine microsomale de transfert des triglycérides; ou
(c) est un dérivé allélique de l'une quelconque des séquences selon la revendication 2 ou (b); ou
(d) est dégénérée pour l'une quelconque des séquences selon la revendication 2, (b) ou (c).

**4.** Vecteur d'expression comprenant la séquence d'ADN selon l'une quelconque des revendications 1 à 3, dans lequel la séquence d'ADN est sous le contrôle d'éléments régulateurs convenant pour l'expression directe dans une cellule

hôte appropriée.

5. Cellule hôte procaryote ou eucaryote comprenant le vecteur d'expression selon la revendication 4.

6. Méthode de production d'une molécule polypeptidique ayant tout ou partie de la sous-unité de haut poids moléculaire de la protéine microsomale de transfert des triglycérides, qui comprend la culture d'une cellule hôte selon la revendication 5 dans des conditions permettant l'expression du polypeptide.

7. Méthode de détection d'une séquence d'acides nucléiques selon l'une quelconque des revendications 1 à 3 codant pour tout ou partie de la sous-unité de haut poids moléculaire de la protéine microsomale de transfert des triglycérides ou d'une séquence d'acides nucléiques apparentée, qui comprend:

   (a) la mise en contact de la séquence d'acides nucléiques avec un marqueur détectable qui se lie spécifiquement à au moins une partie de la séquence d'acides nucléiques, et
   (b) la détection du marqueur ainsi lié;

   dans laquelle la présence du marqueur lié indique la présence de la séquence d'acides nucléiques.

8. Méthode selon la revendication 7, dans laquelle le marqueur détectable est une séquence nucléotidique d'au moins 15 nucléotides de longueur complémentaire à moins une portion de la séquence d'acides nucléiques codant la sous-unité de haut poids moléculaire de la protéine microsomale de transfert des triglycérides.

9. Méthode selon la revendication 8, dans laquelle la séquence nucléotidique est une séquence d'ADN génomique, une séquence d'ADNc, une séquence d'ARN, une séquence d'ARN sens ou une séquence d'ARN antisens.

10. Méthode selon la revendication 9, dans laquelle le marqueur détectable est marqué avec un isotope radioactif, et dans laquelle l'étape de détection est effectuée par autoradiographie.

11. Molécule d'acide nucléique selon l'une quelconque des revendications 1 à 3 ou la sous-unité de haut poids moléculaire de la protéine microsomale de transfert des triglycérides codée par une molécule de l'acide nucléique selon l'une quelconque des revendications 1 à 3 pour utilisation en thérapie ou diagnostic.

12. Composition pharmaceutique comprenant la molécule d'acide nucléique selon l'une quelconque des revendications 1 à 3 ou la sous-unité de haut poids moléculaire de la protéine microsomale de transfert des triglycérides codée par une molécule de l'acide nucléique selon l'une quelconque des revendications 1 à 3, facultativement ensemble avec un support pharmaceutiquement acceptable.

13. Utilisation d'une quantité thérapeutiquement efficace (a) d'un inhibiteur de l'activité de la protéine microsomale de transfert des triglycérides ou (b) de la molécule d'ADN de la revendication 3(a) qui diminue la quantité de la protéine microsomale de transfert des triglycérides pour la préparation d'une composition pharmaceutique pour prévenir, stabiliser ou faire régresser l'athérosclérose chez une espèce mammifère, où ledit inhibiteur est 1-[3-(6-fluoro-1-tétralanyl)méthyl]-4-O-méthoxyphényl pipérazine.

14. Utilisation d'une quantité thérapeutiquement efficace (a) d'un inhibiteur de l'activité de la protéine microsomale de transfert des triglycérides ou (b) de la molécule d'ADN de la revendication 3(a) qui diminue la quantité de la protéine microsomale de transfert des triglycérides pour la préparation d'une composition pharmaceutique pour diminuer les taux de lipides sériques chez une espèce mammifère, où ledit inhibiteur est 1-[3-(6-fluoro-1-tétralanyl)méthyl]-4-O-méthoxyphényl pipérazine.

15. Utilisation selon la revendication 14, dans laquelle le lipide est le cholestérol, un triglycéride, un ester cholestérylique ou la phosphatidylcholine.

16. Utilisation d'une quantité thérapeutiquement efficace (a) d'un inhibiteur de l'activité de la protéine microsomale de transfert des triglycérides ou (b) de la molécule d'ADN de la revendication 3(a) qui diminue la quantité de la protéine microsomale de transfert des triglycérides pour la préparation d'une composition pharmaceutique pour prévenir ou traiter la pancréatite chez une espèce mammifère, où ledit inhibiteur est 1-[3-(6-fluoro-1-tétralanyl)méthyl]-4-O-méthoxyphényl pipérazine.

**17.** Utilisation d'une quantité thérapeutiquement efficace (a) d'un inhibiteur de l'activité de la protéine microsomale de transfert des triglycérides ou (b) de la molécule d'ADN de la revendication 3(a) qui diminue la quantité de la protéine microsomale de transfert des triglycérides pour la préparation d'une composition pharmaceutique pour prévenir ou traiter l'obésité chez une espèce mammifère, où ledit inhibiteur est 1-[3-(6-fluoro-1-tétralanyl)méthyl]-4-O-méthoxyphényl pipérazine.

**18.** Procédé de préparation d'une molécule d'acide nucléique ou d'une molécule d'ADN selon l'une quelconque des revendications 1 à 3 ou 11, qui comprend l'utilisation de l'une quelconque des techniques suivantes:

(a) le criblage d'une bibliothèque génomique ou d'ADNc pour la séquence respective d'ADN et son isolation, ou
(b) la synthèse chimique de la séquence d'ADN, ou
(c) la synthèse de la séquence d'ADN par la réaction en chaîne à la polymérase (PCR).

**19.** Procédé de préparation de la composition pharmaceutique selon la revendication 12, qui comprend la combinaison de la molécule d'acide nucléique ou de la protéine avec le support pharmaceutiquement acceptable.

Figure 1

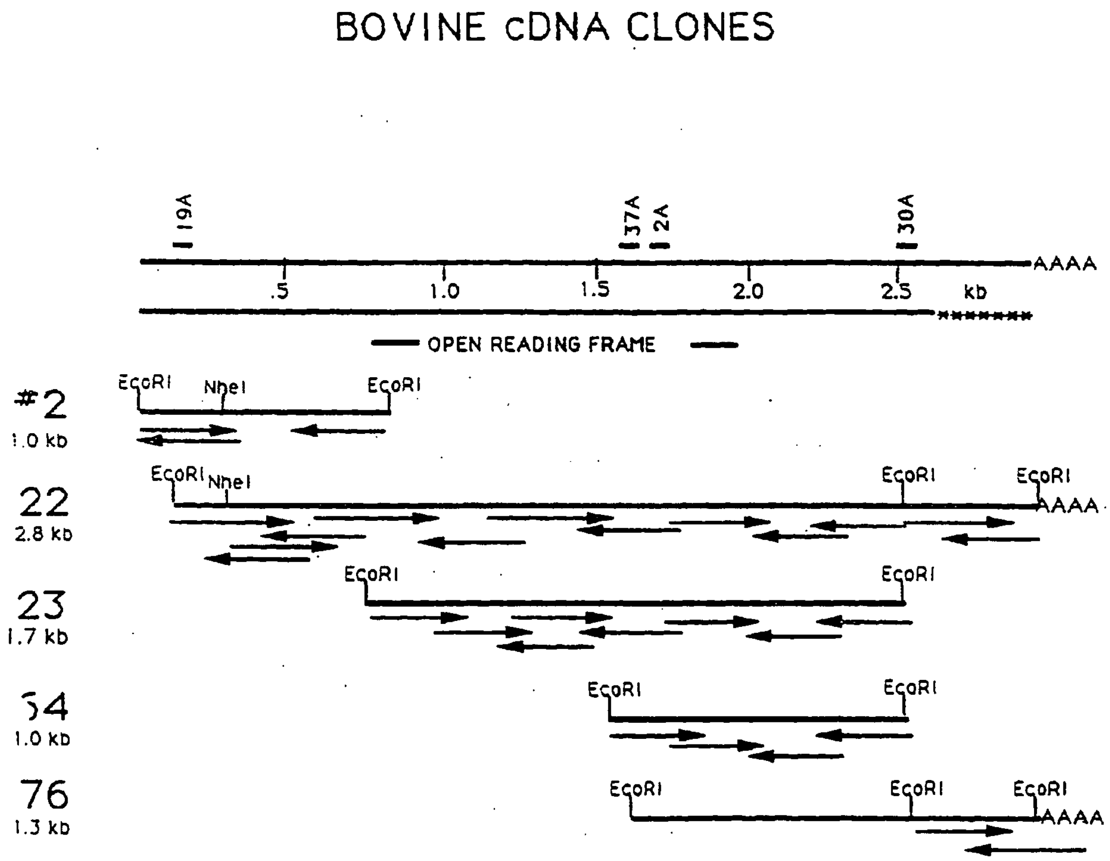
BOVINE cDNA CLONES
19A
37A
2A
30A
AAAA
.5
1.0
1.5
2.0
2.5
kb
OPEN READING FRAME
#2
1.0 kb
EcoRI
NheI
EcoRI
22
2.8 kb
EcoRI
NheI
EcoRI
EcoRI
AAAA
23
1.7 kb
EcoRI
EcoRI
54
1.0 kb
EcoRI
EcoRI
76
1.3 kb
EcoRI
EcoRI
EcoRI
AAAA

Figure 2

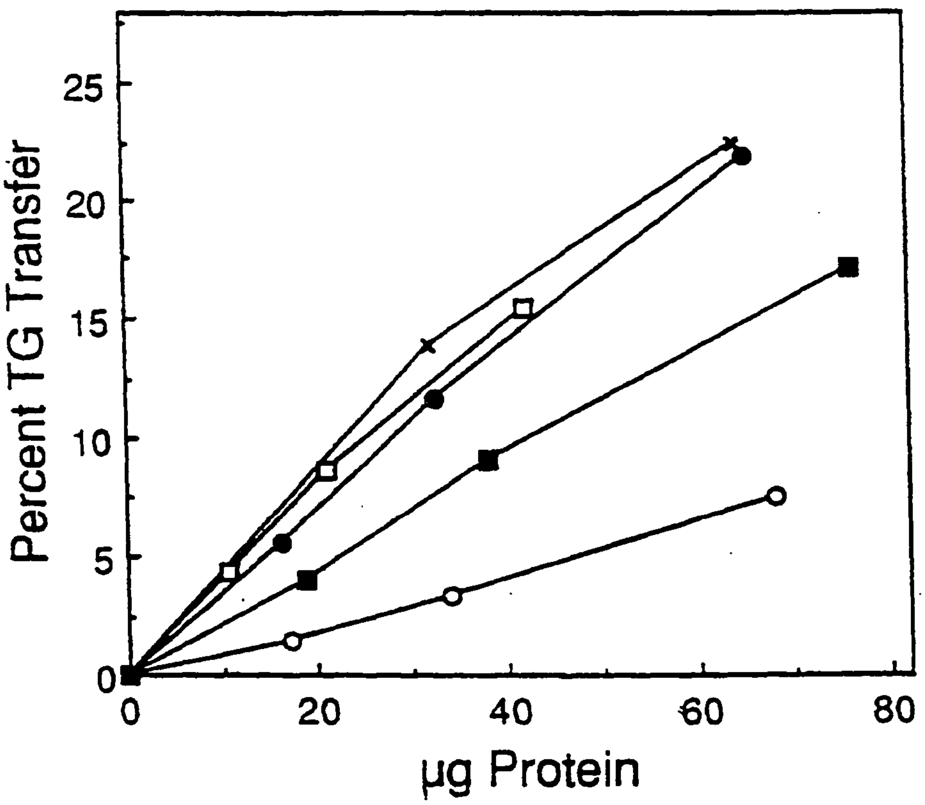

Figure 3

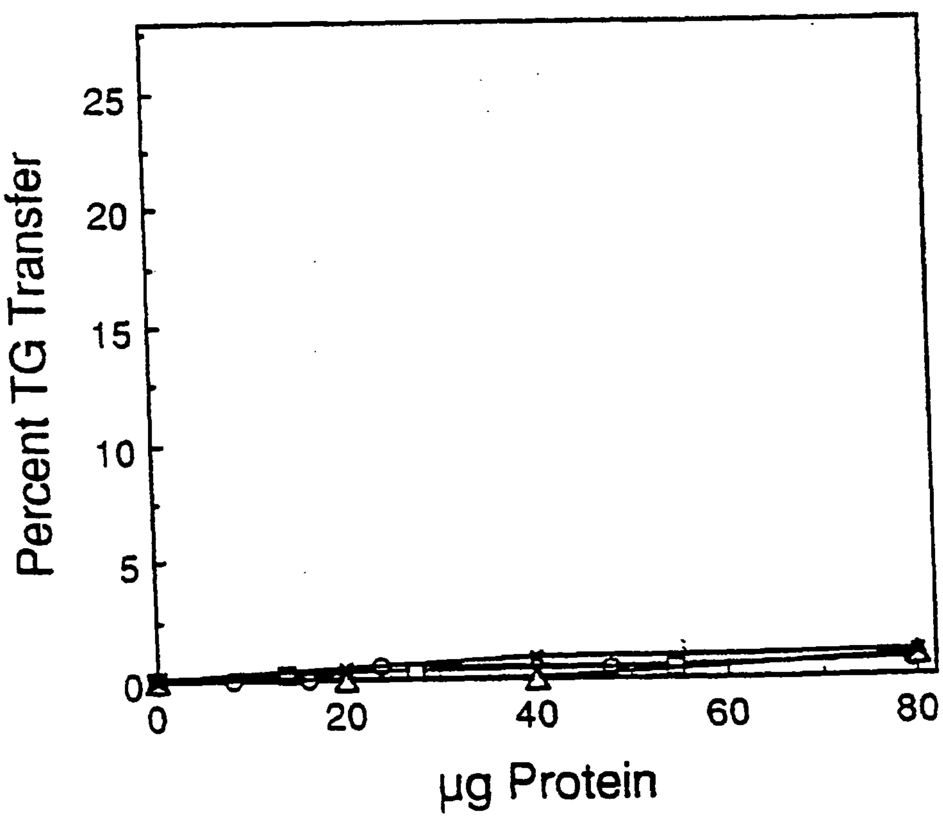

Figure 4

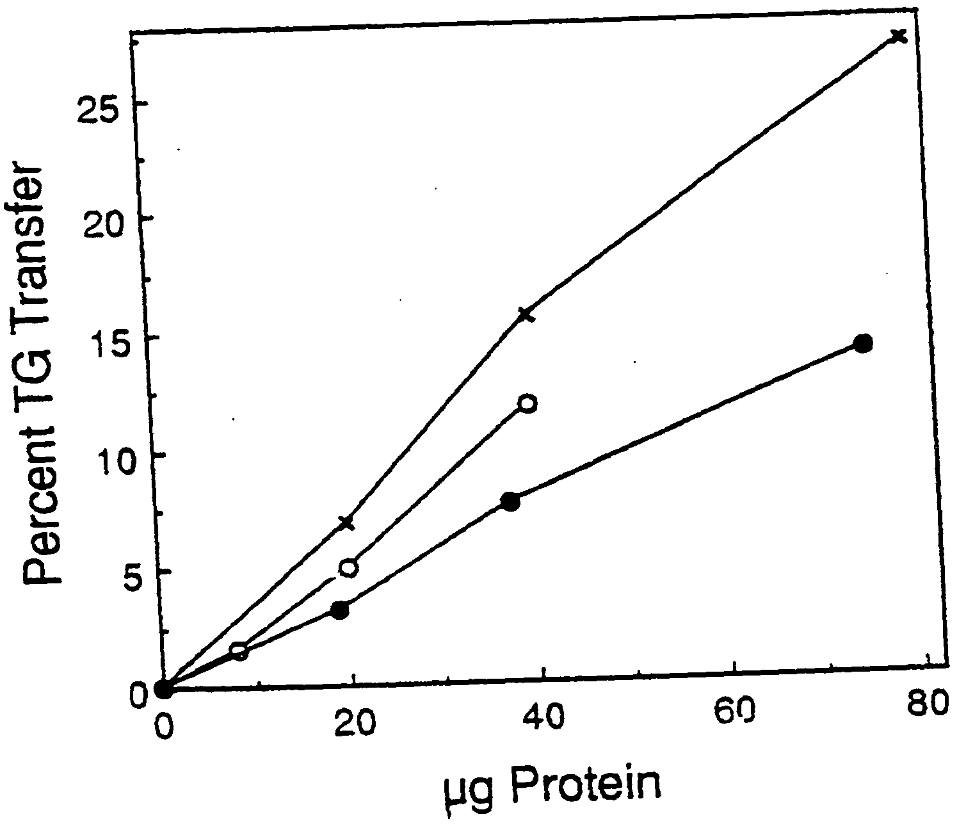

Figure 5

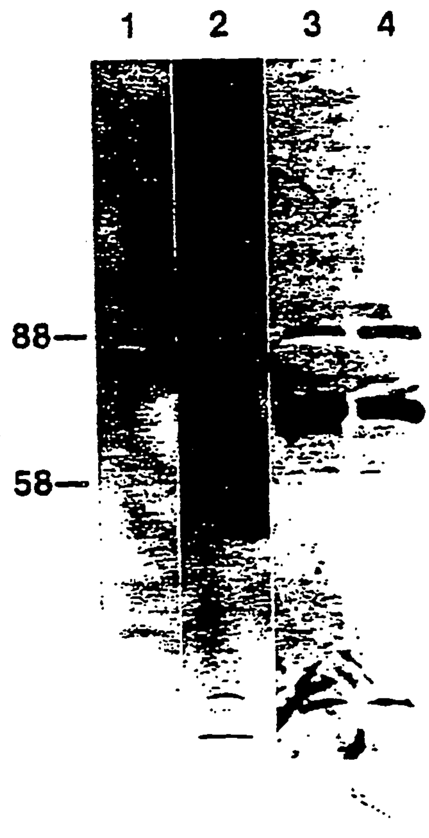

Figure 6

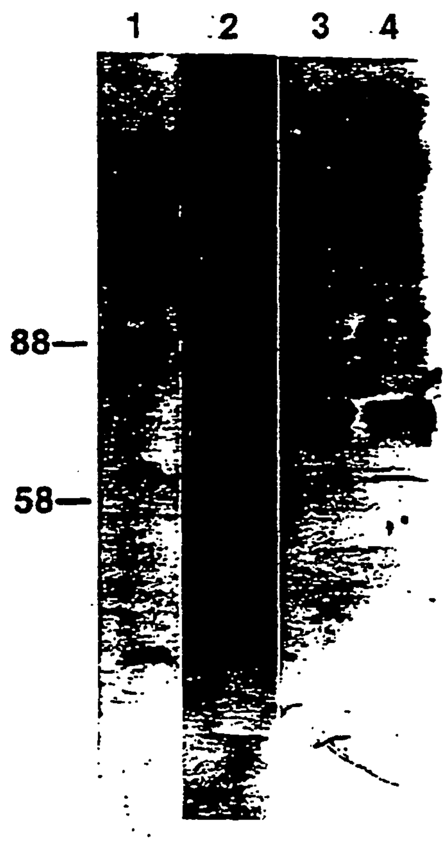

Figure 7

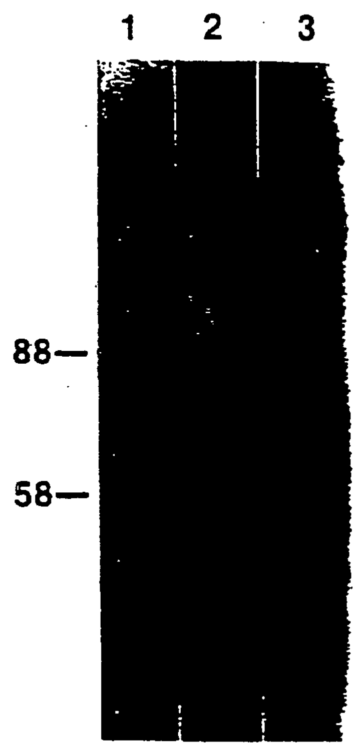

Figure 8

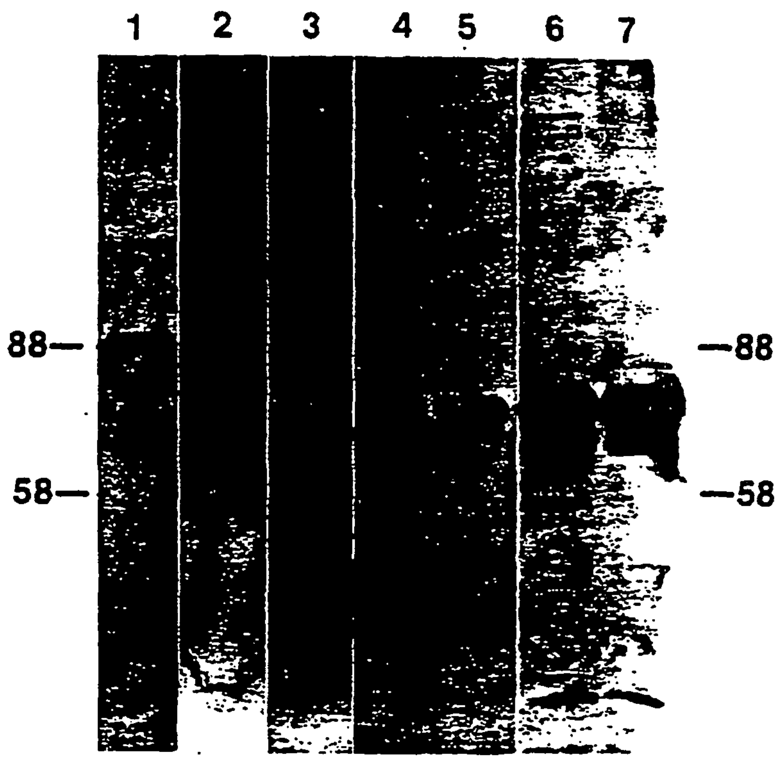

Figure 9

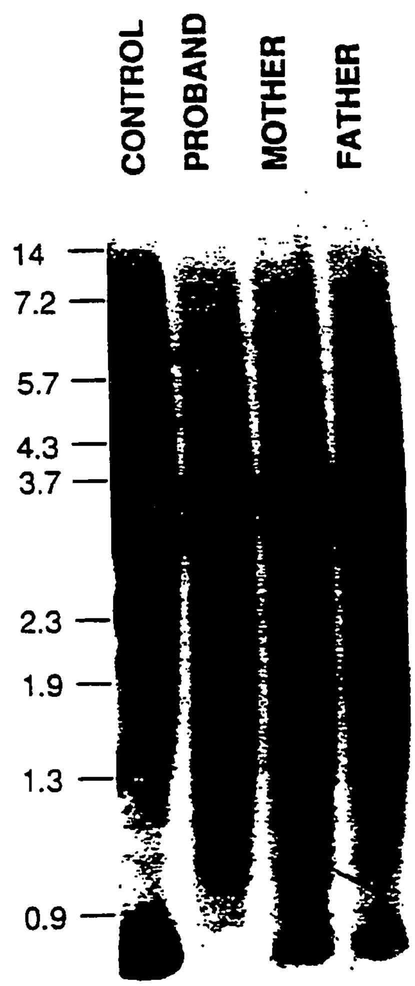

Figure 10

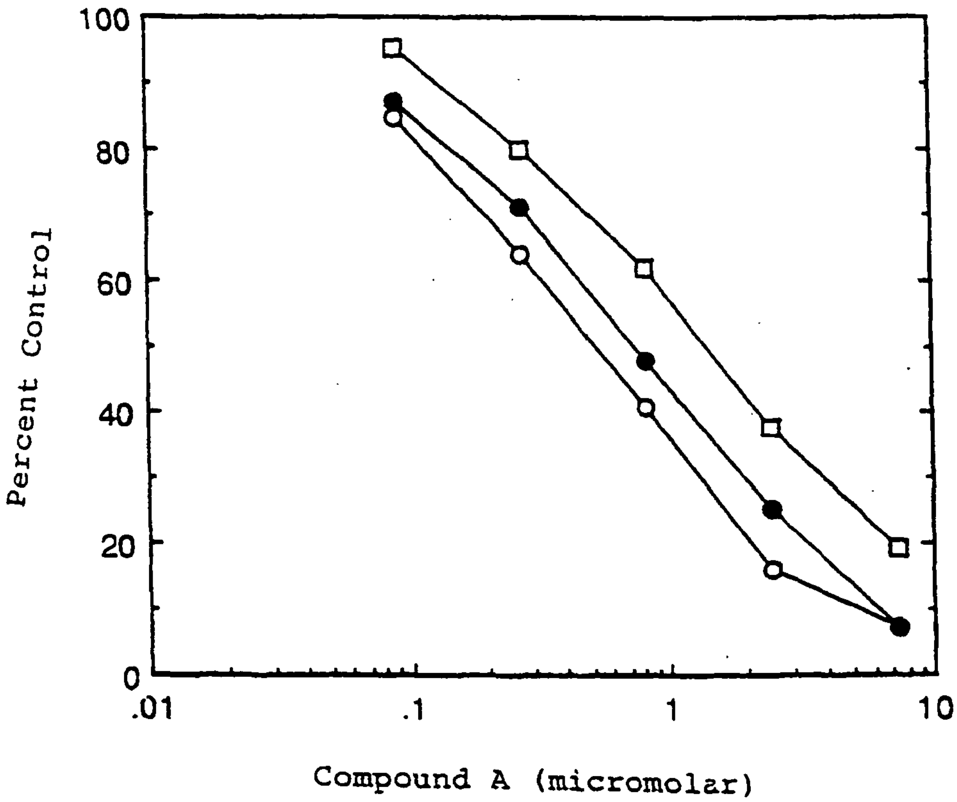
100
80
60
40
20
0
Percent Control
.01
.1
1
10
Compound A (micromolar)

Figure 11

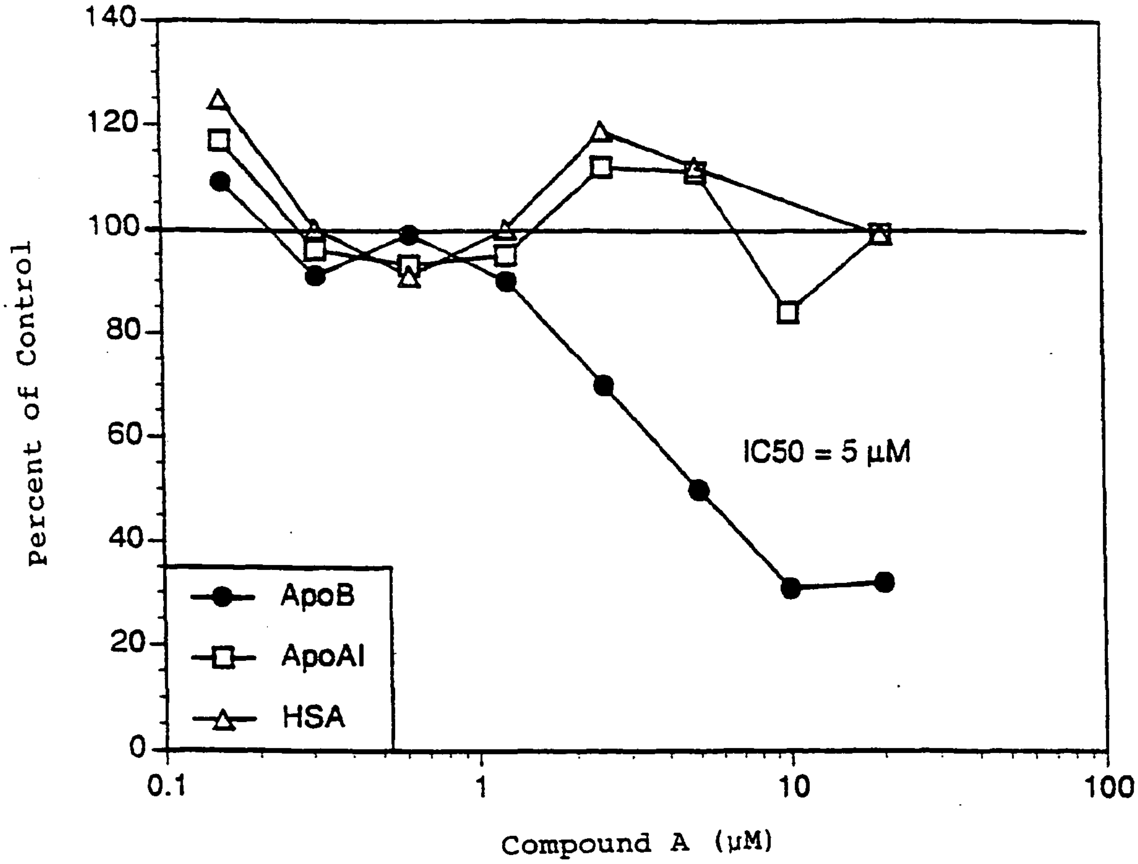
140
120
100
80
60
40
20
0
Percent of Control
IC50 = 5 µM
ApoB
ApoAI
HSA
0.1
1
10
100
Compound A (µM)

Figure 12

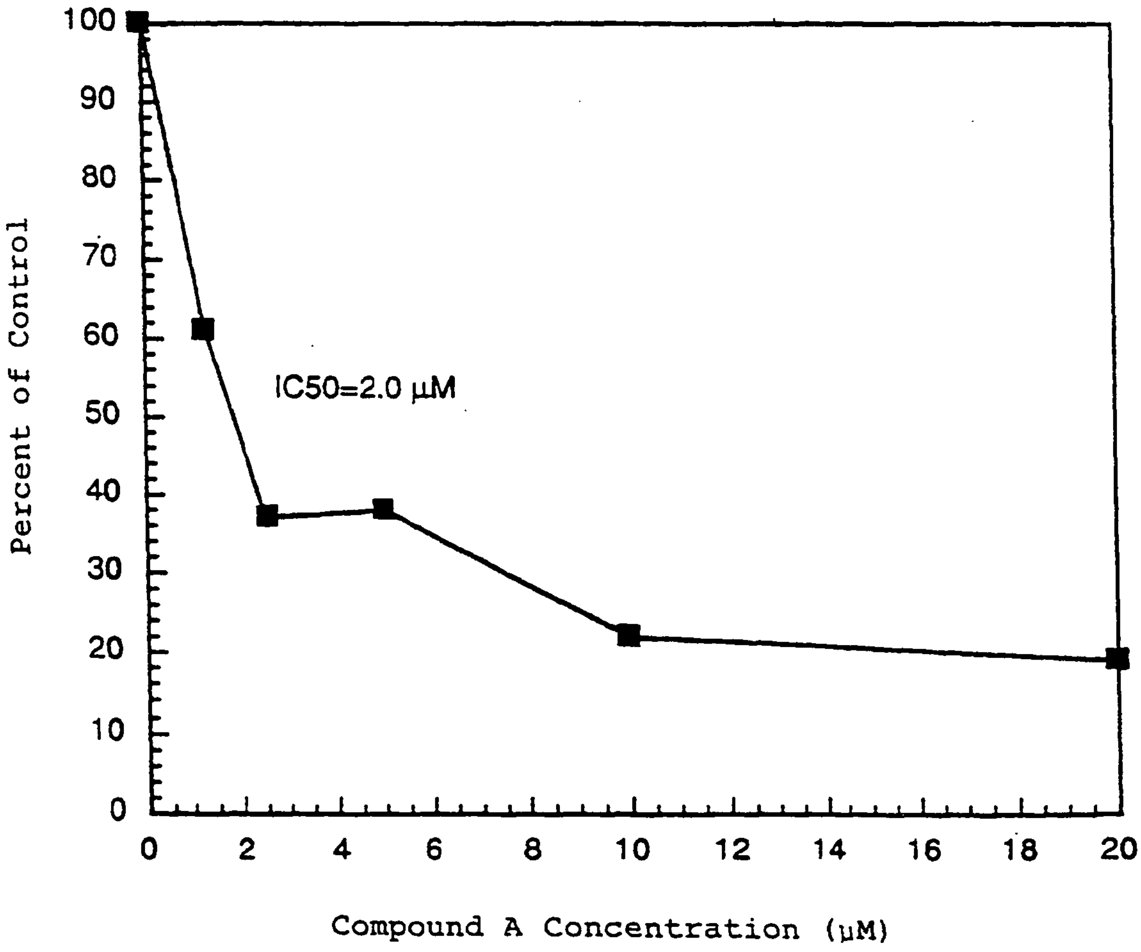
100
90
80
70
60
50
40
30
20
10
0
Percent of Control
IC50=2.0 μM
0
2
4
6
8
10
12
14
16
18
20
Compound A Concentration (μM)

Figure 13

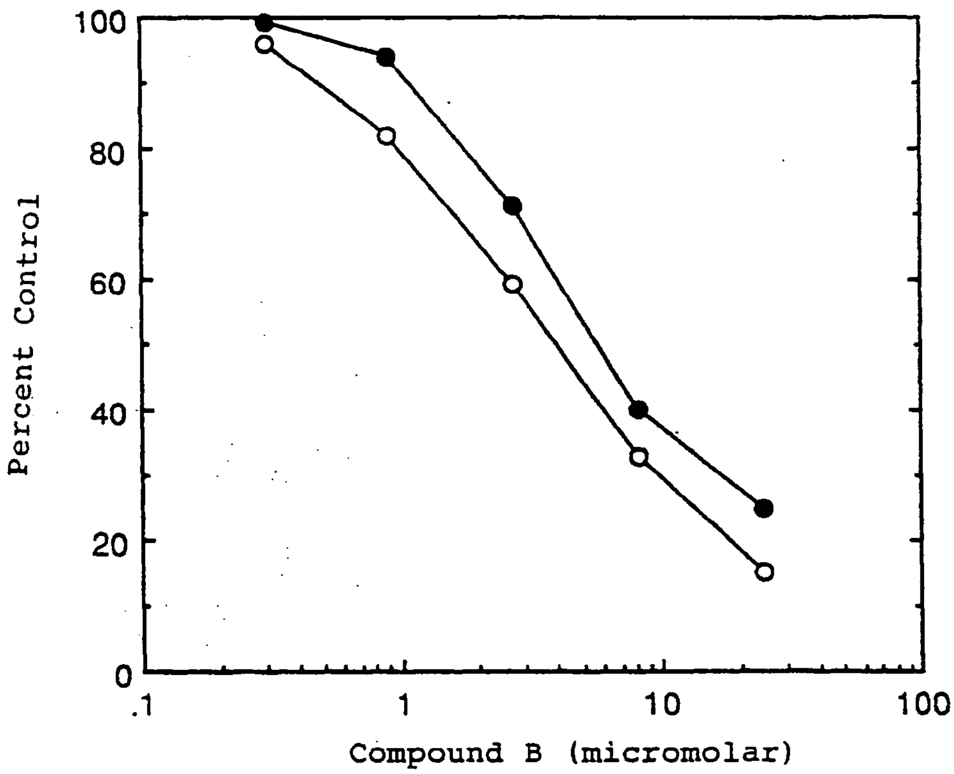
100
80
60
40
20
0
Percent Control
.1
1
10
100
Compound B (micromolar)

Figure 14

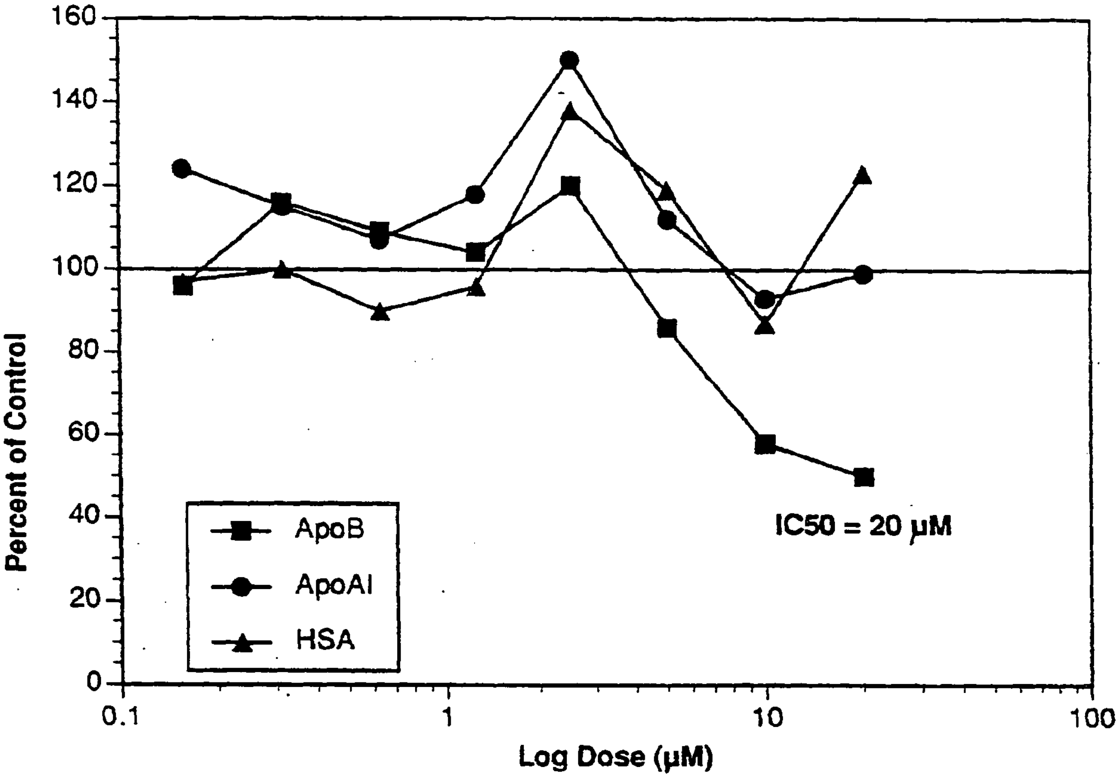
160
140
120
100
80
60
40
20
0
Percent of Control
0.1
1
10
100
Log Dose (µM)
ApoB
ApoAI
HSA
IC50 = 20 µM

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Wetterau ; Zilversmit.** *Chem. Phys. Lipids,* 1985, vol. 38, 205-22
- **Wetterau et al.** *J. Biol. Chem,* 1990, vol. 265, 9800-7
- **Bulleid ; Freedman.** *Nature,* 1988, vol. 335, 649-51
- **Koivu et al.** *J. Biol. Chem.,* 1987, vol. 262, 6447-9
- **Wetterau et al.** *Biochemistry,* 1991, vol. 30, 9728-35
- **Wetterau ; Zilversmit.** *Biochem. Biophys. Acta,* 1986, vol. 875, 610-7
- **Kane ; Havel.** The Metabolic Basis of Inherited Disease. 1989, 1139-64
- **Schaefer et al.** *Clin. Chem.,* 1988, vol. 34, 9-12
- **Talmud et al.** *J. Clin. Invest.,* 1988, vol. 82, 1803-6
- **Huang et al.** *Am. J. Hum. Genet.,* 1990, vol. 46, 1141-8
- **Bostrom et al.** *J. Biol. Chem.,* 1988, vol. 263, 4434-42
- **Howell ; Palade.** *J. Cell Biol.,* 1982, vol. 92, 833-45
- **Higgins ; Hutson.** *J. Lipid Res.,* 1984, vol. 25, 1295-1305
- **Drayna et al.** *Nature,* 1987, vol. 327, 632-634
- **White, T.J. et al.** *Trends Genet.,* 1989, vol. 5, 185-9
- **Taylor, J. W. et al.** *Nucl. Acids Res,* 1985, vol. 13, 8749-64
- **Kunkel, J. A.** *Proc. Natl. Acad. Sci. USA,* 1985, vol. 82, 482-92
- **Sayers, J. R. et al.** *Nucl. Acids Res.,* 1988, vol. 16, 791-800
- **Sanger et al.** *Proc. Natl. Acad. Sci. USA,* 1977, vol. 74, 5463-7
- **Maxam-Gilbert.** *Proc. Natl. Acad. Sci. USA,* 1977, vol. 74, 560-4
- **Pihlajaniemi et al.** *EMBO J.,* 1987, vol. 6, 643-9
- **Yamaguchi et al.** *Biochem. Biophys. Res. Comm.,* 1987, vol. 146, 1485-92
- **Edman et al.** *Nature,* 1985, vol. 317, 267-70
- **Kao et al.** *Connective Tissue Research,* 1988, vol. 18, 157-74
- **Houghton et al.** *Proc. Natl. Acad. Sci.,* 1985, vol. 82, 5131-5
- **Toulme ; Helene.** *Gene,* 1988, vol. 72, 51-8
- **Inouye.** *Gene,* 1988, vol. 72, 25-34
- **Uhlmann ; Peyman.** *Chemical Reviews,* 1990, vol. 90, 543-584
- **Wetterau et al.** *J. Biol. Chem.,* 1990, vol. 265, 9800-7
- **Laemmli.** *Nature,* 1970, vol. 227, 680-5
- **Lowry et al.** *J. Biol. Chem.,* 1951, vol. 193, 265-75
- **Ihm et al.** *J. Biol. Chem.,* 1982, vol. 257, 4818-27
- **E.M. Southern.** *J. Mol. Biol.,* 1975, vol. 98, 503-17
- **Schaefer ; Dische ; Porro.** *Am. J. Med.,* 1970, vol. 49, 568-71
- **Sobrevilla et al.** *Am. J. Med.,* 1964, vol. 37, 821
- **Wetterau ; Zilversmit.** *Chem. Phys. Lipids,* 1985, vol. 38, 205-72
- **Lathe, R.** *J. Mol. Biol,* 1985, vol. 183, 1-12
- **Bimboin ; Doly.** *Nucleic Acids Res.,* 1979, vol. 7, 1513-23
- *J. Biol. Chem.,* 1992, vol. 267, 13229-38
- **Wetterau ; Zilversmit.** *J. Biol. Chem.,* 1984, vol. 259, 10863-6
- **Morton, R.E. et al.** *J. Biol. Chem.,* 1981, vol. 256, 1992-5
- **Schumaker ; Puppion.** *Methods Enzymology,* 1986, vol. 128, 155-170